(19)

Europäisches
Patentamt
European
Patent Office
Office européen
des brevets

(11) **EP 2 169 078 A1**

(12) **EUROPEAN PATENT APPLICATION**

(43) Date of publication:
**31.03.2010 Bulletin 2010/13**

(51) Int Cl.:
**C12Q 1/68** *(2006.01)*

(21) Application number: **08380279.3**

(22) Date of filing: **26.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
- **Fundacion Gaiker
  48170 Zamudilo, Bizkaia (ES)**
- **Universidad del Pais Vasco
  48940 Leioa Vizcaya (ES)**
- **Administración General de la Comunidad Autonoma de
  Euskadi-Osakidetza
  48150 Sondika, Bizkaia (ES)**

(72) Inventors:
- **Garcia Bilbao, Amaia
  48170 Zamudio
  Bizkaia (ES)**

- **Suárez Merino, Blanca
  48170 Zamudio
  Bizkaia (ES)**
- **Betanzos Avellaneda, Mónica
  48170 Zamudio
  Bizkaia (ES)**
- **López Vivanco, Guillermo M.
  48150 Sondika
  Bizkaia (ES)**
- **Armañanzas Arnedillo, Rubén
  48940 Leioa
  Bizkaia (ES)**
- **Inza Cano, Iñaki
  48940 Leioa
  Bizkaia (ES)**
- **Larrañaga Múgica, Pedro
  48940 Leioa
  Vizcaya (ES)**

(74) Representative: **ABG Patentes, S.L.
Avenida de Burgos 16D
Edificio Euromor
28036 Madrid (ES)**

(54) **Methods and kits for the diagnosis and the staging of colorectal cancer**

(57)    The invention relates to methods for the staging and the diagnosis of colorectal cancer based on the determination of the expression levels of a set of genes, the altered expression of which in relation to a reference sample allows diagnosing said cancer with a high reliability as well as determining the stage in which it is.

EP 2 169 078 A1

**Description**

Technical Field of the Invention

The invention is within the field of diagnosis and, more specifically, in the field of diagnosis of colorectal cancer by means of using panels of genes the expression of which is altered in CRC samples compared to non-tumor tissue as well as in tumors of different stages.

Background of the Invention

Colorectal cancer (CRC) is one of the most frequent neoplasias in the western world, it is the third cause of death in men, after lung cancer and prostate cancer and it is the second in frequency among women, after breast cancer. In Spain, approximately 25,000 new cases are diagnosed every year, which constitutes an incidence of 50 new cases for every 100,000 inhabitants and year (www.aecc.org). However, in a European context, the incidence in Spain can be considered as medium-low, in part due to the Mediterranean diet (FINI, L., *et al.,* 2007). Several studies have shown that diets with a high caloric dose and high cholesterol levels are a risk factor for the development of colorectal cancer (BOYLE, P., et al., 2000, BMJ 321: 805-808; KEY, T. J., et al., 2002, Lancet 360: 861-868.; FERNANDEZ, E., et al., 2006, J Br Menopause Soc 12 (4): 139-142.), specially the intake of fats, proteins and refined carbohydrates, as well as the deficiency in fiber.

One of the most used systems for defining the stage of the tumor is Dukes' classification (Dukes, C. E., 1932, J.Pathol. Bacteriol., 35:323-332). Stage A is characterized by a surface invasion of the mucosa. Stage B is characterized by the extension of the tumor through the intestinal wall to the subserous layer. Stage C, in which metastasis to the lymph nodes occurs, comprises stage C1 in which invasion of perirectal nodes occurs and stage C2 in which metastasis in apical nodes appears. Finally, stage D is characterized by the existence of metastasis to distant organs, such as the liver.

Traditionally, the diagnosis of CRC is carried out by means of the detection of blood concealed in feces, flexible sigmoidoscopy and colonoscopy. However, these methods are carried out once the cancer has already begun to invade tissues, due to the symptoms that it causes (changes of intestinal habits or rectal bleeding, among others). This drawback can be solved by means of using molecular markers, i.e., molecules the expression of which is altered in tumor tissue compared to healthy tissue or which show mutations in tumor tissue with respect to the healthy tissue. Different molecular CRC diagnostic markers such as genes APC, K-RAS and TP53 have been described up until now. However, given the diversity of types of tumors, it is unlikely that the use of a single marker can be suitable for the diagnosis of all the possible types of tumors.

For this reason, several CRC diagnosis methods based on the determination of the expression levels of a set of genes have been proposed. Thus, WO07032631 describes a method for the diagnosis of a colorectal tumor based on the determination of alterations in the expression levels of genes CTHRCI, CANP and KIAA0101. WO06015742 describes a method for the diagnosis of a colorectal carcinoma based on the determination of the expression levels of a panel of 120 genes. WO05044990 describes a method for the diagnosis of a colorectal carcinoma based on the determination of the expression levels of a panel of 93 genes. EP1439393 describes a method for the diagnosis of colorectal carcinoma based on the determination of the expression levels of TIMP1. WO03097872 describes a series of genes the expression levels of which are altered in colorectal carcinoma samples as well as methods for the diagnosis of colorectal carcinoma based on the determination of the expression levels of said genes. WO04001072 describes methods for the diagnosis of colorectal cancer based on the determination of the expression levels of 52 genes the expression of which is high in colorectal carcinomas and in 376 genes the expression of which is repressed in colorectal carcinomas with respect to normal colorectal epithelium. This document describes an array comprising probes suitable for the detection of 4 of the 50 genes the expression of which increases in tumors and 24 of the 50 genes the expression of which increases in tumors. EP1355151 describes a method for the diagnosis of colorectal cancer based on the determination of the expression levels of a set of 39 genes.

In addition, the classification or staging of CRC also presents problems and in particular, the distinction between tumors in stage B and tumors in stage C. Several methods for differentiating tumors in different stages have been described up until now. Thus, US2008014579A describes sets of genes which are overexpressed in tumors in Dukes' stage B in relation to tumors in stage C and genes which are repressed in tumors in stage C in relation to tumors in stage B. WO08061527 describes a panel of 30 genes the expression level of which allows predicting the probability of relapse of a CRC patient or the probability of metastasis in distal organs. WO07101609 describes methods for determining the stage of a colorectal tumor by means of determining the expression levels of CEA, EGFR and GA733-2 in disseminated tumor cells obtained from peripheral blood by means of immunoisolation.

Nevertheless, despite the research carried out in this topic, today there are very few tumor markers which are useful from the clinical point of view both for the diagnosis of CRC and for determining the stage of a CRC carcinoma. Therefore, there is a need in the art for markers or panels of markers which allow the diagnosis of CRC and the classification of the stage of colorectal carcinomas with a high reliability.

Summary of the Invention

In a first aspect, the invention relates to a method for the diagnosis of colorectal cancer, for determining the prognosis of a colorectal cancer patient or for determining the effect of a treatment in a colorectal cancer patient comprising determining in a sample of said patient the expression levels of genes TMEM132A, CMTM7, FAM60A, ENC1, ACAT1, MADCAM1 and DDX55, wherein an alteration in the expression levels of said genes with respect to the expression levels in a reference sample indicates that the patient suffers from colorectal cancer, that the colorectal cancer patient has a bad prognosis or that the treatment is not effective.

In a second aspect, the invention relates to a method for differentiating stage B/C colorectal tumors from stage D colorectal tumors comprising determining in a tumor sample the expression levels of genes HAS1, LO8961, NHLRC2, ATP6VOE2, RPS23, GTF2H2, FLJ34077, CASP12 and RP5-1077B9.4, wherein an alteration in the expression levels of said genes with respect to the expression levels in a reference sample indicates that the tumor is a stage B/C tumor according to Dukes' classification.

In a third aspect, the invention relates to a method for differentiating stage B colorectal tumors from stage C colorectal tumors comprising determining in a said tumor the expression levels of genes MEA1, SPRY4, TLR8, SAMSN1, DGCR14, MRCL3, SMAD2, LAPTM4A, SFRS12, CD1B and FAM111A, wherein an alteration in the expression levels of said genes with respect to the expression levels in a reference sample indicates that the tumor is a type B tumor according to Dukes' classification.

In a fourth aspect, the invention relates to a method for determining the stage of a colorectal tumor comprising

(i) determining if the tumor corresponds to stage B/C or to stage D by means of a method as defined in claim 5 and
(ii) in the event that the tumor is a stage B/C tumor, determining if the tumor corresponds to stage B or to stage C by means of a method as defined in claim 8.

In a fifth aspect, the invention relates to a method for the diagnosis of colorectal cancer in a patient comprising

(i) determining if he or she has tumor tissue in a colorectal tissue sample by means of a method as defined in claim 1,
(ii) if tumor tissue is identified in step (a), determining if said tumor tissue corresponds to stage B/C by means of a method as defined in claim 5 and
(iii) if the tumor is identified as stage B/C in step (b), determining if the tumor corresponds to stage B or to stage C by means of a method as defined in claim 8

In a sixth aspect, the invention relates to a kit for the diagnosis of colorectal cancer or for the determination of the stage of a colorectal tumor comprising a first component and, optionally, a second component wherein the first component is a set of reagents consisting of

(i) reagents which allow determining the expression levels of genes TMEM132A, CMTM7, FAM60A, ENC1, ACAT1, MADCAM1 and DDX55,
(ii) reagents which allow determining the expression levels of genes HAS1, LO8961, NHLRC2, ATP6VOE2, RPS23, GTF2H2, FLJ34077, CASP12 and RP5-1077B9.4 or
(iii) reagents which allow determining the expression levels of genes MEA1, SPRY4, TLR8, SAMSN1, DGCR14, MRCL3, SMAD2, LAPTM4A, SFRS12, CD1B and FAM111A

and wherein the second component comprises reagents suitable for determining the expression levels of one or several reference genes.

Brief Description of the Drawings

Figure 1: Schematic representation of the metrics used for the selection of the work probes.

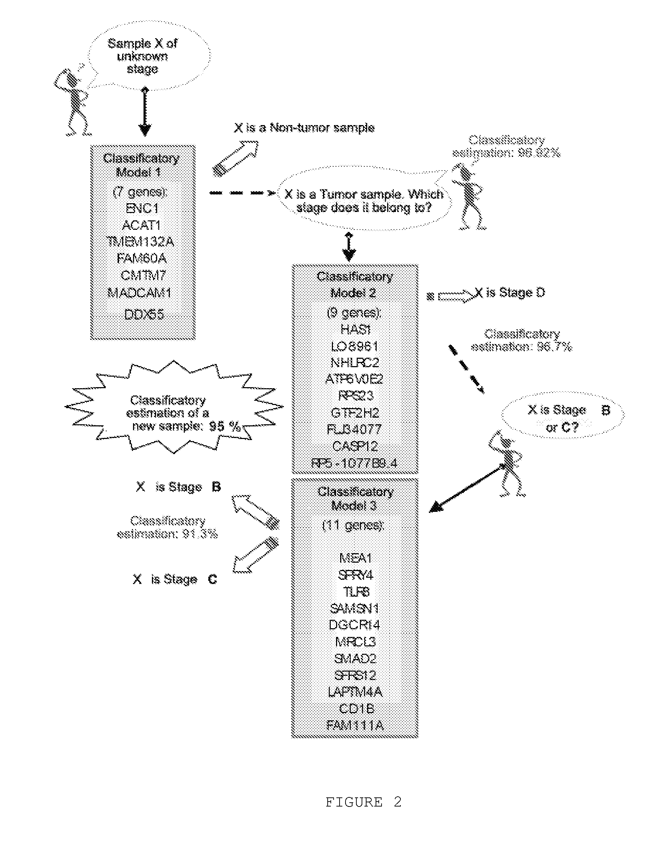

Figure 2: Representative classificatory scheme used for the evaluation of an unknown sample.

Detailed Description of the Invention

Diagnostic method of the invention

The authors of the present invention, using DNA microarrays, have identified a series of genes which are differentially expressed in tumor samples of patients diagnosed with colorectal cancer with respect to healthy colorectal tissue samples. From said initial set of identified genes, a subset of 7 genes the expression variations of which allowed determining if a tissue is a tumor tissue with a reliability of 96.92% was validated by means of real-time PCR. Thus, in a first aspect, the invention relates to a method for the diagnosis of colorectal cancer, for determining the prognosis of a subject diagnosed with colorectal cancer or for determining the effect of a treatment in a colorectal cancer patient comprising determining in a sample of said patient the expression levels of genes TMEM132A, CMTM7, FAM60A, ENC1, ACAT1, MADCAM1 and DDX55, wherein an alteration in the expression levels of said genes with respect to the expression levels in a reference sample indicates that the patient suffers from colorectal cancer, that the colorectal cancer patient has a bad prognosis or that the treatment is not effective.

As used in the present invention, the expression "method for the diagnosis" relates to a method that may essentially consist of the previously mentioned steps or may include additional steps. However, it must be understood that the method, in a preferred embodiment, is a method that is carried out *in vitro,* i.e., it is not carried out in the human or animal body. Diagnosing as used herein relates to evaluating the probability according to which a subject suffers from a disease. As will be understood by persons skilled in the art, such evaluation, although it is preferred that it is, normally may not be correct for 100% of the subjects to be diagnosed. The term, however, requires that a statistically significant part of the subjects can be identified as suffering from the disease or having a predisposition for it. The person skilled in the art can determine if a part is statistically significant without further delay by using several well known statistic evaluation tools, for example, determination of confidence intervals, determination of the p value, Student's t-test, Mann-Whitney test, etc. The details are in Dowdy and Wearden, Statistics for Research, John Wiley & Sons, New York 1983. The preferred confidence intervals are of at least 50%, at least 60%, at least 70%, at least 80%, at least 90%, at least 95%. The p values are preferably 0.2, 0.1, 0.05.

As used in the present invention, the expression "determining the prognosis" relates to the determination of one or several parameters indicating the progression of a patient diagnosed with colorectal cancer. Parameters suitable for determining the evolution of a subject diagnosed with colorectal adenocarcinoma are selected from the group of tumor response, risk of relapse, disease-free survival and/or overall survival of the subject.

In the present invention, "risk of relapse" is understood as the probability of a subject developing colorectal adenocarcinoma and/or a secondary metastasis again after a disease-free period.

In the present invention, "disease-free survival" is understood as the time period after the treatment in which the cancer is not found.

In the present invention, "overall survival of the subject" is understood as the percentage of subjects who survive, from the time of the diagnosis or treatment, after a defined time period.

As used in the present invention, the expression "determining the effect of a therapy" relates to the possibility of determining the response to a therapy. In colorectal cancer therapy, a variety of treatments can be used in an attempt to eliminate or contain the cancer. Depending on the healthy of the subject, as well as the size, location and cancer phase, (i) surgery, consisting of the surgical extirpation of the tumor (ii) cytotoxic/cytostatic treatments, such as chemotherapy, which uses medicinal products against cancer to destroy the cancerous cells upon making the medicinal products circulate through the body through the blood vessels; radiotherapy, which uses high energy radiations to kill the cancer cells, and antitumor agents; and/or (iii) immunotherapy, wherein the administered compound stimulates, enhances or strengthens the natural function of the immune system against cancer to recognize and eliminate the cancerous cells from the body, can be used. Thus, the method of the invention allows determining the response of the patient to a cytotoxic and/or cytostatic treatment and, more specifically, to a treatment by means of chemotherapy, radiotherapy, antitumor agents or combinations thereof.

Suitable chemotherapy agents include but are not limited to alkylating agents such as for example, cyclophosphamide, carmustine, daunorubicin, mechlorethamine, chlorambucil, nimustine, melphalan and the like; anthracyclines, such as for example, daunorubicin, doxorubicin, epirubicin, idarubicin, mitoxantrone, valrubicin and the like; taxane compounds, such as, for example, paclitaxel, docetaxel and the like; topoisomerase inhibitors such as for example, etoposide, teniposide, tuliposide, irinotecan and the like; nucleotide analogs such as for example, azacitidine, azathioprine, capecitabine, cytarabine, doxifluridine, fluorouracil, gemcitabine, mercaptopurine, methotrexate, thioguanine, ftorafur and the like; platinum-based agents such as for example, carboplatin, cisplatin, oxaliplatin and the like; antineoplastic agents such as for example, vincristine, leucovorin, lomustine, procarbazine and the like; hormone modulators such as for example, tamoxifen, finasteride, 5-$\alpha$-reductase inhibitors and the like; vinca alkaloids such as, for example, vinblastine, vincristine, vindesine, vinorelbine and the like. Suitable chemotherapy agents are described with more detail in the literature, such as in The Merck Index in CD-ROM, 13th edition.

In the present invention, "antitumor agent" is understood as that chemical, physical or biological agent or compound with antiproliferative, antioncogenic and/or carcinostatic properties which can be used to inhibit tumor growth, proliferation and/or development. Examples of antitumor agents which can be used in the present invention are (i) antimetabolites, such as antifolates and purine analogs; (ii) natural products, such as antitumor antibiotics and mitotic inhibitors; (iii) hormones and antagonist thereof, such as androgens and corticosteroids; and (iv) biological agents, such as viral vectors. A list of compounds that can be used as antitumor agents is described in patent application WO2005/112973.

In a particular embodiment of the invention, the antitumor agent comprises antiangiogenic agents and signaling pathway inhibitors that, in another more particular embodiment, the antiangiogenic agent is Bevacizumab and the signaling pathway inhibitor is Cetuximab, Panitumumab or Erlotinib.

As used herein, the term "colorectal cancer" includes any type of colon, rectal and appendix neoplasia and refers both to early and late adenomas and to carcinomas as well as to the hereditary, familial or sporadic cancer. Hereditary CRC includes those syndromes which include the presence of polyps, such as the hamartomatous polyposis syndromes and the most known, familial adenomatous polyposis (FAP) as well as nonpolyposis syndromes such as hereditary nonpolyposis colorectal cancer (HNPCC) or Lynch syndrome I. The present invention allows the diagnosis of colorectal cancer in its different stages such as stages A, B, C1, C2 and D according to Dukes' classification, stages A, B1, B2, B3, C1, C2, C3 and D according to the Astler-Coller classification, stages TX, T0, Tis, T1, T2, T3, NX, N0, N1, N2, MX, M0 and M1 according to the TNM system as well as stages 0, I, II, III and IV according to the AJCC (American Joint Committee on Cancer) classification.

In the present invention, the term "sample" or "biological sample" means biological material isolated from a subject. The biological sample can contain any biological material suitable for detecting the desired biomarker and can comprise cell and/or non-cell material of the subject. The sample can be isolated from any suitable tissue or biological fluid such as for example, prostate tissue, blood, blood plasma, serum, urine, feces or cerebrospinal liquid (CSF), feces. The samples used for the determination of the metabolites profiles are preferably colorectal tissue samples obtained by biopsy. Alternatively, the samples are serum samples.

Marker genes in the diagnostic method of the invention include gene TMEM132A (Transmembrane protein 132, NM_017870), gene CMTM7 (CKLF-like MARVEL transmembrane domain containing 7 NM_138410)), FAM60A (family with sequence similarity 60, member A, NM_021238), ENC1 (ectodermal neural cortex-1, NM_003633), ACAT1 (acetyl-coenzyme A acetyltransferase 1, NM_000019), MADCAM1 (mucosal vascular addressing cell adhesion molecule 1, NM_130760) and gene DDX55 (DEAD (Asp-Glu-Ala-Asp) box polypeptide 55, NM_020936).

A "reference sample", as used herein, means a sample obtained from a pool of healthy subjects which does not have a disease state or particular phenotype. The suitable reference expression levels of genes can be determined by measuring the expression levels of said genes in several suitable subjects, and such reference levels can be adjusted to specific subject populations (for example, a reference level can be linked to the age so that comparisons can be made between expression levels in samples of subjects of a certain age and reference levels for a particular disease state, phenotype, or lack thereof in a certain age group). In a preferred embodiment, the reference sample is obtained from several healthy subjects or from subjects without prior history of colorectal cancer. The person skilled in the art will appreciate that the type of reference sample can vary depending on the specific method to be performed. Thus, if it a method of diagnosis or prognosis of the disease is to be carried out, a pool of non-tumor colorectal tissue samples, either from individuals that do not have a history of colorectal cancer or from a pool of distal non-tumor tissues with respect to the respective tumor tissues, can be used as reference. In the event that the method of the invention is aimed at determining the effect of a therapy in said patient, the reference sample is preferably a sample obtained from said patient before starting the treatment.

The expression profile of the genes in the reference sample can preferably, be generated from a population of two or more individuals. The population, for example, can comprise 3, 4, 5, 10, 15, 20, 30, 40, 50 or more individuals. Furthermore, the expression profile of the genes in the reference sample and in the sample of the individual that is going to be diagnosed according to the present invention the methods of the present invention can be generated from the same individual, provided that the profiles to be assayed and the reference profile are generated from biological samples taken at different times and are compared to one another. For example, a sample of an individual can be obtained at the beginning of a study period. A reference biomarker profile from this sample can then be compared with the biomarker profiles generated from subsequent samples of the same individual. In a preferred embodiment, the reference sample is a pool of samples from several individuals and corresponds to portions of colorectal tissue that are far from the tumor area and which have preferably been obtained in the same biopsy but which do not have any anatomopathologic characteristic of tumor tissue.

In a preferred embodiment, the alteration in the expression of the genes that is detected is an increase of the expression of genes ENC1, TMEM132A, FAM60A, CMTM7 and DDX55 and a decrease of the expression of genes ACAT1 and MADCAM1.

The method of the invention comprises the step of determining the expression levels of the marker genes.

Virtually any conventional method can be used within the frame of the invention to detect and quantify the levels of mRNA encoded by the marker genes. By way of a non-limiting illustration, the expression levels are determined by means of the quantification of the levels of mRNA encoded by said genes. The latter can be quantified by means of using conventional methods, for example, methods comprising the amplification of mRNA and the quantification of the amplification product of said mRNA, such as electrophoresis and staining, or alternatively, by means of Northern blot and the use of suitable probes, Northern blot and use of specific probes of the mRNA of the genes of interest or of their corresponding cDNA/cRNA, mapping with the S1 nuclease, RT-PCR, hybridization, microarrays, etc. Similarly, the levels of the cDNA/cRNA corresponding to said mRNA encoded by the marker genes can also be quantified by means of using conventional techniques; in this event, the method of the invention includes a step of synthesis of the corresponding cDNA by means of reverse transcription (RT) of the corresponding mRNA followed by the synthesis (RNA polymerase) and amplification of the cRNA complementary to said cDNA. Conventional methods of quantifying the expression levels can be found, for example, in Sambrook et al., 2001 "Molecular cloning: to Laboratory Manual", 3rd ed., Cold Spring Harbor Laboratory Press, N.Y., Vol. 1-3.

Alternatively, it is also possible to determine the expression levels of the marker genes by means of the determination of the expression levels of the proteins encoded by said genes, since if the expression of genes is increased, an increase of the amount of corresponding protein should occur. The determination of the expression levels of the different proteins can be carried out using any conventional method. By way of a non-limiting example, said determination can be carried out using antibodies with the capacity for binding specifically to the protein to be determined (or to fragments thereof containing the antigenic determinants) and subsequent quantification of the resulting antigen-antibody complexes. The antibodies that are going to be used in this type of assay can be, for example polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemoluminescent reagents, enzyme cofactors or substrates, enzyme inhibitors, particles, dyes, etc. There is a wide variety of well known assays that can be used in the present invention, using non-labeled antibodies (primary antibody) and labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on the colloidal precipitation in formats such as reagent strips. Other forms of detecting and quantifying the protein include affinity chromatography techniques, ligand-binding assays, etc.

Once the expression levels of the marker genes in relation to the expression levels of said genes in a reference sample have been determined, it is necessary to identify if there are alterations in the expression of said genes (increase or decrease of the expression). The expression of a gene is considered increased in a sample of the subject under study when the levels increase with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a gene is considered decreased when its levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

The method of the invention preferably comprises the simultaneous determination of the expression levels of genes TMEM132A, CMTM7, FAM60A, ENC1, ACAT1, MADCAM1 and DDX55. However, the invention contemplates the use of a limited number of said genes, including cancer diagnosis methods in which the expression of genes TMEM132A, CMTM7, FAM60A, ENC1, ACAT1, MADCAM1 and DDX55 is individually determined as well as diagnosis methods based on the determination of each of genes individually as well as of subsets of markers formed by 2, 3, 4, 5 and 6 genes are determined.

Thus, the invention contemplates methods based on the determination of the levels of two genes wherein the two genes are selected from the group:

- TMEM132A, CMTM7
- TMEM132A, FAM60A
- TMEM132A, ENC1
- TMEM132A, ACAT1
- TMEM132A, MADCAM1
- TMEM132A, DDX55

- CMTM7, FAM60A
- CMTM7, ENC1
- CMTM7, ACAT1
- CMTM7, MADCAM1
- CMTM7, DDX55
- FAM60A, ENC1
- FAM60A, ACAT1
- FAM60A, MADCAM1
- FAM60A, DDX55
- ENC1, ACAT1
- ENC1, MADCAM1
- ENC1, DDX55
- ACAT1, MADCAM1
- ACAT1, DDX55
- MADCAM1, DDX55

The invention contemplates methods based on the determination of the levels of three genes wherein the three genes are selected from the group:

- TMEM132A, CMTM7, FAM60A
- TMEM132A, CMTM7, ENC1
- TMEM132A, CMTM7, ACAT1
- TMEM132A, CMTM7, MADCAM1
- TMEM132A, CMTM7, DDX55
- TMEM132A, FAM60A, ENC1
- TMEM132A, FAM60A, ACAT1
- TMEM132A, FAM60A, MADCAM1
- TMEM132A, FAM60A, DDX55
- TMEM132A, ENC1, ACAT1
- TMEM132A, ENC1, MADCAM1
- TMEM132A, ENC1, DDX55
- TMEM132A, ACAT1, MADCAM1
- TMEM132A, ACAT1, DDX55
- TMEM132A, MADCAM1, DDX55
- CMTM7, FAM60A, ENC1
- CMTM7, FAM60A, ACAT1
- CMTM7, FAM60A, MADCAM1
- CMTM7, FAM60A, DDX55
- CMTM7, ENC1, ACAT1
- CMTM7, ENC1, MADCAM1
- CMTM7, ENC1, DDX55
- CMTM7, ACAT1, MADCAM1
- CMTM7, ACAT1, DDX55
- CMTM7, MADCAM1, DDX55
- FAM60A, ENC1, ACAT1
- FAM60A, ENC1, MADCAM1
- FAM60A, ENC1, DDX55
- FAM60A, ACAT1, MADCAM1
- FAM60A, ACAT1, DDX55
- FAM60A, MADCAM1, DDX55
- ENC1, ACAT1, MADCAM1
- ENC1, ACAT1, DDX55
- ENC1, MADCAM1, DDX55
- ACAT1, MADCAM1, DDX55

The invention contemplates methods based on the determination of the levels of 4 genes wherein the 4 genes are selected from the group:

- TMEM132A, CMTM7, FAM60A, ENC1
- TMEM132A, CMTM7, FAM60A, ACAT1
- TMEM132A, CMTM7, FAM60A, MADCAM1
- TMEM132A, CMTM7, FAM60A, DDX55
- TMEM132A, CMTM7, ENC1, ACAT1
- TMEM132A, CMTM7, ENC1, MADCAM1
- TMEM132A, CMTM7, ENC1, DDX55
- TMEM132A, CMTM7, ACAT1, MADCAM1
- TMEM132A, CMTM7, ACAT1, DDX55
- TMEM132A, CMTM7, MADCAM1, DDX55
- TMEM132A, FAM60A, ENC1, ACAT1
- TMEM132A, FAM60A, ENC1, MADCAM1
- TMEM132A, FAM60A, ENC1, DDX55
- TMEM132A, FAM60A, ACAT1, MADCAM1
- TMEM132A, FAM60A, ACAT1, DDX55
- TMEM132A, FAM60A, MADCAM1, DDX55
- TMEM132A, ENC1, ACAT1, MADCAM1
- TMEM132A, ENC1, ACAT1, DDX55
- TMEM132A, ENC1, MADCAM1, DDX55
- TMEM132A, ACAT1, MADCAM1, DDX55
- CMTM7, FAM60A, ENC1, ACAT1
- CMTM7, FAM60A, ENC1, MADCAM1
- CMTM7, FAM60A, ENC1, DDX55
- CMTM7, FAM60A, ACAT1, MADCAM1
- CMTM7, FAM60A, ACAT1, DDX55
- CMTM7, FAM60A, MADCAM1, DDX55
- CMTM7, ENC1, ACAT1, MADCAM1
- CMTM7, ENC1, ACAT1, DDX55
- CMTM7, ENC1, MADCAM1, DDX55
- CMTM7, ACAT1, MADCAM1, DDX55
- FAM60A, ENC1, ACAT1, MADCAM1
- FAM60A, ENC1, ACAT1, DDX55
- FAM60A, ENC1, MADCAM1, DDX55
- FAM60A, ACAT1, MADCAM1, DDX55
- ENC1, ACAT1, MADCAM1, DDX55

The invention contemplates methods based on the determination of the levels of 5 genes wherein the 5 genes are selected from the group of

- TMEM132A, CMTM7, FAM60A, ENC1, ACAT1
- TMEM132A, CMTM7, FAM60A, ENC1, MADCAM1
- TMEM132A, CMTM7, FAM60A, ENC1, DDX55
- TMEM132A, CMTM7, FAM60A, ACAT1, MADCAM1
- TMEM132A, CMTM7, FAM60A, ACAT1, DDX55
- TMEM132A, CMTM7, FAM60A, MADCAM1, DDX55
- TMEM132A, CMTM7, ENC1, ACAT1, MADCAM1
- TMEM132A, CMTM7, ENC1, ACAT1, DDX55
- TMEM132A, CMTM7, ENC1, MADCAM1, DDX55
- TMEM132A, CMTM7, ACAT1, MADCAM1, DDX55
- TMEM132A, FAM60A, ENC1, ACAT1, MADCAM1
- TMEM132A, FAM60A, ENC1, ACAT1, DDX55
- TMEM132A, FAM60A, ENC1, MADCAM1, DDX55
- TMEM132A, FAM60A, ACAT1, MADCAM1, DDX55
- TMEM132A, ENC1, ACAT1, MADCAM1, DDX55
- CMTM7, FAM60A, ENC1, ACAT1, MADCAM1
- CMTM7, FAM60A, ENC1, ACAT1, DDX55
- CMTM7, FAM60A, ENC1, MADCAM1, DDX55
- CMTM7, FAM60A, ACAT1, MADCAM1, DDX55

8

- CMTM7, ENC1, ACAT1, MADCAM1, DDX55
- FAM60A, ENC1, ACAT1, MADCAM1, DDX55

The invention contemplates methods based on the determination of the levels of 6 genes wherein the 6 genes are selected from the group:

- TMEM132A, CMTM7, FAM60A, ENC1, ACAT1, MADCAM1
- TMEM132A, CMTM7, FAM60A, ENC1, ACAT1, DDX55
- TMEM132A, CMTM7, FAM60A, ENC1, MADCAM1, DDX55
- TMEM132A, CMTM7, FAM60A, ACAT1, MADCAM1, DDX55
- TMEM132A, CMTM7, ENC1, ACAT1, MADCAM1, DDX55
- TMEM132A, FAM60A, ENC1, ACAT1, MADCAM1, DDX55
- CMTM7, FAM60A, ENC1, ACAT1, MADCAM1, DDX55

The gene expression profiles of the invention can be used in combination with other genetic and non-genetic markers already known for the diagnosis of CRC. Thus, the invention contemplates the use of the expression profiles previously mentioned together with the determination of the expression levels of one or several additional markers such as serum markers (for example, carcinoembrionic antigen) or analytes such as CA19-9, CA 125, CK-BB and the guanylyl cyclase C or together with methods based on the detection of blood in feces or the detection in feces of polymorphisms in genes K-ras, APC, p53 and BAT-26.

First staging method of the invention

The authors of the present invention have identified another set of genes which allow distinguishing between carcinomas in stage D and in stage B/C with a reliability of 96.7%. Thus, in another aspect, the invention relates to a method (hereinafter first staging method of the invention) to differentiate stage B/C colorectal tumors from stage D colorectal tumors comprising determining in a tumor sample the expression levels of genes HAS1, LO8961, NHLRC2, ATP6VOE2, RPS23, GTF2H2, FLJ34077, CASP12 and RP5-1077B9.4, wherein an altered high expression level of said genes with respect to the expression levels in a reference sample indicates that the tumor is a stage B/C tumor according to Dukes' classification. The method preferably includes the determination of an increased expression level of genes HAS1, RPS23, GTF2H2, FLJ34077, CASP12 and a reduced expression level of genes LO8961, NHLRC2, ATP6VOE2, RP5-1077B9.4 with respect to a reference sample.
Genes which allow distinguishing colorectal carcinomas in stage D against those that are in stage B/C include HAS1 (hyalouronan synthase, NM_001523), LO8961 (Homo sapiens transmembrane tyosine kinase mRNA, complete cds., LO8961) NHLRC2 (NHL repeat containing 2, AK126751), ATP6VOE2 (ATPase H+ transporting V0 subunit e2, NM_145230), RPS23 (Ribosomal protein S3, NM_001023), GTF2H2 (General Transcription factor IIH polypeptide 2, NM_001515), FLJ34077 (Weakly similar to zinc finger protein 195, BC003519), CASP12 (Caspase 12 120329 NM_00035) and RP5-1077B9.4 (Invasion inhibitory protein 45 60672 NM_021933).
The different steps and preferred forms of the invention (sample, methods for determining expression levels, etc.) are essentially carried out as has been previously described in the case of the first method of the invention.
Although the second method of the invention preferably comprises the determination of the expression levels of the previously mentioned 9 genes, the invention contemplates methods in which staging is carried out by means of the determination of the expression levels of a subset of genes formed by 2, 3, 4 , 5, 6, 7 or 8 genes.
Thus, combinations of two genes suitable for the staging of the tumor include

- HAS1, LO8961
- HAS1, NHLRC2
- HAS1, ATP6VOE2
- HAS1, RPS23
- HAS1, GTF2H2
- HAS1, FLJ34077
- HAS1, CASP12
- HAS1, RP5-1077B9.4
- LO8961, NHLRC2
- LO8961, ATP6VOE2
- LO8961, RPS23
- LO8961, GTF2H2
- LO8961, FLJ34077

- LO8961, CASP12
- LO8961, RP5-1077B9.4
- NHLRC2, ATP6VOE2
- NHLRC2, RPS23
- NHLRC2, GTF2H2
- NHLRC2, FLJ34077
- NHLRC2, CASP12
- NHLRC2, RP5-1077B9.4
- ATP6VOE2, RPS23
- ATP6VOE2, GTF2H2
- ATP6VOE2, FLJ34077
- ATP6VOE2, CASP12
- ATP6VOE2, RP5-1077B9.4
- RPS23, GTF2H2
- RPS23, FLJ34077
- RPS23, CASP12
- RPS23, RP5-1077B9.4
- GTF2H2, FLJ34077
- GTF2H2, CASP12
- GTF2H2, RP5-1077B9.4
- FLJ34077, CASP12
- FLJ34077, RP5-1077B9.4
- CASP12, RP5-1077B9.4

Combinations of three genes suitable for the staging of the tumor include:

- HAS1, LO8961, NHLRC2
- HAS1, LO8961, ATP6VOE2
- HAS1, LO8961, RPS23
- HAS1, LO8961, GTF2H2
- HAS1, LO8961, FLJ34077
- HAS1, LO8961, CASP12
- HAS1, LO8961, RP5-1077B9.4
- HAS1, NHLRC2, ATP6VOE2
- HAS1, NHLRC2, RPS23
- HAS1, NHLRC2, GTF2H2
- HAS1, NHLRC2, FLJ34077
- HAS1, NHLRC2, CASP12
- HAS1, NHLRC2, RP5-1077B9.4
- HAS1, ATP6VOE2, RPS23
- HAS1, ATP6VOE2, GTF2H2
- HAS1, ATP6V0E2, FLJ34077
- HAS1, ATP6V0E2, CASP12
- HAS1, ATP6VOE2, RP5-1077B9.4
- HAS1, RPS23, GTF2H2
- HAS1, RPS23, FLJ34077
- HAS1, RPS23, CASP12
- HAS1, RPS23, RP5-1077B9.4
- HAS1, GTF2H2, FLJ34077
- HAS1, GTF2H2, CASP12
- HAS1, GTF2H2, RP5-1077B9.4
- HAS1, FLJ34077, CASP12
- HAS1, FLJ34077, RP5-1077B9.4
- HAS1, CASP12, RP5-1077B9.4
- LO8961, NHLRC2, ATP6VOE2
- LO8961, NHLRC2, RPS23
- LO8961, NHLRC2, GTF2H2
- LO8961, NHLRC2, FLJ34077

- LO8961, NHLRC2, CASP12
- LO8961, NHLRC2, RP5-1077B9.4
- LO8961, ATP6VOE2, RPS23
- LO8961, ATP6VOE2, GTF2H2
- LO8961, ATP6VOE2, FLJ34077
- LO8961, ATP6VOE2, CASP12
- LO8961, ATP6VOE2, RP5-1077B9.4
- LO8961, RPS23, GTF2H2
- LO8961, RPS23, FLJ34077
- LO8961, RPS23, CASP12
- LO8961, RPS23, RP5-1077B9.4
- LO8961, GTF2H2, FLJ34077
- LO8961, GTF2H2, CASP12
- LO8961, GTF2H2, RP5-1077B9.4
- LO8961, FLJ34077, CASP12
- LO8961, FLJ34077, RP5-1077B9.4
- LO8961, CASP12, RP5-1077B9.4
- NHLRC2, ATP6V0E2, RPS23
- NHLRC2, ATP6V0E2, GTF2H2
- NHLRC2, ATP6V0E2, FLJ34077
- NHLRC2, ATP6VOE2, CASP12
- NHLRC2, ATP6VOE2, RP5-1077B9.4
- NHLRC2, RPS23, GTF2H2
- NHLRC2, RPS23, FLJ34077
- NHLRC2, RPS23, CASP12
- NHLRC2, RPS23, RP5-1077B9.4
- NHLRC2, GTF2H2, FLJ34077
- NHLRC2, GTF2H2, CASP12
- NHLRC2, GTF2H2, RP5-1077B9.4
- NHLRC2, FLJ34077, CASP12
- NHLRC2, FLJ34077, RP5-1077B9.4
- NHLRC2, CASP12, RP5-1077B9.4
- ATP6VOE2, RPS23, GTF2H2
- ATP6VOE2, RPS23, FLJ34077
- ATP6VOE2, RPS23, CASP12
- ATP6VOE2, RPS23, RP5-1077B9.4
- ATP6VOE2, GTF2H2, FLJ34077
- ATP6VOE2, GTF2H2, CASP12
- ATP6VOE2, GTF2H2, RP5-1077B9.4
- ATP6VOE2, FLJ34077, CASP12
- ATP6VOE2, FLJ34077, RP5-1077B9.4
- ATP6VOE2, CASP12, RP5-1077B9.4
- RPS23, GTF2H2, FLJ34077
- RPS23, GTF2H2, CASP12
- RPS23, GTF2H2, RP5-1077B9.4
- RPS23, FLJ34077, CASP12
- RPS23, FLJ34077, RP5-1077B9.4
- RPS23, CASP12, RP5-1077B9.4
- GTF2H2, FLJ34077, CASP12
- GTF2H2, FLJ34077, RP5-1077B9.4
- GTF2H2, CASP12, RP5-1077B9.4
- FLJ34077, CASP12, RP5-1077B9.4

Combinations of 4 genes suitable for the staging of the tumor include:

- HAS1, LO8961, NHLRC2, ATP6VOE2
- HAS1, LO8961, NHLRC2, RPS23
- HAS1, LO8961, NHLRC2, GTF2H2

- HAS1, LO8961, NHLRC2, FLJ34077
- HAS1, LO8961, NHLRC2, CASP12
- HAS1, LO8961, NHLRC2, RP5-1077B9.4
- HAS1, LO8961, ATP6VOE2, RPS23
- HAS1, LO8961, ATP6VOE2, GTF2H2
- HAS1, LO8961, ATP6VOE2, FLJ34077
- HAS1, LO8961, ATP6VOE2, CASP12
- HAS1, LO8961, ATP6VOE2, RP5-1077B9.4
- HAS1, LO8961, RPS23, GTF2H2
- HAS1, LO8961, RPS23, FLJ34077
- HAS1, LO8961, RPS23, CASP12
- HAS1, LO8961, RPS23, RP5-1077B9.4
- HAS1, LO8961, GTF2H2, FLJ34077
- HAS1, LO8961, GTF2H2, CASP12
- HAS1, LO8961, GTF2H2, RP5-1077B9.4
- HAS1, LO8961, FLJ34077, CASP12
- HAS1, LO8961, FLJ34077, RP5-1077B9.4
- HAS1, LO8961, CASP12, RP5-1077B9.4
- HAS1, NHLRC2, ATP6VOE2, RPS23
- HAS1, NHLRC2, ATP6VOE2, GTF2H2
- HAS1, NHLRC2, ATP6VOE2, FLJ34077
- HAS1, NHLRC2, ATP6VOE2, CASP12
- HAS1, NHLRC2, ATP6VOE2, RP5-1077B9.4
- HAS1, NHLRC2, RPS23, GTF2H2
- HAS1, NHLRC2, RPS23, FLJ34077
- HAS1, NHLRC2, RPS23, CASP12
- HAS1, NHLRC2, RPS23, RP5-1077B9.4
- HAS1, NHLRC2, GTF2H2, FLJ34077
- HAS1, NHLRC2, GTF2H2, CASP12
- HAS1, NHLRC2, GTF2H2, RP5-1077B9.4
- HAS1, NHLRC2, FLJ34077, CASP12
- HAS1, NHLRC2, FLJ34077, RP5-1077B9.4
- HAS1, NHLRC2, CASP12, RP5-1077B9.4
- HAS1, ATP6VOE2, RPS23, GTF2H2
- HAS1, ATP6VOE2, RPS23, FLJ34077
- HAS1, ATP6VOE2, RPS23, CASP12
- HAS1, ATP6VOE2, RPS23, RP5-1077B9.4
- HAS1, ATP6VOE2, GTF2H2, FLJ34077
- HAS1, ATP6VOE2, GTF2H2, CASP12
- HAS1, ATP6VOE2, GTF2H2, RP5-1077B9.4
- HAS1, ATP6VOE2, FLJ34077, CASP12
- HAS1, ATP6VOE2, FLJ34077, RP5-1077B9.4
- HAS1, ATP6VOE2, CASP12, RP5-1077B9.4
- HAS1, RPS23, GTF2H2, FLJ34077
- HAS1, RPS23, GTF2H2, CASP12
- HAS1, RPS23, GTF2H2, RP5-1077B9.4
- HAS1, RPS23, FLJ34077, CASP12
- HAS1, RPS23, FLJ34077, RP5-1077B9.4
- HAS1, RPS23, CASP12, RP5-1077B9.4
- HAS1, GTF2H2, FLJ34077, CASP12
- HAS1, GTF2H2, FLJ34077, RP5-1077B9.4
- HAS1, GTF2H2, CASP12, RP5-1077B9.4
- HAS1, FLJ34077, CASP12, RP5-1077B9.4
- LO8961, NHLRC2, ATP6VOE2, RPS23
- LO8961, NHLRC2, ATP6VOE2, GTF2H2
- LO8961, NHLRC2, ATP6VOE2, FLJ34077
- LO8961, NHLRC2, ATP6VOE2, CASP12
- LO8961, NHLRC2, ATP6VOE2, RP5-1077B9.4

- LO8961, NHLRC2, RPS23, GTF2H2
- LO8961, NHLRC2, RPS23, FLJ34077
- LO8961, NHLRC2, RPS23, CASP12
- LO8961, NHLRC2, RPS23, RP5-1077B9.4
- LO8961, NHLRC2, GTF2H2, FLJ34077
- LO8961, NHLRC2, GTF2H2, CASP12
- LO8961, NHLRC2, GTF2H2, RP5-1077B9.4
- LO8961, NHLRC2, FLJ34077, CASP12
- LO8961, NHLRC2, FLJ34077, RP5-1077B9.4
- LO8961, NHLRC2, CASP12, RP5-1077B9.4
- LO8961, ATP6VOE2, RPS23, GTF2H2
- LO8961, ATP6VOE2, RPS23, FLJ34077
- LO8961, ATP6VOE2, RPS23, CASP12
- LO8961, ATP6VOE2, RPS23, RP5-1077B9.4
- LO8961, ATP6VOE2, GTF2H2, FLJ34077
- LO8961, ATP6VOE2, GTF2H2, CASP12
- LO8961, ATP6VOE2, GTF2H2, RP5-1077B9.4
- LO8961, ATP6VOE2, FLJ34077, CASP12
- LO8961, ATP6VOE2, FLJ34077, RP5-1077B9.4
- LO8961, ATP6VOE2, CASP12, RP5-1077B9.4
- LO8961, RPS23, GTF2H2, FLJ34077
- LO8961, RPS23, GTF2H2, CASP12
- LO8961, RPS23, GTF2H2, RP5-1077B9.4
- LO8961, RPS23, FLJ34077, CASP12
- LO8961, RPS23, FLJ34077, RP5-1077B9.4
- LO8961, RPS23, CASP12, RP5-1077B9.4
- LO8961, GTF2H2, FLJ34077, CASP12
- LO8961, GTF2H2, FLJ34077, RP5-1077B9.4
- LO8961, GTF2H2, CASP12, RP5-1077B9.4
- LO8961, FLJ34077, CASP12, RP5-1077B9.4
- NHLRC2, ATP6VOE2, RPS23, GTF2H2
- NHLRC2, ATP6VOE2, RPS23, FLJ34077
- NHLRC2, ATP6VOE2, RPS23, CASP12
- NHLRC2, ATP6VOE2, RPS23, RP5-1077B9.4
- NHLRC2, ATP6VOE2, GTF2H2, FLJ34077
- NHLRC2, ATP6VOE2, GTF2H2, CASP12
- NHLRC2, ATP6VOE2, GTF2H2, RP5-1077B9.4
- NHLRC2, ATP6VOE2, FLJ34077, CASP12
- NHLRC2, ATP6V0E2, FLJ34077, RP5-1077B9.4
- NHLRC2, ATP6VOE2, CASP12, RP5-1077B9.4
- NHLRC2, RPS23, GTF2H2, FLJ34077
- NHLRC2, RPS23, GTF2H2, CASP12
- NHLRC2, RPS23, GTF2H2, RP5-1077B9.4
- NHLRC2, RPS23, FLJ34077, CASP12
- NHLRC2, RPS23, FLJ34077, RP5-1077B9.4
- NHLRC2, RPS23, CASP12, RP5-1077B9.4
- NHLRC2, GTF2H2, FLJ34077, CASP12
- NHLRC2, GTF2H2, FLJ34077, RP5-1077B9.4
- NHLRC2, GTF2H2, CASP12, RP5-1077B9.4
- NHLRC2, FLJ34077, CASP12, RP5-1077B9.4
- ATP6VOE2, RPS23, GTF2H2, FLJ34077
- ATP6VOE2, RPS23, GTF2H2, CASP12
- ATP6VOE2, RPS23, GTF2H2, RP5-1077B9.4
- ATP6VOE2, RPS23, FLJ34077, CASP12
- ATP6VOE2, RPS23, FLJ34077, RP5-1077B9.4
- ATP6VOE2, RPS23, CASP12, RP5-1077B9.4
- ATP6VOE2, GTF2H2, FLJ34077, CASP12
- ATP6VOE2, GTF2H2, FLJ34077, RP5-1077B9.4

- ATP6VOE2, GTF2H2, CASP12, RP5-1077B9.4
- ATP6VOE2, FLJ34077, CASP12, RP5-1077B9.4
- RPS23, GTF2H2, FLJ34077, CASP12
- RPS23, GTF2H2, FLJ34077, RP5-1077B9.4
- RPS23, GTF2H2, CASP12, RP5-1077B9.4
- RPS23, FLJ34077, CASP12, RP5-1077B9.4
- GTF2H2, FLJ34077, CASP12, RP5-1077B9.4

Combinations of 5 genes suitable for the staging of the tumor include:

- HAS1, LO8961, NHLRC2, ATP6VOE2, RPS23
- HAS1, LO8961, NHLRC2, ATP6VOE2, GTF2H2
- HAS1, LO8961, NHLRC2, ATP6VOE2, FLJ34077
- HAS1, LO8961, NHLRC2, ATP6VOE2, CASP12
- HAS1, LO8961, NHLRC2, ATP6VOE2, RP5-1077B9.4
- HAS1, LO8961, NHLRC2, RPS23, GTF2H2
- HAS1, LO8961, NHLRC2, RPS23, FLJ34077
- HAS1, LO8961, NHLRC2, RPS23, CASP12
- HAS1, LO8961, NHLRC2, RPS23, RP5-1077B9.4
- HAS1, LO8961, NHLRC2, GTF2H2, FLJ34077
- HAS1, LO8961, NHLRC2, GTF2H2, CASP12
- HAS1, LO8961, NHLRC2, GTF2H2, RP5-1077B9.4
- HAS1, LO8961, NHLRC2, FLJ34077, CASP12
- HAS1, LO8961, NHLRC2, FLJ34077, RP5-1077B9.4
- HAS1, LO8961, NHLRC2, CASP12, RP5-1077B9.4
- HAS1, LO8961, ATP6VOE2, RPS23, GTF2H2
- HAS1, LO8961, ATP6VOE2, RPS23, FLJ34077
- HAS1, LO8961, ATP6VOE2, RPS23, CASP12
- HAS1, LO8961, ATP6VOE2, RPS23, RP5-1077B9.4
- HAS1, LO8961, ATP6VOE2, GTF2H2, FLJ34077
- HAS1, LO8961, ATP6VOE2, GTF2H2, CASP12
- HAS1, LO8961, ATP6VOE2, GTF2H2, RP5-1077B9.4
- HAS1, LO8961, ATP6VOE2, FLJ34077, CASP12
- HAS1, LO8961, ATP6VOE2, FLJ34077, RP5-1077B9.4
- HAS1, LO8961, ATP6VOE2, CASP12, RP5-1077B9.4
- HAS1, LO8961, RPS23, GTF2H2, FLJ34077
- HAS1, LO8961, RPS23, GTF2H2, CASP12
- HAS1, LO8961, RPS23, GTF2H2, RP5-1077B9.4
- HAS1, LO8961, RPS23, FLJ34077, CASP12
- HAS1, LO8961, RPS23, FLJ34077, RP5-1077B9.4
- HAS1, LO8961, RPS23, CASP12, RP5-1077B9.4
- HAS1, LO8961, GTF2H2, FLJ34077, CASP12
- HAS1, LO8961, GTF2H2, FLJ34077, RP5-1077B9.4
- HAS1, LO8961, GTF2H2, CASP12, RP5-1077B9.4
- HAS1, LO8961, FLJ34077, CASP12, RP5-1077B9.4
- HAS1, NHLRC2, ATP6VOE2, RPS23, GTF2H2
- HAS1, NHLRC2, ATP6VOE2, RPS23, FLJ34077
- HAS1, NHLRC2, ATP6VOE2, RPS23, CASP12
- HAS1, NHLRC2, ATP6VOE2, RPS23, RP5-1077B9.4
- HAS1, NHLRC2, ATP6VOE2, GTF2H2, FLJ34077
- HAS1, NHLRC2, ATP6V0E2, GTF2H2, CASP12
- HAS1, NHLRC2, ATP6VOE2, GTF2H2, RP5-1077B9.4
- HAS1, NHLRC2, ATP6VOE2, FLJ34077, CASP12
- HAS1, NHLRC2, ATP6VOE2, FLJ34077, RP5-1077B9.4
- HAS1, NHLRC2, ATP6VOE2, CASP12, RP5-1077B9.4
- HAS1, NHLRC2, RPS23, GTF2H2, FLJ34077
- HAS1, NHLRC2, RPS23, GTF2H2, CASP12
- HAS1, NHLRC2, RPS23, GTF2H2, RP5-1077B9.4

14

- HAS1, NHLRC2, RPS23, FLJ34077, CASP12
- HAS1, NHLRC2, RPS23, FLJ34077, RP5-1077B9.4
- HAS1, NHLRC2, RPS23, CASP12, RP5-1077B9.4
- HAS1, NHLRC2, GTF2H2, FLJ34077, CASP12
- HAS1, NHLRC2, GTF2H2, FLJ34077, RP5-1077B9.4
- HAS1, NHLRC2, GTF2H2, CASP12, RP5-1077B9.4
- HAS1, NHLRC2, FLJ34077, CASP12, RP5-1077B9.4
- HAS1, ATP6VOE2, RPS23, GTF2H2, FLJ34077
- HAS1, ATP6VOE2, RPS23, GTF2H2, CASP12
- HAS1, ATP6VOE2, RPS23, GTF2H2, RP5-1077B9.4
- HAS1, ATP6VOE2, RPS23, FLJ34077, CASP12
- HAS1, ATP6VOE2, RPS23, FLJ34077, RP5-1077B9.4
- HAS1, ATP6VOE2, RPS23, CASP12, RP5-1077B9.4
- HAS1, ATP6VOE2, GTF2H2, FLJ34077, CASP12
- HAS1, ATP6VOE2, GTF2H2, FLJ34077, RP5-1077B9.4
- HAS1, ATP6VOE2, GTF2H2, CASP12, RP5-1077B9.4
- HAS1, ATP6VOE2, FLJ34077, CASP12, RP5-1077B9.4
- HAS1, RPS23, GTF2H2, FLJ34077, CASP12
- HAS1, RPS23, GTF2H2, FLJ34077, RP5-1077B9.4
- HAS1, RPS23, GTF2H2, CASP12, RP5-1077B9.4
- HAS1, RPS23, FLJ34077, CASP12, RP5-1077B9.4
- HAS1, GTF2H2, FLJ34077, CASP12, RP5-1077B9.4
- LO8961, NHLRC2, ATP6VOE2, RPS23, GTF2H2
- LO8961, NHLRC2, ATP6VOE2, RPS23, FLJ34077
- LO8961, NHLRC2, ATP6VOE2, RPS23, CASP12
- LO8961, NHLRC2, ATP6VOE2, RPS23, RP5-1077B9.4
- LO8961, NHLRC2, ATP6VOE2, GTF2H2, FLJ34077
- LO8961, NHLRC2, ATP6VOE2, GTF2H2, CASP12
- LO8961, NHLRC2, ATP6VOE2, GTF2H2, RP5-1077B9.4
- LO8961, NHLRC2, ATP6VOE2, FLJ34077, CASP12
- LO8961, NHLRC2, ATP6VOE2, FLJ34077, RP5-1077B9.4
- LO8961, NHLRC2, ATP6VOE2, CASP12, RP5-1077B9.4
- LO8961, NHLRC2, RPS23, GTF2H2, FLJ34077
- LO8961, NHLRC2, RPS23, GTF2H2, CASP12
- LO8961, NHLRC2, RPS23, GTF2H2, RP5-1077B9.4
- LO8961, NHLRC2, RPS23, FLJ34077, CASP12
- LO8961, NHLRC2, RPS23, FLJ34077, RP5-1077B9.4
- LO8961, NHLRC2, RPS23, CASP12, RP5-1077B9.4
- LO8961, NHLRC2, GTF2H2, FLJ34077, CASP12
- LO8961, NHLRC2, GTF2H2, FLJ34077, RP5-1077B9.4
- LO8961, NHLRC2, GTF2H2, CASP12, RP5-1077B9.4
- LO8961, NHLRC2, FLJ34077, CASP12, RP5-1077B9.4
- LO8961, ATP6VOE2, RPS23, GTF2H2, FLJ34077
- LO8961, ATP6VOE2, RPS23, GTF2H2, CASP12
- LO8961, ATP6VOE2, RPS23, GTF2H2, RP5-1077B9.4
- LO8961, ATP6VOE2, RPS23, FLJ34077, CASP12
- LO8961, ATP6VOE2, RPS23, FLJ34077, RP5-1077B9.4
- LO8961, ATP6VOE2, RPS23, CASP12, RP5-1077B9.4
- LO8961, ATP6VOE2, GTF2H2, FLJ34077, CASP12
- LO8961, ATP6VOE2, GTF2H2, FLJ34077, RP5-1077B9.4
- LO8961, ATP6VOE2, GTF2H2, CASP12, RP5-1077B9.4
- LO8961, ATP6VOE2, FLJ34077, CASP12, RP5-1077B9.4
- LO8961, RPS23, GTF2H2, FLJ34077, CASP12
- LO8961, RPS23, GTF2H2, FLJ34077, RP5-1077B9.4
- LO8961, RPS23, GTF2H2, CASP12, RP5-1077B9.4
- LO8961, RPS23, FLJ34077, CASP12, RP5-1077B9.4
- LO8961, GTF2H2, FLJ34077, CASP12, RP5-1077B9.4
- NHLRC2, ATP6VOE2, RPS23, GTF2H2, FLJ34077

- NHLRC2, ATP6VOE2, RPS23, GTF2H2, CASP12
- NHLRC2, ATP6V0E2, RPS23, GTF2H2, RP5-1077B9.4
- NHLRC2, ATP6VOE2, RPS23, FLJ34077, CASP12
- NHLRC2, ATP6VOE2, RPS23, FLJ34077, RP5-1077B9.4
- NHLRC2, ATP6VOE2, RPS23, CASP12, RP5-1077B9.4
- NHLRC2, ATP6VOE2, GTF2H2, FLJ34077, CASP12
- NHLRC2, ATP6VOE2, GTF2H2, FLJ34077, RP5-1077B9.4
- NHLRC2, ATP6VOE2, GTF2H2, CASP12, RP5-1077B9.4
- NHLRC2, ATP6VOE2, FLJ34077, CASP12, RP5-1077B9.4
- NHLRC2, RPS23, GTF2H2, FLJ34077, CASP12
- NHLRC2, RPS23, GTF2H2, FLJ34077, RP5-1077B9.4
- NHLRC2, RPS23, GTF2H2, CASP12, RP5-1077B9.4
- NHLRC2, RPS23, FLJ34077, CASP12, RP5-1077B9.4
- NHLRC2, GTF2H2, FLJ34077, CASP12, RP5-1077B9.4
- ATP6VOE2, RPS23, GTF2H2, FLJ34077, CASP12
- ATP6VOE2, RPS23, GTF2H2, FLJ34077, RP5-1077B9.4
- ATP6VOE2, RPS23, GTF2H2, CASP12, RP5-1077B9.4
- ATP6VOE2, RPS23, FLJ34077, CASP12, RP5-1077B9.4
- ATP6VOE2, GTF2H2, FLJ34077, CASP12, RP5-1077B9.4
- RPS23, GTF2H2, FLJ34077, CASP12, RP5-1077B9.4

Combinations of 6 genes suitable for the staging of the tumor include:

- HAS1, LO8961, NHLRC2, ATP6VOE2, RPS23, GTF2H2
- HAS1, LO8961, NHLRC2, ATP6VOE2, RPS23, FLJ34077
- HAS1, LO8961, NHLRC2, ATP6VOE2, RPS23, CASP12
- HAS1, LO8961, NHLRC2, ATP6VOE2, RPS23, RP5-1077B9.4
- HAS1, LO8961, NHLRC2, ATP6VOE2, GTF2H2, FLJ34077
- HAS1, LO8961, NHLRC2, ATP6VOE2, GTF2H2, CASP12
- HAS1, LO8961, NHLRC2, ATP6VOE2, GTF2H2, RP5-1077B9.4
- HAS1, LO8961, NHLRC2, ATP6VOE2, FLJ34077, CASP12
- HAS1, LO8961, NHLRC2, ATP6VOE2, FLJ34077, RP5-1077B9.4
- HAS1, LO8961, NHLRC2, ATP6VOE2, CASP12, RP5-1077B9.4
- HAS1, LO8961, NHLRC2, RPS23, GTF2H2, FLJ34077
- HAS1, LO8961, NHLRC2, RPS23, GTF2H2, CASP12
- HAS1, LO8961, NHLRC2, RPS23, GTF2H2, RP5-1077B9.4
- HAS1, LO8961, NHLRC2, RPS23, FLJ34077, CASP12
- HAS1, LO8961, NHLRC2, RPS23, FLJ34077, RP5-1077B9.4
- HAS1, LO8961, NHLRC2, RPS23, CASP12, RP5-1077B9.4
- HAS1, LO8961, NHLRC2, GTF2H2, FLJ34077, CASP12
- HAS1, LO8961, NHLRC2, GTF2H2, FLJ34077, RP5-1077B9.4
- HAS1, LO8961, NHLRC2, GTF2H2, CASP12, RP5-1077B9.4
- HAS1, LO8961, NHLRC2, FLJ34077, CASP12, RP5-1077B9.4
- HAS1, LO8961, ATP6VOE2, RPS23, GTF2H2, FLJ34077
- HAS1, LO8961, ATP6VOE2, RPS23, GTF2H2, CASP12
- HAS1, LO8961, ATP6VOE2, RPS23, GTF2H2, RP5-1077B9.4
- HAS1, LO8961, ATP6VOE2, RPS23, FLJ34077, CASP12
- HAS1, LO8961, ATP6VOE2, RPS23, FLJ34077, RP5-1077B9.4
- HAS1, LO8961, ATP6VOE2, RPS23, CASP12, RP5-1077B9.4
- HAS1, LO8961, ATP6VOE2, GTF2H2, FLJ34077, CASP12
- HAS1, LO8961, ATP6VOE2, GTF2H2, FLJ34077, RP5-1077B9.4
- HAS1, LO8961, ATP6VOE2, GTF2H2, CASP12, RP5-1077B9.4
- HAS1, LO8961, ATP6VOE2, FLJ34077, CASP12, RP5-1077B9.4
- HAS1, LO8961, RPS23, GTF2H2, FLJ34077, CASP12
- HAS1, LO8961, RPS23, GTF2H2, FLJ34077, RP5-1077B9.4
- HAS1, LO8961, RPS23, GTF2H2, CASP12, RP5-1077B9.4
- HAS1, LO8961, RPS23, FLJ34077, CASP12, RP5-1077B9.4
- HAS1, LO8961, GTF2H2, FLJ34077, CASP12, RP5-1077B9.4

- HAS1, NHLRC2, ATP6VOE2, RPS23, GTF2H2, FLJ34077
- HAS1, NHLRC2, ATP6VOE2, RPS23, GTF2H2, CASP12
- HAS1, NHLRC2, ATP6VOE2, RPS23, GTF2H2, RP5-1077B9.4
- HAS1, NHLRC2, ATP6VOE2, RPS23, FLJ34077, CASP12
- HAS1, NHLRC2, ATP6VOE2, RPS23, FLJ34077, RP5-1077B9.4
- HAS1, NHLRC2, ATP6VOE2, RPS23, CASP12, RP5-1077B9.4
- HAS1, NHLRC2, ATP6VOE2, GTF2H2, FLJ34077, CASP12
- HAS1, NHLRC2, ATP6VOE2, GTF2H2, FLJ34077, RP5-1077B9.4
- HAS1, NHLRC2, ATP6VOE2, GTF2H2, CASP12, RP5-1077B9.4
- HAS1, NHLRC2, ATP6VOE2, FLJ34077, CASP12, RP5-1077B9.4
- HAS1, NHLRC2, RPS23, GTF2H2, FLJ34077, CASP12
- HAS1, NHLRC2, RPS23, GTF2H2, FLJ34077, RP5-1077B9.4
- HAS1, NHLRC2, RPS23, GTF2H2, CASP12, RP5-1077B9.4
- HAS1, NHLRC2, RPS23, FLJ34077, CASP12, RP5-1077B9.4
- HAS1, NHLRC2, GTF2H2, FLJ34077, CASP12, RP5-1077B9.4
- HAS1, ATP6VOE2, RPS23, GTF2H2, FLJ34077, CASP12
- HAS1, ATP6VOE2, RPS23, GTF2H2, FLJ34077, RP5-1077B9.4
- HAS1, ATP6VOE2, RPS23, GTF2H2, CASP12, RP5-1077B9.4
- HAS1, ATP6VOE2, RPS23, FLJ34077, CASP12, RP5-1077B9.4
- HAS1, ATP6VOE2, GTF2H2, FLJ34077, CASP12, RP5-1077B9.4
- HAS1, RPS23, GTF2H2, FLJ34077, CASP12, RP5-1077B9.4
- LO8961, NHLRC2, ATP6VOE2, RPS23, GTF2H2, FLJ34077
- LO8961, NHLRC2, ATP6VOE2, RPS23, GTF2H2, CASP12
- LO8961, NHLRC2, ATP6VOE2, RPS23, GTF2H2, RP5-1077B9.4
- LO8961, NHLRC2, ATP6VOE2, RPS23, FLJ34077, CASP12
- LO8961, NHLRC2, ATP6VOE2, RPS23, FLJ34077, RP5-1077B9.4
- LO8961, NHLRC2, ATP6VOE2, RPS23, CASP12, RP5-1077B9.4
- LO8961, NHLRC2, ATP6VOE2, GTF2H2, FLJ34077, CASP12
- LO8961, NHLRC2, ATP6VOE2, GTF2H2, FLJ34077, RP5-1077B9.4
- LO8961, NHLRC2, ATP6VOE2, GTF2H2, CASP12, RP5-1077B9.4
- LO8961, NHLRC2, ATP6VOE2, FLJ34077, CASP12, RP5-1077B9.4
- LO8961, NHLRC2, RPS23, GTF2H2, FLJ34077, CASP12
- LO8961, NHLRC2, RPS23, GTF2H2, FLJ34077, RP5-1077B9.4
- LO8961, NHLRC2, RPS23, GTF2H2, CASP12, RP5-1077B9.4
- LO8961, NHLRC2, RPS23, FLJ34077, CASP12, RP5-1077B9.4
- LO8961, NHLRC2, GTF2H2, FLJ34077, CASP12, RP5-1077B9.4
- LO8961, ATP6V0E2, RPS23, GTF2H2, FLJ34077, CASP12
- LO8961, ATP6VOE2, RPS23, GTF2H2, FLJ34077, RP5-1077B9.4
- LO8961, ATP6VOE2, RPS23, GTF2H2, CASP12, RP5-1077B9.4
- LO8961, ATP6VOE2, RPS23, FLJ34077, CASP12, RP5-1077B9.4
- LO8961, ATP6VOE2, GTF2H2, FLJ34077, CASP12, RP5-1077B9.4
- LO8961, RPS23, GTF2H2, FLJ34077, CASP12, RP5-1077B9.4
- NHLRC2, ATP6VOE2, RPS23, GTF2H2, FLJ34077, CASP12
- NHLRC2, ATP6VOE2, RPS23, GTF2H2, FLJ34077, RP5-1077B9.4
- NHLRC2, ATP6VOE2, RPS23, GTF2H2, CASP12, RP5-1077B9.4
- NHLRC2, ATP6VOE2, RPS23, FLJ34077, CASP12, RP5-1077B9.4
- NHLRC2, ATP6VOE2, GTF2H2, FLJ34077, CASP12, RP5-1077B9.4
- NHLRC2, RPS23, GTF2H2, FLJ34077, CASP12, RP5-1077B9.4
- ATP6VOE2, RPS23, GTF2H2, FLJ34077, CASP12, RP5-1077B9.4

Combinations of 7 genes suitable for the differentiation of the stage of the tumor include:

- HAS1, LO8961, NHLRC2, ATP6VOE2, RPS23, GTF2H2, FLJ34077
- HAS1, LO8961, NHLRC2, ATP6VOE2, RPS23, GTF2H2, CASP12
- HAS1, LO8961, NHLRC2, ATP6VOE2, RPS23, GTF2H2, RP5-1077B9.4
- HAS1, LO8961, NHLRC2, ATP6VOE2, RPS23, FLJ34077, CASP12
- HAS1, LO8961, NHLRC2, ATP6VOE2, RPS23, FLJ34077, RP5-1077B9.4
- HAS1, LO8961, NHLRC2, ATP6VOE2, RPS23, CASP12, RP5-1077B9.4

- HAS1, LO8961, NHLRC2, ATP6VOE2, GTF2H2, FLJ34077, CASP12
- HAS1, LO8961, NHLRC2, ATP6VOE2, GTF2H2, FLJ34077, RP5-1077B9.4
- HAS1, LO8961, NHLRC2, ATP6VOE2, GTF2H2, CASP12, RP5-1077B9.4
- HAS1, LO8961, NHLRC2, ATP6VOE2, FLJ34077, CASP12, RP5-1077B9.4
- HAS1, LO8961, NHLRC2, RPS23, GTF2H2, FLJ34077, CASP12
- HAS1, LO8961, NHLRC2, RPS23, GTF2H2, FLJ34077, RP5-1077B9.4
- HAS1, LO8961, NHLRC2, RPS23, GTF2H2, CASP12, RP5-1077B9.4
- HAS1, LO8961 NHLRC2, RPS23, FLJ34077, CASP12, RP5-1077B9.4
- HAS1, LO8961, NHLRC2, GTF2H2, FLJ34077, CASP12, RP5-1077B9.4
- HAS1, LO8961, ATP6VOE2, RPS23, GTF2H2, FLJ34077, CASP12
- HAS1, LO8961, ATP6VOE2, RPS23, GTF2H2, FLJ34077, RP5-1077B9.4
- HAS1, LO8961, ATP6VOE2, RPS23, GTF2H2, CASP12, RP5-1077B9.4
- HAS1, LO8961, ATP6VOE2, RPS23, FLJ34077, CASP12, RP5-1077B9.4
- HAS1, LO8961, ATP6VOE2, GTF2H2, FLJ34077, CASP12, RP5-1077B9.4
- HAS1, LO8961, RPS23, GTF2H2, FLJ34077, CASP12, RP5-1077B9.4
- HAS1, NHLRC2, ATP6VOE2, RPS23, GTF2H2, FLJ34077, CASP12
- HAS1, NHLRC2, ATP6VOE2, RPS23, GTF2H2, FLJ34077, RP5-1077B9.4
- HAS1, NHLRC2, ATP6VOE2, RPS23, GTF2H2, CASP12, RP5-1077B9.4
- HAS1, NHLRC2, ATP6VOE2, RPS23, FLJ34077, CASP12, RP5-1077B9.4
- HAS1, NHLRC2, ATP6VOE2, GTF2H2, FLJ34077, CASP12, RP5-1077B9.4
- HAS1, NHLRC2, RPS23, GTF2H2, FLJ34077, CASP12, RP5-1077B9.4
- HAS1, ATP6VOE2, RPS23, GTF2H2, FLJ34077, CASP12, RP5-1077B9.4
- LO8961, NHLRC2, ATP6VOE2, RPS23, GTF2H2, FLJ34077, CASP12
- LO8961, NHLRC2, ATP6VOE2, RPS23, GTF2H2, FLJ34077, RP5-1077B9.4
- LO8961, NHLRC2, ATP6VOE2, RPS23, GTF2H2, CASP12, RP5-1077B9.4
- LO8961, NHLRC2, ATP6VOE2, RPS23, FLJ34077, CASP12, RP5-1077B9.4
- LO8961, NHLRC2, ATP6VOE2, GTF2H2, FLJ34077, CASP12, RP5-1077B9.4
- LO8961, NHLRC2, RPS23, GTF2H2, FLJ34077, CASP12, RP5-1077B9.4
- LO8961, ATP6VOE2, RPS23, GTF2H2, FLJ34077, CASP12, RP5-1077B9.4
- NHLRC2, ATP6VOE2, RPS23, GTF2H2, FLJ34077, CASP12, RP5-1077B9.4

Combinations of 8 genes suitable for the staging of the tumor include:

- HAS1, LO8961, NHLRC2, ATP6VOE2, RPS23, GTF2H2, FLJ34077, CASP12
- HAS1, LO8961, NHLRC2, ATP6VOE2, RPS23, GTF2H2, FLJ34077, RP5-1077B9.4
- HAS1, LO8961, NHLRC2, ATP6VOE2, RPS23, GTF2H2, CASP12, RP5-1077B9.4
- HAS1, LO8961, NHLRC2, ATP6VOE2, RPS23, FLJ34077, CASP12, RP5-1077B9.4
- HAS1, LO8961, NHLRC2, ATP6VOE2, GTF2H2, FLJ34077, CASP12, RP5-1077B9.4
- HAS1, LO8961, NHLRC2, RPS23, GTF2H2, FLJ34077, CASP12, RP5-1077B9.4
- HAS1, LO8961, ATP6VOE2, RPS23, GTF2H2, FLJ34077, CASP12, RP5-1077B9.4
- HAS1, NHLRC2, ATP6VOE2, RPS23, GTF2H2, FLJ34077, CASP12, RP5-1077B9.4
- LO8961, NHLRC2, ATP6VOE2, RPS23, GTF2H2, FLJ34077, CASP12, RP5-1077B9.4

Second staging method of the invention

The authors of the present invention have additionally identified a set of genes which allow distinguishing tumors in stage B from tumors with stage C with a reliability of 91.3%. Thus, in another aspect, the invention relates to a method (hereinafter second staging method of the invention) for differentiating stage B colorectal tumors from stage C colorectal tumors comprising determining in a said tumor the expression levels of genes MEA1, SPRY4, TLR8, SAMSN1, DGCR14, MRCL3, SMAD2, LAPTM4A, SFRS12, CD1B and FAM111A, wherein an altered expression level of said genes indicates that the tumor is a type B tumor according to Dukes' classification. The method preferably includes determining a high expression level of genes SPRY4 and CD1B and a reduced expression level of genes MEA1, TLR8, SAMSN1, DGCR14, MRCL3, SMAD2, LAPTM4A, SFRS12 and FAM111A in relation to a reference sample.

The genes the expression of which serves as an indicator in the second staging method of the invention include genes MEA1 (Male-enhanced antigen 1, NM_014623), SPRY4 (Sprouty homolog 4 NM_030964), TLR8 (Toll-like receptor 8, NM_016610), SAMSN1 (SAM domain, SH3 domain and nuclear location signals 1, NM_022136), DGCR14 (DiGeorge syndrome critical region gene 14, NM_022719), MRCL3 (Myosin regulatory light chain MRCL3, NM_006471), SMAD2 (Smad family member 2 4087 NM_001003652), LAPTM4A (Lysosomal-associated protein transmembrane 4, NM_014713), SFRS12 (Splicing factor, arginine/serine-rich 12, NM_139168), CD1B (CD1b molecule 910 NM_001764) and FAM111A (Family with sequence similarity 111, member A 63901 NM_022074).

The different steps and preferred forms of the invention (sample, methods for determining expression levels, etc.) are essentially carried out as has been previously described in the case of the diagnostic method of the invention.

Although the second staging method of the invention preferably comprises the determination of the expression levels of the previously mentioned 11 genes, the invention contemplates methods in which the staging is carried out by means of the determination of the expression levels of a subset of genes formed by 2, 3, 4, 5, 6, 7, 8, 9 or 10 genes.

Thus, combinations of 2 genes suitable for distinguishing between tumors that are in stage B and in stage C include:

- MEA1, SPRY4
- MEA1, TLR8
- MEA1, SAMSN1
- MEA1, DGCR14
- MEA1, MRCL3
- MEA1, SMAD2
- MEA1, LAPTM4A
- MEA1, SFRS12
- MEA1, CD1B
- MEA1, FAM111A
- SPRY4, TLR8
- SPRY4, SAMSN1
- SPRY4, DGCR14
- SPRY4, MRCL3
- SPRY4, SMAD2
- SPRY4, LAPTM4A
- SPRY4, SFRS12
- SPRY4, CD1B
- SPRY4, FAM111A
- TLR8, SAMSN1
- TLR8, DGCR14
- TLR8, MRCL3
- TLR8, SMAD2
- TLR8, LAPTM4A
- TLR8, SFRS12
- TLR8, CD1B
- TLR8, FAM111A
- SAMSN1, DGCR14
- SAMSN1, MRCL3
- SAMSN1, SMAD2
- SAMSN1, LAPTM4A
- SAMSN1, SFRS12
- SAMSN1, CD1B
- SAMSN1, FAM111A
- DGCR14, MRCL3
- DGCR14, SMAD2
- DGCR14, LAPTM4A
- DGCR14, SFRS12
- DGCR14, CD1B
- DGCR14, FAM111A
- MRCL3, SMAD2
- MRCL3, LAPTM4A
- MRCL3, SFRS12
- MRCL3, CD1B
- MRCL3, FAM111A
- SMAD2, LAPTM4A
- SMAD2, SFRS12
- SMAD2, CD1B

- SMAD2, FAM111A
- LAPTM4A, SFRS12
- LAPTM4A, CD1B
- LAPTM4A, FAM111A
- SFRS12, CD1B
- SFRS12, FAM111A
- CD1B, FAM111A

Thus, combinations of 3 genes suitable for the staging of the tumor include

- MEA1, SPRY4, TLR8
- MEA1, SPRY4, SAMSN1
- MEA1, SPRY4, DGCR14
- MEA1, SPRY4, MRCL3
- MEA1, SPRY4, SMAD2
- MEA1, SPRY4, LAPTM4A
- MEA1, SPRY4, SFRS12
- MEA1, SPRY4, CD1B
- MEA1, SPRY4, FAM111A
- MEA1, TLR8, SAMSN1
- MEA1, TLR8, DGCR14
- MEA1, TLR8, MRCL3
- MEA1, TLR8, SMAD2
- MEA1, TLR8, LAPTM4A
- MEA1, TLR8, SFRS12
- MEA1, TLR8, CD1B
- MEA1, TLR8, FAM111A
- MEA1, SAMSN1, DGCR14
- MEA1, SAMSN1, MRCL3
- MEA1, SAMSN1, SMAD2
- MEA1, SAMSN1, LAPTM4A
- MEA1, SAMSN1, SFRS12
- MEA1, SAMSN1, CD1B
- MEA1, SAMSN1, FAM111A
- MEA1, DGCR14, MRCL3
- MEA1, DGCR14, SMAD2
- MEA1, DGCR14, LAPTM4A
- MEA1, DGCR14, SFRS12
- MEA1, DGCR14, CD1B
- MEA1, DGCR14, FAM111A
- MEA1, MRCL3, SMAD2
- MEA1, MRCL3, LAPTM4A
- MEA1, MRCL3, SFRS12
- MEA1, MRCL3, CD1B
- MEA1, MRCL3, FAM111A
- MEA1, SMAD2, LAPTM4A
- MEA1, SMAD2, SFRS12
- MEA1, SMAD2, CD1B
- MEA1, SMAD2, FAM111A
- MEA1, LAPTM4A, SFRS12
- MEA1, LAPTM4A, CD1B
- MEA1, LAPTM4A, FAM111A
- MEA1, SFRS12, CD1B
- MEA1, SFRS12, FAM111A
- MEA1, CD1B, FAM111A
- SPRY4, TLR8, SAMSN1
- SPRY4, TLR8, DGCR14
- SPRY4, TLR8, MRCL3

- SPRY4, TLR8, SMAD2
- SPRY4, TLR8, LAPTM4A
- SPRY4, TLR8, SFRS12
- SPRY4, TLR8, CD1B
- SPRY4, TLR8, FAM111A
- SPRY4, SAMSN1, DGCR14
- SPRY4, SAMSN1, MRCL3
- SPRY4, SAMSN1, SMAD2
- SPRY4, SAMSN1, LAPTM4A
- SPRY4, SAMSN1, SFRS12
- SPRY4, SAMSN1, CD1B
- SPRY4, SAMSN1, FAM111A
- SPRY4, DGCR14, MRCL3
- SPRY4, DGCR14, SMAD2
- SPRY4, DGCR14, LAPTM4A
- SPRY4, DGCR14, SFRS12
- SPRY4, DGCR14, CD1B
- SPRY4, DGCR14, FAM111A
- SPRY4, MRCL3, SMAD2
- SPRY4, MRCL3, LAPTM4A
- SPRY4, MRCL3, SFRS12
- SPRY4, MRCL3, CD1B
- SPRY4, MRCL3, FAM111A
- SPRY4, SMAD2, LAPTM4A
- SPRY4, SMAD2, SFRS12
- SPRY4, SMAD2, CD1B
- SPRY4, SMAD2, FAM111A
- SPRY4, LAPTM4A, SFRS12
- SPRY4, LAPTM4A, CD1B
- SPRY4, LAPTM4A, FAM111A
- SPRY4, SFRS12, CD1B
- SPRY4, SFRS12, FAM111A
- SPRY4, CD1B, FAM111A
- TLR8, SAMSN1, DGCR14
- TLR8, SAMSN1, MRCL3
- TLR8, SAMSN1, SMAD2
- TLR8, SAMSN1, LAPTM4A
- TLR8, SAMSN1, SFRS12
- TLR8, SAMSN1, CD1B
- TLR8, SAMSN1, FAM111A
- TLR8, DGCR14, MRCL3
- TLR8, DGCR14, SMAD2
- TLR8, DGCR14, LAPTM4A
- TLR8, DGCR14, SFRS12
- TLR8, DGCR14, CD1B
- TLR8, DGCR14, FAM111A
- TLR8, MRCL3, SMAD2
- TLR8, MRCL3, LAPTM4A
- TLR8, MRCL3, SFRS12
- TLR8, MRCL3, CD1B
- TLR8, MRCL3, FAM111A
- TLR8, SMAD2, LAPTM4A
- TLR8, SMAD2, SFRS12
- TLR8, SMAD2, CD1B
- TLR8, SMAD2, FAM111A
- TLR8, LAPTM4A, SFRS12
- TLR8, LAPTM4A, CD1B
- TLR8, LAPTM4A, FAM111A

- TLR8, SFRS12, CD1B
- TLR8, SFRS12, FAM111A
- TLR8, CD1B, FAM111A
- SAMSN1, DGCR14, MRCL3
- SAMSN1, DGCR14, SMAD2
- SAMSN1, DGCR14, LAPTM4A
- SAMSN1, DGCR14, SFRS12
- SAMSN1, DGCR14, CD1B
- SAMSN1, DGCR14, FAM111A
- SAMSN1, MRCL3, SMAD2
- SAMSN1, MRCL3, LAPTM4A
- SAMSN1, MRCL3, SFRS12
- SAMSN1, MRCL3, CD1B
- SAMSN1, MRCL3, FAM111A
- SAMSN1, SMAD2, LAPTM4A
- SAMSN1, SMAD2, SFRS12
- SAMSN1, SMAD2, CD1B
- SAMSN1, SMAD2, FAM111A
- SAMSN1, LAPTM4A, SFRS12
- SAMSN1, LAPTM4A, CD1B
- SAMSN1, LAPTM4A, FAM111A
- SAMSN1, SFRS12, CD1B
- SAMSN1, SFRS12, FAM111A
- SAMSN1, CD1B, FAM111A
- DGCR14, MRCL3, SMAD2
- DGCR14, MRCL3, LAPTM4A
- DGCR14, MRCL3, SFRS12
- DGCR14, MRCL3, CD1B
- DGCR14, MRCL3, FAM111A
- DGCR14, SMAD2, LAPTM4A
- DGCR14, SMAD2, SFRS12
- DGCR14, SMAD2, CD1B
- DGCR14, SMAD2, FAM111A
- DGCR14, LAPTM4A, SFRS12
- DGCR14, LAPTM4A, CD1B
- DGCR14, LAPTM4A, FAM111A
- DGCR14, SFRS12, CD1B
- DGCR14, SFRS12, FAM111A
- DGCR14, CD1B, FAM111A
- MRCL3, SMAD2, LAPTM4A
- MRCL3, SMAD2, SFRS12
- MRCL3, SMAD2, CD1B
- MRCL3, SMAD2, FAM111A
- MRCL3, LAPTM4A, SFRS12
- MRCL3, LAPTM4A, CD1B
- MRCL3, LAPTM4A, FAM111A
- MRCL3, SFRS12, CD1B
- MRCL3, SFRS12, FAM111A
- MRCL3, CD1B, FAM111A
- SMAD2, LAPTM4A, SFRS12
- SMAD2, LAPTM4A, CD1B
- SMAD2, LAPTM4A, FAM111A
- SMAD2, SFRS12, CD1B
- SMAD2, SFRS12, FAM111A
- SMAD2, CD1B, FAM111A
- LAPTM4A, SFRS12, CD1B
- LAPTM4A, SFRS12, FAM111A
- LAPTM4A, CD1B, FAM111A

- SFRS12, CD1B, FAM111A

Thus, combinations of 4 genes suitable for the staging of the tumor include

- MEA1, SPRY4, TLR8, SAMSN1
- MEA1, SPRY4, TLR8, DGCR14
- MEA1, SPRY4, TLR8, MRCL3
- MEA1, SPRY4, TLR8, SMAD2
- MEA1, SPRY4, TLR8, LAPTM4A
- MEA1, SPRY4, TLR8, SFRS12
- MEA1, SPRY4, TLR8, CD1B
- MEA1, SPRY4, TLR8, FAM111A
- MEA1, SPRY4, SAMSN1, DGCR14
- MEA1, SPRY4, SAMSN1, MRCL3
- MEA1, SPRY4, SAMSN1, SMAD2
- MEA1, SPRY4, SAMSN1, LAPTM4A
- MEA1, SPRY4, SAMSN1, SFRS12
- MEA1, SPRY4, SAMSN1, CD1B
- MEA1, SPRY4, SAMSN1, FAM111A
- MEA1, SPRY4, DGCR14, MRCL3
- MEA1, SPRY4, DGCR14, SMAD2
- MEA1, SPRY4, DGCR14, LAPTM4A
- MEA1, SPRY4, DGCR14, SFRS12
- MEA1, SPRY4, DGCR14, CD1B
- MEA1, SPRY4, DGCR14, FAM111A
- MEA1, SPRY4, MRCL3, SMAD2
- MEA1, SPRY4, MRCL3, LAPTM4A
- MEA1, SPRY4, MRCL3, SFRS12
- MEA1, SPRY4, MRCL3, CD1B
- MEA1, SPRY4, MRCL3, FAM111A
- MEA1, SPRY4, SMAD2, LAPTM4A
- MEA1, SPRY4, SMAD2, SFRS12
- MEA1, SPRY4, SMAD2, CD1B
- MEA1, SPRY4, SMAD2, FAM111A
- MEA1, SPRY4, LAPTM4A, SFRS12
- MEA1, SPRY4, LAPTM4A, CD1B
- MEA1, SPRY4, LAPTM4A, FAM111A
- MEA1, SPRY4, SFRS12, CD1B
- MEA1, SPRY4, SFRS12, FAM111A
- MEA1, SPRY4, CD1B, FAM111A
- MEA1, TLR8, SAMSN1, DGCR14
- MEA1, TLR8, SAMSN1, MRCL3
- MEA1, TLR8, SAMSN1, SMAD2
- MEA1, TLR8, SAMSN1, LAPTM4A
- MEA1, TLR8, SAMSN1, SFRS12
- MEA1, TLR8, SAMSN1, CD1B
- MEA1, TLR8, SAMSN1, FAM111A
- MEA1, TLR8, DGCR14, MRCL3
- MEA1, TLR8, DGCR14, SMAD2
- MEA1, TLR8, DGCR14, LAPTM4A
- MEA1, TLR8, DGCR14, SFRS12
- MEA1, TLR8, DGCR14, CD1B
- MEA1, TLR8, DGCR14, FAM111A
- MEA1, TLR8, MRCL3, SMAD2
- MEA1, TLR8, MRCL3, LAPTM4A
- MEA1, TLR8, MRCL3, SFRS12
- MEA1, TLR8, MRCL3, CD1B
- MEA1, TLR8, MRCL3, FAM111A

- MEA1, TLR8, SMAD2, LAPTM4A
- MEA1, TLR8, SMAD2, SFRS12
- MEA1, TLR8, SMAD2, CD1B
- MEA1, TLR8, SMAD2, FAM111A
- MEA1, TLR8, LAPTM4A, SFRS12
- MEA1, TLR8, LAPTM4A, CD1B
- MEA1, TLR8, LAPTM4A, FAM111A
- MEA1, TLR8, SFRS12, CD1B
- MEA1, TLR8, SFRS12, FAM111A
- MEA1, TLR8, CD1B, FAM111A
- MEA1, SAMSN1, DGCR14, MRCL3
- MEA1, SAMSN1, DGCR14, SMAD2
- MEA1, SAMSN1, DGCR14, LAPTM4A
- MEA1, SAMSN1, DGCR14, SFRS12
- MEA1, SAMSN1, DGCR14, CD1B
- MEA1, SAMSN1, DGCR14, FAM111A
- MEA1, SAMSN1, MRCL3, SMAD2
- MEA1, SAMSN1, MRCL3, LAPTM4A
- MEA1, SAMSN1, MRCL3, SFRS12
- MEA1, SAMSN1, MRCL3, CD1B
- MEA1, SAMSN1, MRCL3, FAM111A
- MEA1, SAMSN1, SMAD2, LAPTM4A
- MEA1, SAMSN1, SMAD2, SFRS12
- MEA1, SAMSN1, SMAD2, CD1B
- MEA1, SAMSN1, SMAD2, FAM111A
- MEA1, SAMSN1, LAPTM4A, SFRS12
- MEA1, SAMSN1, LAPTM4A, CD1B
- MEA1, SAMSN1, LAPTM4A, FAM111A
- MEA1, SAMSN1, SFRS12, CD1B
- MEA1, SAMSN1, SFRS12, FAM111A
- MEA1, SAMSN1, CD1B, FAM111A
- MEA1, DGCR14, MRCL3, SMAD2
- MEA1, DGCR14, MRCL3, LAPTM4A
- MEA1, DGCR14, MRCL3, SFRS12
- MEA1, DGCR14, MRCL3, CD1B
- MEA1, DGCR14, MRCL3, FAM111A
- MEA1, DGCR14, SMAD2, LAPTM4A
- MEA1, DGCR14, SMAD2, SFRS12
- MEA1, DGCR14, SMAD2, CD1B
- MEA1, DGCR14, SMAD2, FAM111A
- MEA1, DGCR14, LAPTM4A, SFRS12
- MEA1, DGCR14, LAPTM4A, CD1B
- MEA1, DGCR14, LAPTM4A, FAM111A
- MEA1, DGCR14, SFRS12, CD1B
- MEA1, DGCR14, SFRS12, FAM111A
- MEA1, DGCR14, CD1B, FAM111A
- MEA1, MRCL3, SMAD2, LAPTM4A
- MEA1, MRCL3, SMAD2, SFRS12
- MEA1, MRCL3, SMAD2, CD1B
- MEA1, MRCL3, SMAD2, FAM111A
- MEA1, MRCL3, LAPTM4A, SFRS12
- MEA1, MRCL3, LAPTM4A, CD1B
- MEA1, MRCL3, LAPTM4A, FAM111A
- MEA1, MRCL3, SFRS12, CD1B
- MEA1, MRCL3, SFRS12, FAM111A
- MEA1, MRCL3, CD1B, FAM111A
- MEA1, SMAD2, LAPTM4A, SFRS12
- MEA1, SMAD2, LAPTM4A, CD1B

- MEA1, SMAD2, LAPTM4A, FAM111A
- MEA1, SMAD2, SFRS12, CD1B
- MEA1, SMAD2, SFRS12, FAM111A
- MEA1, SMAD2, CD1B, FAM111A
- MEA1, LAPTM4A, SFRS12, CD1B
- MEA1, LAPTM4A, SFRS12, FAM111A
- MEA1, LAPTM4A, CD1B, FAM111A
- MEA1, SFRS12, CD1B, FAM111A
- SPRY4, TLR8, SAMSN1, DGCR14
- SPRY4, TLR8, SAMSN1, MRCL3
- SPRY4, TLR8, SAMSN1, SMAD2
- SPRY4, TLR8, SAMSN1, LAPTM4A
- SPRY4, TLR8, SAMSN1, SFRS12
- SPRY4, TLR8, SAMSN1, CD1B
- SPRY4, TLR8, SAMSN1, FAM111A
- SPRY4, TLR8, DGCR14, MRCL3
- SPRY4, TLR8, DGCR14, SMAD2
- SPRY4, TLR8, DGCR14, LAPTM4A
- SPRY4, TLR8, DGCR14, SFRS12
- SPRY4, TLR8, DGCR14, CD1B
- SPRY4, TLR8, DGCR14, FAM111A
- SPRY4, TLR8, MRCL3, SMAD2
- SPRY4, TLR8, MRCL3, LAPTM4A
- SPRY4, TLR8, MRCL3, SFRS12
- SPRY4, TLR8, MRCL3, CD1B
- SPRY4, TLR8, MRCL3, FAM111A
- SPRY4, TLR8, SMAD2, LAPTM4A
- SPRY4, TLR8, SMAD2, SFRS12
- SPRY4, TLR8, SMAD2, CD1B
- SPRY4, TLR8, SMAD2, FAM111A
- SPRY4, TLR8, LAPTM4A, SFRS12
- SPRY4, TLR8, LAPTM4A, CD1B
- SPRY4, TLR8, LAPTM4A, FAM111A
- SPRY4, TLR8, SFRS12, CD1B
- SPRY4, TLR8, SFRS12, FAM111A
- SPRY4, TLR8, CD1B, FAM111A
- SPRY4, SAMSN1, DGCR14, MRCL3
- SPRY4, SAMSN1, DGCR14, SMAD2
- SPRY4, SAMSN1, DGCR14, LAPTM4A
- SPRY4, SAMSN1, DGCR14, SFRS12
- SPRY4, SAMSN1, DGCR14, CD1B
- SPRY4, SAMSN1, DGCR14, FAM111A
- SPRY4, SAMSN1, MRCL3, SMAD2
- SPRY4, SAMSN1, MRCL3, LAPTM4A
- SPRY4, SAMSN1, MRCL3, SFRS12
- SPRY4, SAMSN1, MRCL3, CD1B
- SPRY4, SAMSN1, MRCL3, FAM111A
- SPRY4, SAMSN1, SMAD2, LAPTM4A
- SPRY4, SAMSN1, SMAD2, SFRS12
- SPRY4, SAMSN1, SMAD2, CD1B
- SPRY4, SAMSN1, SMAD2, FAM111A
- SPRY4, SAMSN1, LAPTM4A, SFRS12
- SPRY4, SAMSN1, LAPTM4A, CD1B
- SPRY4, SAMSN1, LAPTM4A, FAM111A
- SPRY4, SAMSN1, SFRS12, CD1B
- SPRY4, SAMSN1, SFRS12, FAM111A
- SPRY4, SAMSN1, CD1B, FAM111A
- SPRY4, DGCR14, MRCL3, SMAD2

- SPRY4, DGCR14, MRCL3, LAPTM4A
- SPRY4, DGCR14, MRCL3, SFRS12
- SPRY4, DGCR14, MRCL3, CD1B
- SPRY4, DGCR14, MRCL3, FAM111A
- SPRY4, DGCR14, SMAD2, LAPTM4A
- SPRY4, DGCR14, SMAD2, SFRS12
- SPRY4, DGCR14, SMAD2, CD1B
- SPRY4, DGCR14, SMAD2, FAM111A
- SPRY4, DGCR14, LAPTM4A, SFRS12
- SPRY4, DGCR14, LAPTM4A, CD1B
- SPRY4, DGCR14, LAPTM4A, FAM111A
- SPRY4, DGCR14, SFRS12, CD1B
- SPRY4, DGCR14, SFRS12, FAM111A
- SPRY4, DGCR14, CD1B, FAM111A
- SPRY4, MRCL3, SMAD2, LAPTM4A
- SPRY4, MRCL3, SMAD2, SFRS12
- SPRY4, MRCL3, SMAD2, CD1B
- SPRY4, MRCL3, SMAD2, FAM111A
- SPRY4, MRCL3, LAPTM4A, SFRS12
- SPRY4, MRCL3, LAPTM4A, CD1B
- SPRY4, MRCL3, LAPTM4A, FAM111A
- SPRY4, MRCL3, SFRS12, CD1B
- SPRY4, MRCL3, SFRS12, FAM111A
- SPRY4, MRCL3, CD1B, FAM111A
- SPRY4, SMAD2, LAPTM4A, SFRS12
- SPRY4, SMAD2, LAPTM4A, CD1B
- SPRY4, SMAD2, LAPTM4A, FAM111A
- SPRY4, SMAD2, SFRS12, CD1B
- SPRY4, SMAD2, SFRS12, FAM111A
- SPRY4, SMAD2, CD1B, FAM111A
- SPRY4, LAPTM4A, SFRS12, CD1B
- SPRY4, LAPTM4A, SFRS12, FAM111A
- SPRY4, LAPTM4A, CD1B, FAM111A
- SPRY4, SFRS12, CD1B, FAM111A
- TLR8, SAMSN1, DGCR14, MRCL3
- TLR8, SAMSN1, DGCR14, SMAD2
- TLR8, SAMSN1, DGCR14, LAPTM4A
- TLR8, SAMSN1, DGCR14, SFRS12
- TLR8, SAMSN1, DGCR14, CD1B
- TLR8, SAMSN1, DGCR14, FAM111A
- TLR8, SAMSN1, MRCL3, SMAD2
- TLR8, SAMSN1, MRCL3, LAPTM4A
- TLR8, SAMSN1, MRCL3, SFRS12
- TLR8, SAMSN1, MRCL3, CD1B
- TLR8, SAMSN1, MRCL3, FAM111A
- TLR8, SAMSN1, SMAD2, LAPTM4A
- TLR8, SAMSN1, SMAD2, SFRS12
- TLR8, SAMSN1, SMAD2, CD1B
- TLR8, SAMSN1, SMAD2, FAM111A
- TLR8, SAMSN1, LAPTM4A, SFRS12
- TLR8, SAMSN1, LAPTM4A, CD1B
- TLR8, SAMSN1, LAPTM4A, FAM111A
- TLR8, SAMSN1, SFRS12, CD1B
- TLR8, SAMSN1, SFRS12, FAM111A
- TLR8, SAMSN1, CD1B, FAM111A
- TLR8, DGCR14, MRCL3, SMAD2
- TLR8, DGCR14, MRCL3, LAPTM4A
- TLR8, DGCR14, MRCL3, SFRS12

- TLR8, DGCR14, MRCL3, CD1B
- TLR8, DGCR14, MRCL3, FAM111A
- TLR8, DGCR14, SMAD2, LAPTM4A
- TLR8, DGCR14, SMAD2, SFRS12
- TLR8, DGCR14, SMAD2, CD1B
- TLR8, DGCR14, SMAD2, FAM111A
- TLR8, DGCR14, LAPTM4A, SFRS12
- TLR8, DGCR14, LAPTM4A, CD1B
- TLR8, DGCR14, LAPTM4A, FAM111A
- TLR8, DGCR14, SFRS12, CD1B
- TLR8, DGCR14, SFRS12, FAM111A
- TLR8, DGCR14, CD1B, FAM111A
- TLR8, MRCL3, SMAD2, LAPTM4A
- TLR8, MRCL3, SMAD2, SFRS12
- TLR8, MRCL3, SMAD2, CD1B
- TLR8, MRCL3, SMAD2, FAM111A
- TLR8, MRCL3, LAPTM4A, SFRS12
- TLR8, MRCL3, LAPTM4A, CD1B
- TLR8, MRCL3, LAPTM4A, FAM111A
- TLR8, MRCL3, SFRS12, CD1B
- TLR8, MRCL3, SFRS12, FAM111A
- TLR8, MRCL3, CD1B, FAM111A
- TLR8, SMAD2, LAPTM4A, SFRS12
- TLR8, SMAD2, LAPTM4A, CD1B
- TLR8, SMAD2, LAPTM4A, FAM111A
- TLR8, SMAD2, SFRS12, CD1B
- TLR8, SMAD2, SFRS12, FAM111A
- TLR8, SMAD2, CD1B, FAM111A
- TLR8, LAPTM4A, SFRS12, CD1B
- TLR8, LAPTM4A, SFRS12, FAM111A
- TLR8, LAPTM4A, CD1B, FAM111A
- TLR8, SFRS12, CD1B, FAM111A
- SAMSN1, DGCR14, MRCL3, SMAD2
- SAMSN1, DGCR14, MRCL3, LAPTM4A
- SAMSN1, DGCR14, MRCL3, SFRS12
- SAMSN1, DGCR14, MRCL3, CD1B
- SAMSN1, DGCR14, MRCL3, FAM111A
- SAMSN1, DGCR14, SMAD2, LAPTM4A
- SAMSN1, DGCR14, SMAD2, SFRS12
- SAMSN1, DGCR14, SMAD2, CD1B
- SAMSN1, DGCR14, SMAD2, FAM111A
- SAMSN1, DGCR14, LAPTM4A, SFRS12
- SAMSN1, DGCR14, LAPTM4A, CD1B
- SAMSN1, DGCR14, LAPTM4A, FAM111A
- SAMSN1, DGCR14, SFRS12, CD1B
- SAMSN1, DGCR14, SFRS12, FAM111A
- SAMSN1, DGCR14, CD1B, FAM111A
- SAMSN1, MRCL3, SMAD2, LAPTM4A
- SAMSN1, MRCL3, SMAD2, SFRS12
- SAMSN1, MRCL3, SMAD2, CD1B
- SAMSN1, MRCL3, SMAD2, FAM111A
- SAMSN1, MRCL3, LAPTM4A, SFRS12
- SAMSN1, MRCL3, LAPTM4A, CD1B
- SAMSN1, MRCL3, LAPTM4A, FAM111A
- SAMSN1, MRCL3, SFRS12, CD1B
- SAMSN1, MRCL3, SFRS12, FAM111A
- SAMSN1, MRCL3, CD1B, FAM111A
- SAMSN1, SMAD2, LAPTM4A, SFRS12

- SAMSN1, SMAD2, LAPTM4A, CD1B
- SAMSN1, SMAD2, LAPTM4A, FAM111A
- SAMSN1, SMAD2, SFRS12, CD1B
- SAMSN1, SMAD2, SFRS12, FAM111A
- SAMSN1, SMAD2, CD1B, FAM111A
- SAMSN1, LAPTM4A, SFRS12, CD1B
- SAMSN1, LAPTM4A, SFRS12, FAM111A
- SAMSN1, LAPTM4A, CD1B, FAM111A
- SAMSN1, SFRS12, CD1B, FAM111A
- DGCR14, MRCL3, SMAD2, LAPTM4A
- DGCR14, MRCL3, SMAD2, SFRS12
- DGCR14, MRCL3, SMAD2, CD1B
- DGCR14, MRCL3, SMAD2, FAM111A
- DGCR14, MRCL3, LAPTM4A, SFRS12
- DGCR14, MRCL3, LAPTM4A, CD1B
- DGCR14, MRCL3, LAPTM4A, FAM111A
- DGCR14, MRCL3, SFRS12, CD1B
- DGCR14, MRCL3, SFRS12, FAM111A
- DGCR14, MRCL3, CD1B, FAM111A
- DGCR14, SMAD2, LAPTM4A, SFRS12
- DGCR14, SMAD2, LAPTM4A, CD1B
- DGCR14, SMAD2, LAPTM4A, FAM111A
- DGCR14, SMAD2, SFRS12, CD1B
- DGCR14, SMAD2, SFRS12, FAM111A
- DGCR14, SMAD2, CD1B, FAM111A
- DGCR14, LAPTM4A, SFRS12, CD1B
- DGCR14, LAPTM4A, SFRS12, FAM111A
- DGCR14, LAPTM4A, CD1B, FAM111A
- DGCR14, SFRS12, CD1B, FAM111A
- MRCL3, SMAD2, LAPTM4A, SFRS12
- MRCL3, SMAD2, LAPTM4A, CD1B
- MRCL3, SMAD2, LAPTM4A, FAM111A
- MRCL3, SMAD2, SFRS12, CD1B
- MRCL3, SMAD2, SFRS12, FAM111A
- MRCL3, SMAD2, CD1B, FAM111A
- MRCL3, LAPTM4A, SFRS12, CD1B
- MRCL3, LAPTM4A, SFRS12, FAM111A
- MRCL3, LAPTM4A, CD1B, FAM111A
- MRCL3, SFRS12, CD1B, FAM111A
- SMAD2, LAPTM4A, SFRS12, CD1B
- SMAD2, LAPTM4A, SFRS12, FAM111A
- SMAD2, LAPTM4A, CD1B, FAM111A
- SMAD2, SFRS12, CD1B, FAM111A
- LAPTM4A, SFRS12, CD1B, FAM111A

Thus, combinations of 5 genes suitable for the staging of the tumor include

- MEA1, SPRY4, TLR8, SAMSN1, DGCR14
- MEA1, SPRY4, TLR8, SAMSN1, MRCL3
- MEA1, SPRY4, TLR8, SAMSN1, SMAD2
- MEA1, SPRY4, TLR8, SAMSN1, LAPTM4A
- MEA1, SPRY4, TLR8, SAMSN1, SFRS12
- MEA1, SPRY4, TLR8, SAMSN1, CD1B
- MEA1, SPRY4, TLR8, SAMSN1, FAM111A
- MEA1, SPRY4, TLR8, DGCR14, MRCL3
- MEA1, SPRY4, TLR8, DGCR14, SMAD2
- MEA1, SPRY4, TLR8, DGCR14, LAPTM4A
- MEA1, SPRY4, TLR8, DGCR14, SFRS12

- MEA1, SPRY4, TLR8, DGCR14, CD1B
- MEA1, SPRY4, TLR8, DGCR14, FAM111A
- MEA1, SPRY4, TLR8, MRCL3, SMAD2
- MEA1, SPRY4, TLR8, MRCL3, LAPTM4A
- MEA1, SPRY4, TLR8, MRCL3, SFRS12
- MEA1, SPRY4, TLR8, MRCL3, CD1B
- MEA1, SPRY4, TLR8, MRCL3, FAM111A
- MEA1, SPRY4, TLR8, SMAD2, LAPTM4A
- MEA1, SPRY4, TLR8, SMAD2, SFRS12
- MEA1, SPRY4, TLR8, SMAD2, CD1B
- MEA1, SPRY4, TLR8, SMAD2, FAM111A
- MEA1, SPRY4, TLR8, LAPTM4A, SFRS12
- MEA1, SPRY4, TLR8, LAPTM4A, CD1B
- MEA1, SPRY4, TLR8, LAPTM4A, FAM111A
- MEA1, SPRY4, TLR8, SFRS12, CD1B
- MEA1, SPRY4, TLR8, SFRS12, FAM111A
- MEA1, SPRY4, TLR8, CD1B, FAM111A
- MEA1, SPRY4, SAMSN1, DGCR14, MRCL3
- MEA1, SPRY4, SAMSN1, DGCR14, SMAD2
- MEA1, SPRY4, SAMSN1, DGCR14, LAPTM4A
- MEA1, SPRY4, SAMSN1, DGCR14, SFRS12
- MEA1, SPRY4, SAMSN1, DGCR14, CD1B
- MEA1, SPRY4, SAMSN1, DGCR14, FAM111A
- MEA1, SPRY4, SAMSN1, MRCL3, SMAD2
- MEA1, SPRY4, SAMSN1, MRCL3, LAPTM4A
- MEA1, SPRY4, SAMSN1, MRCL3, SFRS12
- MEA1, SPRY4, SAMSN1, MRCL3, CD1B
- MEA1, SPRY4, SAMSN1, MRCL3, FAM111A
- MEA1, SPRY4, SAMSN1, SMAD2, LAPTM4A
- MEA1, SPRY4, SAMSN1, SMAD2, SFRS12
- MEA1, SPRY4, SAMSN1, SMAD2, CD1B
- MEA1, SPRY4, SAMSN1, SMAD2, FAM111A
- MEA1, SPRY4, SAMSN1, LAPTM4A, SFRS12
- MEA1, SPRY4, SAMSN1, LAPTM4A, CD1B
- MEA1, SPRY4, SAMSN1, LAPTM4A, FAM111A
- MEA1, SPRY4, SAMSN1, SFRS12, CD1B
- MEA1, SPRY4, SAMSN1, SFRS12, FAM111A
- MEA1, SPRY4, SAMSN1, CD1B, FAM111A
- MEA1, SPRY4, DGCR14, MRCL3, SMAD2
- MEA1, SPRY4, DGCR14, MRCL3, LAPTM4A
- MEA1, SPRY4, DGCR14, MRCL3, SFRS12
- MEA1, SPRY4, DGCR14, MRCL3, CD1B
- MEA1, SPRY4, DGCR14, MRCL3, FAM111A
- MEA1, SPRY4, DGCR14, SMAD2, LAPTM4A
- MEA1, SPRY4, DGCR14, SMAD2, SFRS12
- MEA1, SPRY4, DGCR14, SMAD2, CD1B
- MEA1, SPRY4, DGCR14, SMAD2, FAM111A
- MEA1, SPRY4, DGCR14, LAPTM4A, SFRS12
- MEA1, SPRY4, DGCR14, LAPTM4A, CD1B
- MEA1, SPRY4, DGCR14, LAPTM4A, FAM111A
- MEA1, SPRY4, DGCR14, SFRS12, CD1B
- MEA1, SPRY4, DGCR14, SFRS12, FAM111A
- MEA1, SPRY4, DGCR14, CD1B, FAM111A
- MEA1, SPRY4, MRCL3, SMAD2, LAPTM4A
- MEA1, SPRY4, MRCL3, SMAD2, SFRS12
- MEA1, SPRY4, MRCL3, SMAD2, CD1B
- MEA1, SPRY4, MRCL3, SMAD2, FAM111A
- MEA1, SPRY4, MRCL3, LAPTM4A, SFRS12

- MEA1, SPRY4, MRCL3, LAPTM4A, CD1B
- MEA1, SPRY4, MRCL3, LAPTM4A, FAM111A
- MEA1, SPRY4, MRCL3, SFRS12, CD1B
- MEA1, SPRY4, MRCL3, SFRS12, FAM111A
- MEA1, SPRY4, MRCL3, CD1B, FAM111A
- MEA1, SPRY4, SMAD2, LAPTM4A, SFRS12
- MEA1, SPRY4, SMAD2, LAPTM4A, CD1B
- MEA1, SPRY4, SMAD2, LAPTM4A, FAM111A
- MEA1, SPRY4, SMAD2, SFRS12, CD1B
- MEA1, SPRY4, SMAD2, SFRS12, FAM111A
- MEA1, SPRY4, SMAD2, CD1B, FAM111A
- MEA1, SPRY4, LAPTM4A, SFRS12, CD1B
- MEA1, SPRY4, LAPTM4A, SFRS12, FAM111A
- MEA1, SPRY4, LAPTM4A, CD1B, FAM111A
- MEA1, SPRY4, SFRS12, CD1B, FAM111A
- MEA1, TLR8, SAMSN1, DGCR14, MRCL3
- MEA1, TLR8, SAMSN1, DGCR14, SMAD2
- MEA1, TLR8, SAMSN1, DGCR14, LAPTM4A
- MEA1, TLR8, SAMSN1, DGCR14, SFRS12
- MEA1, TLR8, SAMSN1, DGCR14, CD1B
- MEA1, TLR8, SAMSN1, DGCR14, FAM111A
- MEA1, TLR8, SAMSN1, MRCL3, SMAD2
- MEA1, TLR8, SAMSN1, MRCL3, LAPTM4A
- MEA1, TLR8, SAMSN1, MRCL3, SFRS12
- MEA1, TLR8, SAMSN1, MRCL3, CD1B
- MEA1, TLR8, SAMSN1, MRCL3, FAM111A
- MEA1, TLR8, SAMSN1, SMAD2, LAPTM4A
- MEA1, TLR8, SAMSN1, SMAD2, SFRS12
- MEA1, TLR8, SAMSN1, SMAD2, CD1B
- MEA1, TLR8, SAMSN1, SMAD2, FAM111A
- MEA1, TLR8, SAMSN1, LAPTM4A, SFRS12
- MEA1, TLR8, SAMSN1, LAPTM4A, CD1B
- MEA1, TLR8, SAMSN1, LAPTM4A, FAM111A
- MEA1, TLR8, SAMSN1, SFRS12, CD1B
- MEA1, TLR8, SAMSN1, SFRS12, FAM111A
- MEA1, TLR8, SAMSN1, CD1B, FAM111A
- MEA1, TLR8, DGCR14, MRCL3, SMAD2
- MEA1, TLR8, DGCR14, MRCL3, LAPTM4A
- MEA1, TLR8, DGCR14, MRCL3, SFRS12
- MEA1, TLR8, DGCR14, MRCL3, CD1B
- MEA1, TLR8, DGCR14, MRCL3, FAM111A
- MEA1, TLR8, DGCR14, SMAD2, LAPTM4A
- MEA1, TLR8, DGCR14, SMAD2, SFRS12
- MEA1, TLR8, DGCR14, SMAD2, CD1B
- MEA1, TLR8, DGCR14, SMAD2, FAM111A
- MEA1, TLR8, DGCR14, LAPTM4A, SFRS12
- MEA1, TLR8, DGCR14, LAPTM4A, CD1B
- MEA1, TLR8, DGCR14, LAPTM4A, FAM111A
- MEA1, TLR8, DGCR14, SFRS12, CD1B
- MEA1, TLR8, DGCR14, SFRS12, FAM111A
- MEA1, TLR8, DGCR14, CD1B, FAM111A
- MEA1, TLR8, MRCL3, SMAD2, LAPTM4A
- MEA1, TLR8, MRCL3, SMAD2, SFRS12
- MEA1, TLR8, MRCL3, SMAD2, CD1B
- MEA1, TLR8, MRCL3, SMAD2, FAM111A
- MEA1, TLR8, MRCL3, LAPTM4A, SFRS12
- MEA1, TLR8, MRCL3, LAPTM4A, CD1B
- MEA1, TLR8, MRCL3, LAPTM4A, FAM111A

- MEA1, TLR8, MRCL3, SFRS12, CD1B
- MEA1, TLR8, MRCL3, SFRS12, FAM111A
- MEA1, TLR8, MRCL3, CD1B, FAM111A
- MEA1, TLR8, SMAD2, LAPTM4A, SFRS12
- MEA1, TLR8, SMAD2, LAPTM4A, CD1B
- MEA1, TLR8, SMAD2, LAPTM4A, FAM111A
- MEA1, TLR8, SMAD2, SFRS12, CD1B
- MEA1, TLR8, SMAD2, SFRS12, FAM111A
- MEA1, TLR8, SMAD2, CD1B, FAM111A
- MEA1, TLR8, LAPTM4A, SFRS12, CD1B
- MEA1, TLR8, LAPTM4A, SFRS12, FAM111A
- MEA1, TLR8, LAPTM4A, CD1B, FAM111A
- MEA1, TLR8, SFRS12, CD1B, FAM111A
- MEA1, SAMSN1, DGCR14, MRCL3, SMAD2
- MEA1, SAMSN1, DGCR14, MRCL3, LAPTM4A
- MEA1, SAMSN1, DGCR14, MRCL3, SFRS12
- MEA1, SAMSN1, DGCR14, MRCL3, CD1B
- MEA1, SAMSN1, DGCR14, MRCL3, FAM111A
- MEA1, SAMSN1, DGCR14, SMAD2, LAPTM4A
- MEA1, SAMSN1, DGCR14, SMAD2, SFRS12
- MEA1, SAMSN1, DGCR14, SMAD2, CD1B
- MEA1, SAMSN1, DGCR14, SMAD2, FAM111A
- MEA1, SAMSN1, DGCR14, LAPTM4A, SFRS12
- MEA1, SAMSN1, DGCR14, LAPTM4A, CD1B
- MEA1, SAMSN1, DGCR14, LAPTM4A, FAM111A
- MEA1, SAMSN1, DGCR14, SFRS12, CD1B
- MEA1, SAMSN1, DGCR14, SFRS12, FAM111A
- MEA1, SAMSN1, DGCR14, CD1B, FAM111A
- MEA1, SAMSN1, MRCL3, SMAD2, LAPTM4A
- MEA1, SAMSN1, MRCL3, SMAD2, SFRS12
- MEA1, SAMSN1, MRCL3, SMAD2, CD1B
- MEA1, SAMSN1, MRCL3, SMAD2, FAM111A
- MEA1, SAMSN1, MRCL3, LAPTM4A, SFRS12
- MEA1, SAMSN1, MRCL3, LAPTM4A, CD1B
- MEA1, SAMSN1, MRCL3, LAPTM4A, FAM111A
- MEA1, SAMSN1, MRCL3, SFRS12, CD1B
- MEA1, SAMSN1, MRCL3, SFRS12, FAM111A
- MEA1, SAMSN1, MRCL3, CD1B, FAM111A
- MEA1, SAMSN1, SMAD2, LAPTM4A, SFRS12
- MEA1, SAMSN1, SMAD2, LAPTM4A, CD1B
- MEA1, SAMSN1, SMAD2, LAPTM4A, FAM111A
- MEA1, SAMSN1, SMAD2, SFRS12, CD1B
- MEA1, SAMSN1, SMAD2, SFRS12, FAM111A
- MEA1, SAMSN1, SMAD2, CD1B, FAM111A
- MEA1, SAMSN1, LAPTM4A, SFRS12, CD1B
- MEA1, SAMSN1, LAPTM4A, SFRS12, FAM111A
- MEA1, SAMSN1, LAPTM4A, CD1B, FAM111A
- MEA1, SAMSN1, SFRS12, CD1B, FAM111A
- MEA1, DGCR14, MRCL3, SMAD2, LAPTM4A
- MEA1, DGCR14, MRCL3, SMAD2, SFRS12
- MEA1, DGCR14, MRCL3, SMAD2, CD1B
- MEA1, DGCR14, MRCL3, SMAD2, FAM111A
- MEA1, DGCR14, MRCL3, LAPTM4A, SFRS12
- MEA1, DGCR14, MRCL3, LAPTM4A, CD1B
- MEA1, DGCR14, MRCL3, LAPTM4A, FAM111A
- MEA1, DGCR14, MRCL3, SFRS12, CD1B
- MEA1, DGCR14, MRCL3, SFRS12, FAM111A
- MEA1, DGCR14, MRCL3, CD1B, FAM111A

- MEA1, DGCR14, SMAD2, LAPTM4A, SFRS12
- MEA1, DGCR14, SMAD2, LAPTM4A, CD1B
- MEA1, DGCR14, SMAD2, LAPTM4A, FAM111A
- MEA1, DGCR14, SMAD2, SFRS12, CD1B
- MEA1, DGCR14, SMAD2, SFRS12, FAM111A
- MEA1, DGCR14, SMAD2, CD1B, FAM111A
- MEA1, DGCR14, LAPTM4A, SFRS12, CD1B
- MEA1, DGCR14, LAPTM4A, SFRS12, FAM111A
- MEA1, DGCR14, LAPTM4A, CD1B, FAM111A
- MEA1, DGCR14, SFRS12, CD1B, FAM111A
- MEA1, MRCL3, SMAD2, LAPTM4A, SFRS12
- MEA1, MRCL3, SMAD2, LAPTM4A, CD1B
- MEA1, MRCL3, SMAD2, LAPTM4A, FAM111A
- MEA1, MRCL3, SMAD2, SFRS12, CD1B
- MEA1, MRCL3, SMAD2, SFRS12, FAM111A
- MEA1, MRCL3, SMAD2, CD1B, FAM111A
- MEA1, MRCL3, LAPTM4A, SFRS12, CD1B
- MEA1, MRCL3, LAPTM4A, SFRS12, FAM111A
- MEA1, MRCL3, LAPTM4A, CD1B, FAM111A
- MEA1, MRCL3, SFRS12, CD1B, FAM111A
- MEA1, SMAD2, LAPTM4A, SFRS12, CD1B
- MEA1, SMAD2, LAPTM4A, SFRS12, FAM111A
- MEA1, SMAD2, LAPTM4A, CD1B, FAM111A
- MEA1, SMAD2, SFRS12, CD1B, FAM111A
- MEA1, LAPTM4A, SFRS12, CD1B, FAM111A
- SPRY4, TLR8, SAMSN1, DGCR14, MRCL3
- SPRY4, TLR8, SAMSN1, DGCR14, SMAD2
- SPRY4, TLR8, SAMSN1, DGCR14, LAPTM4A
- SPRY4, TLR8, SAMSN1, DGCR14, SFRS12
- SPRY4, TLR8, SAMSN1, DGCR14, CD1B
- SPRY4, TLR8, SAMSN1, DGCR14, FAM111A
- SPRY4, TLR8, SAMSN1, MRCL3, SMAD2
- SPRY4, TLR8, SAMSN1, MRCL3, LAPTM4A
- SPRY4, TLR8, SAMSN1, MRCL3, SFRS12
- SPRY4, TLR8, SAMSN1, MRCL3, CD1B
- SPRY4, TLR8, SAMSN1, MRCL3, FAM111A
- SPRY4, TLR8, SAMSN1, SMAD2, LAPTM4A
- SPRY4, TLR8, SAMSN1, SMAD2, SFRS12
- SPRY4, TLR8, SAMSN1, SMAD2, CD1B
- SPRY4, TLR8, SAMSN1, SMAD2, FAM111A
- SPRY4, TLR8, SAMSN1, LAPTM4A, SFRS12
- SPRY4, TLR8, SAMSN1, LAPTM4A, CD1B
- SPRY4, TLR8, SAMSN1, LAPTM4A, FAM111A
- SPRY4, TLR8, SAMSN1, SFRS12, CD1B
- SPRY4, TLR8, SAMSN1, SFRS12, FAM111A
- SPRY4, TLR8, SAMSN1, CD1B, FAM111A
- SPRY4, TLR8, DGCR14, MRCL3, SMAD2
- SPRY4, TLR8, DGCR14, MRCL3, LAPTM4A
- SPRY4, TLR8, DGCR14, MRCL3, SFRS12
- SPRY4, TLR8, DGCR14, MRCL3, CD1B
- SPRY4, TLR8, DGCR14, MRCL3, FAM111A
- SPRY4, TLR8, DGCR14, SMAD2, LAPTM4A
- SPRY4, TLR8, DGCR14, SMAD2, SFRS12
- SPRY4, TLR8, DGCR14, SMAD2, CD1B
- SPRY4, TLR8, DGCR14, SMAD2, FAM111A
- SPRY4, TLR8, DGCR14, LAPTM4A, SFRS12
- SPRY4, TLR8, DGCR14, LAPTM4A, CD1B
- SPRY4, TLR8, DGCR14, LAPTM4A, FAM111A

- SPRY4, TLR8, DGCR14, SFRS12, CD1B
- SPRY4, TLR8, DGCR14, SFRS12, FAM111A
- SPRY4, TLR8, DGCR14, CD1B, FAM111A
- SPRY4, TLR8, MRCL3, SMAD2, LAPTM4A
- SPRY4, TLR8, MRCL3, SMAD2, SFRS12
- SPRY4, TLR8, MRCL3, SMAD2, CD1B
- SPRY4, TLR8, MRCL3, SMAD2, FAM111A
- SPRY4, TLR8, MRCL3, LAPTM4A, SFRS12
- SPRY4, TLR8, MRCL3, LAPTM4A, CD1B
- SPRY4, TLR8, MRCL3, LAPTM4A, FAM111A
- SPRY4, TLR8, MRCL3, SFRS12, CD1B
- SPRY4, TLR8, MRCL3, SFRS12, FAM111A
- SPRY4, TLR8, MRCL3, CD1B, FAM111A
- SPRY4, TLR8, SMAD2, LAPTM4A, SFRS12
- SPRY4, TLR8, SMAD2, LAPTM4A, CD1B
- SPRY4, TLR8, SMAD2, LAPTM4A, FAM111A
- SPRY4, TLR8, SMAD2, SFRS12, CD1B
- SPRY4, TLR8, SMAD2, SFRS12, FAM111A
- SPRY4, TLR8, SMAD2, CD1B, FAM111A
- SPRY4, TLR8, LAPTM4A, SFRS12, CD1B
- SPRY4, TLR8, LAPTM4A, SFRS12, FAM111A
- SPRY4, TLR8, LAPTM4A, CD1B, FAM111A
- SPRY4, TLR8, SFRS12, CD1B, FAM111A
- SPRY4, SAMSN1, DGCR14, MRCL3, SMAD2
- SPRY4, SAMSN1, DGCR14, MRCL3, LAPTM4A
- SPRY4, SAMSN1, DGCR14, MRCL3, SFRS12
- SPRY4, SAMSN1, DGCR14, MRCL3, CD1B
- SPRY4, SAMSN1, DGCR14, MRCL3, FAM111A
- SPRY4, SAMSN1, DGCR14, SMAD2, LAPTM4A
- SPRY4, SAMSN1, DGCR14, SMAD2, SFRS12
- SPRY4, SAMSN1, DGCR14, SMAD2, CD1B
- SPRY4, SAMSN1, DGCR14, SMAD2, FAM111A
- SPRY4, SAMSN1, DGCR14, LAPTM4A, SFRS12
- SPRY4, SAMSN1, DGCR14, LAPTM4A, CD1B
- SPRY4, SAMSN1, DGCR14, LAPTM4A, FAM111A
- SPRY4, SAMSN1, DGCR14, SFRS12, CD1B
- SPRY4, SAMSN1, DGCR14, SFRS12, FAM111A
- SPRY4, SAMSN1, DGCR14, CD1B, FAM111A
- SPRY4, SAMSN1, MRCL3, SMAD2, LAPTM4A
- SPRY4, SAMSN1, MRCL3, SMAD2, SFRS12
- SPRY4, SAMSN1, MRCL3, SMAD2, CD1B
- SPRY4, SAMSN1, MRCL3, SMAD2, FAM111A
- SPRY4, SAMSN1, MRCL3, LAPTM4A, SFRS12
- SPRY4, SAMSN1, MRCL3, LAPTM4A, CD1B
- SPRY4, SAMSN1, MRCL3, LAPTM4A, FAM111A
- SPRY4, SAMSN1, MRCL3, SFRS12, CD1B
- SPRY4, SAMSN1, MRCL3, SFRS12, FAM111A
- SPRY4, SAMSN1, MRCL3, CD1B, FAM111A
- SPRY4, SAMSN1, SMAD2, LAPTM4A, SFRS12
- SPRY4, SAMSN1, SMAD2, LAPTM4A, CD1B
- SPRY4, SAMSN1, SMAD2, LAPTM4A, FAM111A
- SPRY4, SAMSN1, SMAD2, SFRS12, CD1B
- SPRY4, SAMSN1, SMAD2, SFRS12, FAM111A
- SPRY4, SAMSN1, SMAD2, CD1B, FAM111A
- SPRY4, SAMSN1, LAPTM4A, SFRS12, CD1B
- SPRY4, SAMSN1, LAPTM4A, SFRS12, FAM111A
- SPRY4, SAMSN1, LAPTM4A, CD1B, FAM111A
- SPRY4, SAMSN1, SFRS12, CD1B, FAM111A

- SPRY4, DGCR14, MRCL3, SMAD2, LAPTM4A
- SPRY4, DGCR14, MRCL3, SMAD2, SFRS12
- SPRY4, DGCR14, MRCL3, SMAD2, CD1B
- SPRY4, DGCR14, MRCL3, SMAD2, FAM111A
- SPRY4, DGCR14, MRCL3, LAPTM4A, SFRS12
- SPRY4, DGCR14, MRCL3, LAPTM4A, CD1B
- SPRY4, DGCR14, MRCL3, LAPTM4A, FAM111A
- SPRY4, DGCR14, MRCL3, SFRS12, CD1B
- SPRY4, DGCR14, MRCL3, SFRS12, FAM111A
- SPRY4, DGCR14, MRCL3, CD1B, FAM111A
- SPRY4, DGCR14, SMAD2, LAPTM4A, SFRS12
- SPRY4, DGCR14, SMAD2, LAPTM4A, CD1B
- SPRY4, DGCR14, SMAD2, LAPTM4A, FAM111A
- SPRY4, DGCR14, SMAD2, SFRS12, CD1B
- SPRY4, DGCR14, SMAD2, SFRS12, FAM111A
- SPRY4, DGCR14, SMAD2, CD1B, FAM111A
- SPRY4, DGCR14, LAPTM4A, SFRS12, CD1B
- SPRY4, DGCR14, LAPTM4A, SFRS12, FAM111A
- SPRY4, DGCR14, LAPTM4A, CD1B, FAM111A
- SPRY4, DGCR14, SFRS12, CD1B, FAM111A
- SPRY4, MRCL3, SMAD2, LAPTM4A, SFRS12
- SPRY4, MRCL3, SMAD2, LAPTM4A, CD1B
- SPRY4, MRCL3, SMAD2, LAPTM4A, FAM111A
- SPRY4, MRCL3, SMAD2, SFRS12, CD1B
- SPRY4, MRCL3, SMAD2, SFRS12, FAM111A
- SPRY4, MRCL3, SMAD2, CD1B, FAM111A
- SPRY4, MRCL3, LAPTM4A, SFRS12, CD1B
- SPRY4, MRCL3, LAPTM4A, SFRS12, FAM111A
- SPRY4, MRCL3, LAPTM4A, CD1B, FAM111A
- SPRY4, MRCL3, SFRS12, CD1B, FAM111A
- SPRY4, SMAD2, LAPTM4A, SFRS12, CD1B
- SPRY4, SMAD2, LAPTM4A, SFRS12, FAM111A
- SPRY4, SMAD2, LAPTM4A, CD1B, FAM111A
- SPRY4, SMAD2, SFRS12, CD1B, FAM111A
- SPRY4, LAPTM4A, SFRS12, CD1B, FAM111A
- TLR8, SAMSN1, DGCR14, MRCL3, SMAD2
- TLR8, SAMSN1, DGCR14, MRCL3, LAPTM4A
- TLR8, SAMSN1, DGCR14, MRCL3, SFRS12
- TLR8, SAMSN1, DGCR14, MRCL3, CD1B
- TLR8, SAMSN1, DGCR14, MRCL3, FAM111A
- TLR8, SAMSN1, DGCR14, SMAD2, LAPTM4A
- TLR8, SAMSN1, DGCR14, SMAD2, SFRS12
- TLR8, SAMSN1, DGCR14, SMAD2, CD1B
- TLR8, SAMSN1, DGCR14, SMAD2, FAM111A
- TLR8, SAMSN1, DGCR14, LAPTM4A, SFRS12
- TLR8, SAMSN1, DGCR14, LAPTM4A, CD1B
- TLR8, SAMSN1, DGCR14, LAPTM4A, FAM111A
- TLR8, SAMSN1, DGCR14, SFRS12, CD1B
- TLR8, SAMSN1, DGCR14, SFRS12, FAM111A
- TLR8, SAMSN1, DGCR14, CD1B, FAM111A
- TLR8, SAMSN1, MRCL3, SMAD2, LAPTM4A
- TLR8, SAMSN1, MRCL3, SMAD2, SFRS12
- TLR8, SAMSN1, MRCL3, SMAD2, CD1B
- TLR8, SAMSN1, MRCL3, SMAD2, FAM111A
- TLR8, SAMSN1, MRCL3, LAPTM4A, SFRS12
- TLR8, SAMSN1, MRCL3, LAPTM4A, CD1B
- TLR8, SAMSN1, MRCL3, LAPTM4A, FAM111A
- TLR8, SAMSN1, MRCL3, SFRS12, CD1B

- TLR8, SAMSN1, MRCL3, SFRS12, FAM111A
- TLR8, SAMSN1, MRCL3, CD1B, FAM111A
- TLR8, SAMSN1, SMAD2, LAPTM4A, SFRS12
- TLR8, SAMSN1, SMAD2, LAPTM4A, CD1B
- TLR8, SAMSN1, SMAD2, LAPTM4A, FAM111A
- TLR8, SAMSN1, SMAD2, SFRS12, CD1B
- TLR8, SAMSN1, SMAD2, SFRS12, FAM111A
- TLR8, SAMSN1, SMAD2, CD1B, FAM111A
- TLR8, SAMSN1, LAPTM4A, SFRS12, CD1B
- TLR8, SAMSN1, LAPTM4A, SFRS12, FAM111A
- TLR8, SAMSN1, LAPTM4A, CD1B, FAM111A
- TLR8, SAMSN1, SFRS12, CD1B, FAM111A
- TLR8, DGCR14, MRCL3, SMAD2, LAPTM4A
- TLR8, DGCR14, MRCL3, SMAD2, SFRS12
- TLR8, DGCR14, MRCL3, SMAD2, CD1B
- TLR8, DGCR14, MRCL3, SMAD2, FAM111A
- TLR8, DGCR14, MRCL3, LAPTM4A, SFRS12
- TLR8, DGCR14, MRCL3, LAPTM4A, CD1B
- TLR8, DGCR14, MRCL3, LAPTM4A, FAM111A
- TLR8, DGCR14, MRCL3, SFRS12, CD1B
- TLR8, DGCR14, MRCL3, SFRS12, FAM111A
- TLR8, DGCR14, MRCL3, CD1B, FAM111A
- TLR8, DGCR14, SMAD2, LAPTM4A, SFRS12
- TLR8, DGCR14, SMAD2, LAPTM4A, CD1B
- TLR8, DGCR14, SMAD2, LAPTM4A, FAM111A
- TLR8, DGCR14, SMAD2, SFRS12, CD1B
- TLR8, DGCR14, SMAD2, SFRS12, FAM111A
- TLR8, DGCR14, SMAD2, CD1B, FAM111A
- TLR8, DGCR14, LAPTM4A, SFRS12, CD1B
- TLR8, DGCR14, LAPTM4A, SFRS12, FAM111A
- TLR8, DGCR14, LAPTM4A, CD1B, FAM111A
- TLR8, DGCR14, SFRS12, CD1B, FAM111A
- TLR8, MRCL3, SMAD2, LAPTM4A, SFRS12
- TLR8, MRCL3, SMAD2, LAPTM4A, CD1B
- TLR8, MRCL3, SMAD2, LAPTM4A, FAM111A
- TLR8, MRCL3, SMAD2, SFRS12, CD1B
- TLR8, MRCL3, SMAD2, SFRS12, FAM111A
- TLR8, MRCL3, SMAD2, CD1B, FAM111A
- TLR8, MRCL3, LAPTM4A, SFRS12, CD1B
- TLR8, MRCL3, LAPTM4A, SFRS12, FAM111A
- TLR8, MRCL3, LAPTM4A, CD1B, FAM111A
- TLR8, MRCL3, SFRS12, CD1B, FAM111A
- TLR8, SMAD2, LAPTM4A, SFRS12, CD1B
- TLR8, SMAD2, LAPTM4A, SFRS12, FAM111A
- TLR8, SMAD2, LAPTM4A, CD1B, FAM111A
- TLR8, SMAD2, SFRS12, CD1B, FAM111A
- TLR8, LAPTM4A, SFRS12, CD1B, FAM111A
- SAMSN1, DGCR14, MRCL3, SMAD2, LAPTM4A
- SAMSN1, DGCR14, MRCL3, SMAD2, SFRS12
- SAMSN1, DGCR14, MRCL3, SMAD2, CD1B
- SAMSN1, DGCR14, MRCL3, SMAD2, FAM111A
- SAMSN1, DGCR14, MRCL3, LAPTM4A, SFRS12
- SAMSN1, DGCR14, MRCL3, LAPTM4A, CD1B
- SAMSN1, DGCR14, MRCL3, LAPTM4A, FAM111A
- SAMSN1, DGCR14, MRCL3, SFRS12, CD1B
- SAMSN1, DGCR14, MRCL3, SFRS12, FAM111A
- SAMSN1, DGCR14, MRCL3, CD1B, FAM111A
- SAMSN1, DGCR14, SMAD2, LAPTM4A, SFRS12

- SAMSN1, DGCR14, SMAD2, LAPTM4A, CD1B
- SAMSN1, DGCR14, SMAD2, LAPTM4A, FAM111A
- SAMSN1, DGCR14, SMAD2, SFRS12, CD1B
- SAMSN1, DGCR14, SMAD2, SFRS12, FAM111A
- SAMSN1, DGCR14, SMAD2, CD1B, FAM111A
- SAMSN1, DGCR14, LAPTM4A, SFRS12, CD1B
- SAMSN1, DGCR14, LAPTM4A, SFRS12, FAM111A
- SAMSN1, DGCR14, LAPTM4A, CD1B, FAM111A
- SAMSN1, DGCR14, SFRS12, CD1B, FAM111A
- SAMSN1, MRCL3, SMAD2, LAPTM4A, SFRS12
- SAMSN1, MRCL3, SMAD2, LAPTM4A, CD1B
- SAMSN1, MRCL3, SMAD2, LAPTM4A, FAM111A
- SAMSN1, MRCL3, SMAD2, SFRS12, CD1B
- SAMSN1, MRCL3, SMAD2, SFRS12, FAM111A
- SAMSN1, MRCL3, SMAD2, CD1B, FAM111A
- SAMSN1, MRCL3, LAPTM4A, SFRS12, CD1B
- SAMSN1, MRCL3, LAPTM4A, SFRS12, FAM111A
- SAMSN1, MRCL3, LAPTM4A, CD1B, FAM111A
- SAMSN1, MRCL3, SFRS12, CD1B, FAM111A
- SAMSN1, SMAD2, LAPTM4A, SFRS12, CD1B
- SAMSN1, SMAD2, LAPTM4A, SFRS12, FAM111A
- SAMSN1, SMAD2, LAPTM4A, CD1B, FAM111A
- SAMSN1, SMAD2, SFRS12, CD1B, FAM111A
- SAMSN1, LAPTM4A, SFRS12, CD1B, FAM111A
- DGCR14, MRCL3, SMAD2, LAPTM4A, SFRS12
- DGCR14, MRCL3, SMAD2, LAPTM4A, CD1B
- DGCR14, MRCL3, SMAD2, LAPTM4A, FAM111A
- DGCR14, MRCL3, SMAD2, SFRS12, CD1B
- DGCR14, MRCL3, SMAD2, SFRS12, FAM111A
- DGCR14, MRCL3, SMAD2, CD1B, FAM111A
- DGCR14, MRCL3, LAPTM4A, SFRS12, CD1B
- DGCR14, MRCL3, LAPTM4A, SFRS12, FAM111A
- DGCR14, MRCL3, LAPTM4A, CD1B, FAM111A
- DGCR14, MRCL3, SFRS12, CD1B, FAM111A
- DGCR14, SMAD2, LAPTM4A, SFRS12, CD1B
- DGCR14, SMAD2, LAPTM4A, SFRS12, FAM111A
- DGCR14, SMAD2, LAPTM4A, CD1B, FAM111A
- DGCR14, SMAD2, SFRS12, CD1B, FAM111A
- DGCR14, LAPTM4A, SFRS12, CD1B, FAM111A
- MRCL3, SMAD2, LAPTM4A, SFRS12, CD1B
- MRCL3, SMAD2, LAPTM4A, SFRS12, FAM111A
- MRCL3, SMAD2, LAPTM4A, CD1B, FAM111A
- MRCL3, SMAD2, SFRS12, CD1B, FAM111A
- MRCL3, LAPTM4A, SFRS12, CD1B, FAM111A
- SMAD2, LAPTM4A, SFRS12, CD1B, FAM111A

Thus, combinations of 6 genes suitable for the staging of the tumor include

- MEA1, SPRY4, TLR8, SAMSN1, DGCR14, MRCL3
- MEA1, SPRY4, TLR8, SAMSN1, DGCR14, SMAD2
- MEA1, SPRY4, TLR8, SAMSN1, DGCR14, LAPTM4A
- MEA1, SPRY4, TLR8, SAMSN1, DGCR14, SFRS12
- MEA1, SPRY4, TLR8, SAMSN1, DGCR14, CD1B
- MEA1, SPRY4, TLR8, SAMSN1, DGCR14, FAM111A
- MEA1, SPRY4, TLR8, SAMSN1, MRCL3, SMAD2
- MEA1, SPRY4, TLR8, SAMSN1, MRCL3, LAPTM4A
- MEA1, SPRY4, TLR8, SAMSN1, MRCL3, SFRS12
- MEA1, SPRY4, TLR8, SAMSN1, MRCL3, CD1B

- MEA1, SPRY4, TLR8, SAMSN1, MRCL3, FAM111A
- MEA1, SPRY4, TLR8, SAMSN1, SMAD2, LAPTM4A
- MEA1, SPRY4, TLR8, SAMSN1, SMAD2, SFRS12
- MEA1, SPRY4, TLR8, SAMSN1, SMAD2, CD1B
- MEA1, SPRY4, TLR8, SAMSN1, SMAD2, FAM111A
- MEA1, SPRY4, TLR8, SAMSN1, LAPTM4A, SFRS12
- MEA1, SPRY4, TLR8, SAMSN1, LAPTM4A, CD1B
- MEA1, SPRY4, TLR8, SAMSN1, LAPTM4A, FAM111A
- MEA1, SPRY4, TLR8, SAMSN1, SFRS12, CD1B
- MEA1, SPRY4, TLR8, SAMSN1, SFRS12, FAM111A
- MEA1, SPRY4, TLR8, SAMSN1, CD1B, FAM111A
- MEA1, SPRY4, TLR8, DGCR14, MRCL3, SMAD2
- MEA1, SPRY4, TLR8, DGCR14, MRCL3, LAPTM4A
- MEA1, SPRY4, TLR8, DGCR14, MRCL3, SFRS12
- MEA1, SPRY4, TLR8, DGCR14, MRCL3, CD1B
- MEA1, SPRY4, TLR8, DGCR14, MRCL3, FAM111A
- MEA1, SPRY4, TLR8, DGCR14, SMAD2, LAPTM4A
- MEA1, SPRY4, TLR8, DGCR14, SMAD2, SFRS12
- MEA1, SPRY4, TLR8, DGCR14, SMAD2, CD1B
- MEA1, SPRY4, TLR8, DGCR14, SMAD2, FAM111A
- MEA1, SPRY4, TLR8, DGCR14, LAPTM4A, SFRS12
- MEA1, SPRY4, TLR8, DGCR14, LAPTM4A, CD1B
- MEA1, SPRY4, TLR8, DGCR14, LAPTM4A, FAM111A
- MEA1, SPRY4, TLR8, DGCR14, SFRS12, CD1B
- MEA1, SPRY4, TLR8, DGCR14, SFRS12, FAM111A
- MEA1, SPRY4, TLR8, DGCR14, CD1B, FAM111A
- MEA1, SPRY4, TLR8, MRCL3, SMAD2, LAPTM4A
- MEA1, SPRY4, TLR8, MRCL3, SMAD2, SFRS12
- MEA1, SPRY4, TLR8, MRCL3, SMAD2, CD1B
- MEA1, SPRY4, TLR8, MRCL3, SMAD2, FAM111A
- MEA1, SPRY4, TLR8, MRCL3, LAPTM4A, SFRS12
- MEA1, SPRY4, TLR8, MRCL3, LAPTM4A, CD1B
- MEA1, SPRY4, TLR8, MRCL3, LAPTM4A, FAM111A
- MEA1, SPRY4, TLR8, MRCL3, SFRS12, CD1B
- MEA1, SPRY4, TLR8, MRCL3, SFRS12, FAM111A
- MEA1, SPRY4, TLR8, MRCL3, CD1B, FAM111A
- MEA1, SPRY4, TLR8, SMAD2, LAPTM4A, SFRS12
- MEA1, SPRY4, TLR8, SMAD2, LAPTM4A, CD1B
- MEA1, SPRY4, TLR8, SMAD2, LAPTM4A, FAM111A
- MEA1, SPRY4, TLR8, SMAD2, SFRS12, CD1B
- MEA1, SPRY4, TLR8, SMAD2, SFRS12, FAM111A
- MEA1, SPRY4, TLR8, SMAD2, CD1B, FAM111A
- MEA1, SPRY4, TLR8, LAPTM4A, SFRS12, CD1B
- MEA1, SPRY4, TLR8, LAPTM4A, SFRS12, FAM111A
- MEA1, SPRY4, TLR8, LAPTM4A, CD1B, FAM111A
- MEA1, SPRY4, TLR8, SFRS12, CD1B, FAM111A
- MEA1, SPRY4, SAMSN1, DGCR14, MRCL3, SMAD2
- MEA1, SPRY4, SAMSN1, DGCR14, MRCL3, LAPTM4A
- MEA1, SPRY4, SAMSN1, DGCR14, MRCL3, SFRS12
- MEA1, SPRY4, SAMSN1, DGCR14, MRCL3, CD1B
- MEA1, SPRY4, SAMSN1, DGCR14, MRCL3, FAM111A
- MEA1, SPRY4, SAMSN1, DGCR14, SMAD2, LAPTM4A
- MEA1, SPRY4, SAMSN1, DGCR14, SMAD2, SFRS12
- MEA1, SPRY4, SAMSN1, DGCR14, SMAD2, CD1B
- MEA1, SPRY4, SAMSN1, DGCR14, SMAD2, FAM111A
- MEA1, SPRY4, SAMSN1, DGCR14, LAPTM4A, SFRS12
- MEA1, SPRY4, SAMSN1, DGCR14, LAPTM4A, CD1B
- MEA1, SPRY4, SAMSN1, DGCR14, LAPTM4A, FAM111A

- MEA1, SPRY4, SAMSN1, DGCR14, SFRS12, CD1B
- MEA1, SPRY4, SAMSN1, DGCR14, SFRS12, FAM111A
- MEA1, SPRY4, SAMSN1, DGCR14, CD1B, FAM111A
- MEA1, SPRY4, SAMSN1, MRCL3, SMAD2, LAPTM4A
- MEA1, SPRY4, SAMSN1, MRCL3, SMAD2, SFRS12
- MEA1, SPRY4, SAMSN1, MRCL3, SMAD2, CD1B
- MEA1, SPRY4, SAMSN1, MRCL3, SMAD2, FAM111A
- MEA1, SPRY4, SAMSN1, MRCL3, LAPTM4A, SFRS12
- MEA1, SPRY4, SAMSN1, MRCL3, LAPTM4A, CD1B
- MEA1, SPRY4, SAMSN1, MRCL3, LAPTM4A, FAM111A
- MEA1, SPRY4, SAMSN1, MRCL3, SFRS12, CD1B
- MEA1, SPRY4, SAMSN1, MRCL3, SFRS12, FAM111A
- MEA1, SPRY4, SAMSN1, MRCL3, CD1B, FAM111A
- MEA1, SPRY4, SAMSN1, SMAD2, LAPTM4A, SFRS12
- MEA1, SPRY4, SAMSN1, SMAD2, LAPTM4A, CD1B
- MEA1, SPRY4, SAMSN1, SMAD2, LAPTM4A, FAM111A
- MEA1, SPRY4, SAMSN1, SMAD2, SFRS12, CD1B
- MEA1, SPRY4, SAMSN1, SMAD2, SFRS12, FAM111A
- MEA1, SPRY4, SAMSN1, SMAD2, CD1B, FAM111A
- MEA1, SPRY4, SAMSN1, LAPTM4A, SFRS12, CD1B
- MEA1, SPRY4, SAMSN1, LAPTM4A, SFRS12, FAM111A
- MEA1, SPRY4, SAMSN1, LAPTM4A, CD1B, FAM111A
- MEA1, SPRY4, SAMSN1, SFRS12, CD1B, FAM111A
- MEA1, SPRY4, DGCR14, MRCL3, SMAD2, LAPTM4A
- MEA1, SPRY4, DGCR14, MRCL3, SMAD2, SFRS12
- MEA1, SPRY4, DGCR14, MRCL3, SMAD2, CD1B
- MEA1, SPRY4, DGCR14, MRCL3, SMAD2, FAM111A
- MEA1, SPRY4, DGCR14, MRCL3, LAPTM4A, SFRS12
- MEA1, SPRY4, DGCR14, MRCL3, LAPTM4A, CD1B
- MEA1, SPRY4, DGCR14, MRCL3, LAPTM4A, FAM111A
- MEA1, SPRY4, DGCR14, MRCL3, SFRS12, CD1B
- MEA1, SPRY4, DGCR14, MRCL3, SFRS12, FAM111A
- MEA1, SPRY4, DGCR14, MRCL3, CD1B, FAM111A
- MEA1, SPRY4, DGCR14, SMAD2, LAPTM4A, SFRS12
- MEA1, SPRY4, DGCR14, SMAD2, LAPTM4A, CD1B
- MEA1, SPRY4, DGCR14, SMAD2, LAPTM4A, FAM111A
- MEA1, SPRY4, DGCR14, SMAD2, SFRS12, CD1B
- MEA1, SPRY4, DGCR14, SMAD2, SFRS12, FAM111A
- MEA1, SPRY4, DGCR14, SMAD2, CD1B, FAM111A
- MEA1, SPRY4, DGCR14, LAPTM4A, SFRS12, CD1B
- MEA1, SPRY4, DGCR14, LAPTM4A, SFRS12, FAM111A
- MEA1, SPRY4, DGCR14, LAPTM4A, CD1B, FAM111A
- MEA1, SPRY4, DGCR14, SFRS12, CD1B, FAM111A
- MEA1, SPRY4, MRCL3, SMAD2, LAPTM4A, SFRS12
- MEA1, SPRY4, MRCL3, SMAD2, LAPTM4A, CD1B
- MEA1, SPRY4, MRCL3, SMAD2, LAPTM4A, FAM111A
- MEA1, SPRY4, MRCL3, SMAD2, SFRS12, CD1B
- MEA1, SPRY4, MRCL3, SMAD2, SFRS12, FAM111A
- MEA1, SPRY4, MRCL3, SMAD2, CD1B, FAM111A
- MEA1, SPRY4, MRCL3, LAPTM4A, SFRS12, CD1B
- MEA1, SPRY4, MRCL3, LAPTM4A, SFRS12, FAM111A
- MEA1, SPRY4, MRCL3, LAPTM4A, CD1B, FAM111A
- MEA1, SPRY4, MRCL3, SFRS12, CD1B, FAM111A
- MEA1, SPRY4, SMAD2, LAPTM4A, SFRS12, CD1B
- MEA1, SPRY4, SMAD2, LAPTM4A, SFRS12, FAM111A
- MEA1, SPRY4, SMAD2, LAPTM4A, CD1B, FAM111A
- MEA1, SPRY4, SMAD2, SFRS12, CD1B, FAM111A
- MEA1, SPRY4, LAPTM4A, SFRS12, CD1B, FAM111A

- MEA1, TLR8, SAMSN1, DGCR14, MRCL3, SMAD2
- MEA1, TLR8, SAMSN1, DGCR14, MRCL3, LAPTM4A
- MEA1, TLR8, SAMSN1, DGCR14, MRCL3, SFRS12
- MEA1, TLR8, SAMSN1, DGCR14, MRCL3, CD1B
- MEA1, TLR8, SAMSN1, DGCR14, MRCL3, FAM111A
- MEA1, TLR8, SAMSN1, DGCR14, SMAD2, LAPTM4A
- MEA1, TLR8, SAMSN1, DGCR14, SMAD2, SFRS12
- MEA1, TLR8, SAMSN1, DGCR14, SMAD2, CD1B
- MEA1, TLR8, SAMSN1, DGCR14, SMAD2, FAM111A
- MEA1, TLR8, SAMSN1, DGCR14, LAPTM4A, SFRS12
- MEA1, TLR8, SAMSN1, DGCR14, LAPTM4A, CD1B
- MEA1, TLR8, SAMSN1, DGCR14, LAPTM4A, FAM111A
- MEA1, TLR8, SAMSN1, DGCR14, SFRS12, CD1B
- MEA1, TLR8, SAMSN1, DGCR14, SFRS12, FAM111A
- MEA1, TLR8, SAMSN1, DGCR14, CD1B, FAM111A
- MEA1, TLR8, SAMSN1, MRCL3, SMAD2, LAPTM4A
- MEA1, TLR8, SAMSN1, MRCL3, SMAD2, SFRS12
- MEA1, TLR8, SAMSN1, MRCL3, SMAD2, CD1B
- MEA1, TLR8, SAMSN1, MRCL3, SMAD2, FAM111A
- MEA1, TLR8, SAMSN1, MRCL3, LAPTM4A, SFRS12
- MEA1, TLR8, SAMSN1, MRCL3, LAPTM4A, CD1B
- MEA1, TLR8, SAMSN1, MRCL3, LAPTM4A, FAM111A
- MEA1, TLR8, SAMSN1, MRCL3, SFRS12, CD1B
- MEA1, TLR8, SAMSN1, MRCL3, SFRS12, FAM111A
- MEA1, TLR8, SAMSN1, MRCL3, CD1B, FAM111A
- MEA1, TLR8, SAMSN1, SMAD2, LAPTM4A, SFRS12
- MEA1, TLR8, SAMSN1, SMAD2, LAPTM4A, CD1B
- MEA1, TLR8, SAMSN1, SMAD2, LAPTM4A, FAM111A
- MEA1, TLR8, SAMSN1, SMAD2, SFRS12, CD1B
- MEA1, TLR8, SAMSN1, SMAD2, SFRS12, FAM111A
- MEA1, TLR8, SAMSN1, SMAD2, CD1B, FAM111A
- MEA1, TLR8, SAMSN1, LAPTM4A, SFRS12, CD1B
- MEA1, TLR8, SAMSN1, LAPTM4A, SFRS12, FAM111A
- MEA1, TLR8, SAMSN1, LAPTM4A, CD1B, FAM111A
- MEA1, TLR8, SAMSN1, SFRS12, CD1B, FAM111A
- MEA1, TLR8, DGCR14, MRCL3, SMAD2, LAPTM4A
- MEA1, TLR8, DGCR14, MRCL3, SMAD2, SFRS12
- MEA1, TLR8, DGCR14, MRCL3, SMAD2, CD1B
- MEA1, TLR8, DGCR14, MRCL3, SMAD2, FAM111A
- MEA1, TLR8, DGCR14, MRCL3, LAPTM4A, SFRS12
- MEA1, TLR8, DGCR14, MRCL3, LAPTM4A, CD1B
- MEA1, TLR8, DGCR14, MRCL3, LAPTM4A, FAM111A
- MEA1, TLR8, DGCR14, MRCL3, SFRS12, CD1B
- MEA1, TLR8, DGCR14, MRCL3, SFRS12, FAM111A
- MEA1, TLR8, DGCR14, MRCL3, CD1B, FAM111A
- MEA1, TLR8, DGCR14, SMAD2, LAPTM4A, SFRS12
- MEA1, TLR8, DGCR14, SMAD2, LAPTM4A, CD1B
- MEA1, TLR8, DGCR14, SMAD2, LAPTM4A, FAM111A
- MEA1, TLR8, DGCR14, SMAD2, SFRS12, CD1B
- MEA1, TLR8, DGCR14, SMAD2, SFRS12, FAM111A
- MEA1, TLR8, DGCR14, SMAD2, CD1B, FAM111A
- MEA1, TLR8, DGCR14, LAPTM4A, SFRS12, CD1B
- MEA1, TLR8, DGCR14, LAPTM4A, SFRS12, FAM111A
- MEA1, TLR8, DGCR14, LAPTM4A, CD1B, FAM111A
- MEA1, TLR8, DGCR14, SFRS12, CD1B, FAM111A
- MEA1, TLR8, MRCL3, SMAD2, LAPTM4A, SFRS12
- MEA1, TLR8, MRCL3, SMAD2, LAPTM4A, CD1B
- MEA1, TLR8, MRCL3, SMAD2, LAPTM4A, FAM111A

- MEA1, TLR8, MRCL3, SMAD2, SFRS12, CD1B
- MEA1, TLR8, MRCL3, SMAD2, SFRS12, FAM111A
- MEA1, TLR8, MRCL3, SMAD2, CD1B, FAM111A
- MEA1, TLR8, MRCL3, LAPTM4A, SFRS12, CD1B
- MEA1, TLR8, MRCL3, LAPTM4A, SFRS12, FAM111A
- MEA1, TLR8, MRCL3, LAPTM4A, CD1B, FAM111A
- MEA1, TLR8, MRCL3, SFRS12, CD1B, FAM111A
- MEA1, TLR8, SMAD2, LAPTM4A, SFRS12, CD1B
- MEA1, TLR8, SMAD2, LAPTM4A, SFRS12, FAM111A
- MEA1, TLR8, SMAD2, LAPTM4A, CD1B, FAM111A
- MEA1, TLR8, SMAD2, SFRS12, CD1B, FAM111A
- MEA1, TLR8, LAPTM4A, SFRS12, CD1B, FAM111A
- MEA1, SAMSN1, DGCR14, MRCL3, SMAD2, LAPTM4A
- MEA1, SAMSN1, DGCR14, MRCL3, SMAD2, SFRS12
- MEA1, SAMSN1, DGCR14, MRCL3, SMAD2, CD1B
- MEA1, SAMSN1, DGCR14, MRCL3, SMAD2, FAM111A
- MEA1, SAMSN1, DGCR14, MRCL3, LAPTM4A, SFRS12
- MEA1, SAMSN1, DGCR14, MRCL3, LAPTM4A, CD1B
- MEA1, SAMSN1, DGCR14, MRCL3, LAPTM4A, FAM111A
- MEA1, SAMSN1, DGCR14, MRCL3, SFRS12, CD1B
- MEA1, SAMSN1, DGCR14, MRCL3, SFRS12, FAM111A
- MEA1, SAMSN1, DGCR14, MRCL3, CD1B, FAM111A
- MEA1, SAMSN1, DGCR14, SMAD2, LAPTM4A, SFRS12
- MEA1, SAMSN1, DGCR14, SMAD2, LAPTM4A, CD1B
- MEA1, SAMSN1, DGCR14, SMAD2, LAPTM4A, FAM111A
- MEA1, SAMSN1, DGCR14, SMAD2, SFRS12, CD1B
- MEA1, SAMSN1, DGCR14, SMAD2, SFRS12, FAM111A
- MEA1, SAMSN1, DGCR14, SMAD2, CD1B, FAM111A
- MEA1, SAMSN1, DGCR14, LAPTM4A, SFRS12, CD1B
- MEA1, SAMSN1, DGCR14, LAPTM4A, SFRS12, FAM111A
- MEA1, SAMSN1, DGCR14, LAPTM4A, CD1B, FAM111A
- MEA1, SAMSN1, DGCR14, SFRS12, CD1B, FAM111A
- MEA1, SAMSN1, MRCL3, SMAD2, LAPTM4A, SFRS12
- MEA1, SAMSN1, MRCL3, SMAD2, LAPTM4A, CD1B
- MEA1, SAMSN1, MRCL3, SMAD2, LAPTM4A, FAM111A
- MEA1, SAMSN1, MRCL3, SMAD2, SFRS12, CD1B
- MEA1, SAMSN1, MRCL3, SMAD2, SFRS12, FAM111A
- MEA1, SAMSN1, MRCL3, SMAD2, CD1B, FAM111A
- MEA1, SAMSN1, MRCL3, LAPTM4A, SFRS12, CD1B
- MEA1, SAMSN1, MRCL3, LAPTM4A, SFRS12, FAM111A
- MEA1, SAMSN1, MRCL3, LAPTM4A, CD1B, FAM111A
- MEA1, SAMSN1, MRCL3, SFRS12, CD1B, FAM111A
- MEA1, SAMSN1, SMAD2, LAPTM4A, SFRS12, CD1B
- MEA1, SAMSN1, SMAD2, LAPTM4A, SFRS12, FAM111A
- MEA1, SAMSN1, SMAD2, LAPTM4A, CD1B, FAM111A
- MEA1, SAMSN1, SMAD2, SFRS12, CD1B, FAM111A
- MEA1, SAMSN1, LAPTM4A, SFRS12, CD1B, FAM111A
- MEA1, DGCR14, MRCL3, SMAD2, LAPTM4A, SFRS12
- MEA1, DGCR14, MRCL3, SMAD2, LAPTM4A, CD1B
- MEA1, DGCR14, MRCL3, SMAD2, LAPTM4A, FAM111A
- MEA1, DGCR14, MRCL3, SMAD2, SFRS12, CD1B
- MEA1, DGCR14, MRCL3, SMAD2, SFRS12, FAM111A
- MEA1, DGCR14, MRCL3, SMAD2, CD1B, FAM111A
- MEA1, DGCR14, MRCL3, LAPTM4A, SFRS12, CD1B
- MEA1, DGCR14, MRCL3, LAPTM4A, SFRS12, FAM111A
- MEA1, DGCR14, MRCL3, LAPTM4A, CD1B, FAM111A
- MEA1, DGCR14, MRCL3, SFRS12, CD1B, FAM111A
- MEA1, DGCR14, SMAD2, LAPTM4A, SFRS12, CD1B

- MEA1, DGCR14, SMAD2, LAPTM4A, SFRS12, FAM111A
- MEA1, DGCR14, SMAD2, LAPTM4A, CD1B, FAM111A
- MEA1, DGCR14, SMAD2, SFRS12, CD1B, FAM111A
- MEA1, DGCR14, LAPTM4A, SFRS12, CD1B, FAM111A
- MEA1, MRCL3, SMAD2, LAPTM4A, SFRS12, CD1B
- MEA1, MRCL3, SMAD2, LAPTM4A, SFRS12, FAM111A
- MEA1, MRCL3, SMAD2, LAPTM4A, CD1B, FAM111A
- MEA1, MRCL3, SMAD2, SFRS12, CD1B, FAM111A
- MEA1, MRCL3, LAPTM4A, SFRS12, CD1B, FAM111A
- MEA1, SMAD2, LAPTM4A, SFRS12, CD1B, FAM111A
- SPRY4, TLR8, SAMSN1, DGCR14, MRCL3, SMAD2
- SPRY4, TLR8, SAMSN1, DGCR14, MRCL3, LAPTM4A
- SPRY4, TLR8, SAMSN1, DGCR14, MRCL3, SFRS12
- SPRY4, TLR8, SAMSN1, DGCR14, MRCL3, CD1B
- SPRY4, TLR8, SAMSN1, DGCR14, MRCL3, FAM111A
- SPRY4, TLR8, SAMSN1, DGCR14, SMAD2, LAPTM4A
- SPRY4, TLR8, SAMSN1, DGCR14, SMAD2, SFRS12
- SPRY4, TLR8, SAMSN1, DGCR14, SMAD2, CD1B
- SPRY4, TLR8, SAMSN1, DGCR14, SMAD2, FAM111A
- SPRY4, TLR8, SAMSN1, DGCR14, LAPTM4A, SFRS12
- SPRY4, TLR8, SAMSN1, DGCR14, LAPTM4A, CD1B
- SPRY4, TLR8, SAMSN1, DGCR14, LAPTM4A, FAM111A
- SPRY4, TLR8, SAMSN1, DGCR14, SFRS12, CD1B
- SPRY4, TLR8, SAMSN1, DGCR14, SFRS12, FAM111A
- SPRY4, TLR8, SAMSN1, DGCR14, CD1B, FAM111A
- SPRY4, TLR8, SAMSN1, MRCL3, SMAD2, LAPTM4A
- SPRY4, TLR8, SAMSN1, MRCL3, SMAD2, SFRS12
- SPRY4, TLR8, SAMSN1, MRCL3, SMAD2, CD1B
- SPRY4, TLR8, SAMSN1, MRCL3, SMAD2, FAM111A
- SPRY4, TLR8, SAMSN1, MRCL3, LAPTM4A, SFRS12
- SPRY4, TLR8, SAMSN1, MRCL3, LAPTM4A, CD1B
- SPRY4, TLR8, SAMSN1, MRCL3, LAPTM4A, FAM111A
- SPRY4, TLR8, SAMSN1, MRCL3, SFRS12, CD1B
- SPRY4, TLR8, SAMSN1, MRCL3, SFRS12, FAM111A
- SPRY4, TLR8, SAMSN1, MRCL3, CD1B, FAM111A
- SPRY4, TLR8, SAMSN1, SMAD2, LAPTM4A, SFRS12
- SPRY4, TLR8, SAMSN1, SMAD2, LAPTM4A, CD1B
- SPRY4, TLR8, SAMSN1, SMAD2, LAPTM4A, FAM111A
- SPRY4, TLR8, SAMSN1, SMAD2, SFRS12, CD1B
- SPRY4, TLR8, SAMSN1, SMAD2, SFRS12, FAM111A
- SPRY4, TLR8, SAMSN1, SMAD2, CD1B, FAM111A
- SPRY4, TLR8, SAMSN1, LAPTM4A, SFRS12, CD1B
- SPRY4, TLR8, SAMSN1, LAPTM4A, SFRS12, FAM111A
- SPRY4, TLR8, SAMSN1, LAPTM4A, CD1B, FAM111A
- SPRY4, TLR8, SAMSN1, SFRS12, CD1B, FAM111A
- SPRY4, TLR8, DGCR14, MRCL3, SMAD2, LAPTM4A
- SPRY4, TLR8, DGCR14, MRCL3, SMAD2, SFRS12
- SPRY4, TLR8, DGCR14, MRCL3, SMAD2, CD1B
- SPRY4, TLR8, DGCR14, MRCL3, SMAD2, FAM111A
- SPRY4, TLR8, DGCR14, MRCL3, LAPTM4A, SFRS12
- SPRY4, TLR8, DGCR14, MRCL3, LAPTM4A, CD1B
- SPRY4, TLR8, DGCR14, MRCL3, LAPTM4A, FAM111A
- SPRY4, TLR8, DGCR14, MRCL3, SFRS12, CD1B
- SPRY4, TLR8, DGCR14, MRCL3, SFRS12, FAM111A
- SPRY4, TLR8, DGCR14, MRCL3, CD1B, FAM111A
- SPRY4, TLR8, DGCR14, SMAD2, LAPTM4A, SFRS12
- SPRY4, TLR8, DGCR14, SMAD2, LAPTM4A, CD1B
- SPRY4, TLR8, DGCR14, SMAD2, LAPTM4A, FAM111A

- SPRY4, TLR8, DGCR14, SMAD2, SFRS12, CD1B
- SPRY4, TLR8, DGCR14, SMAD2, SFRS12, FAM111A
- SPRY4, TLR8, DGCR14, SMAD2, CD1B, FAM111A
- SPRY4, TLR8, DGCR14, LAPTM4A, SFRS12, CD1B
- SPRY4, TLR8, DGCR14, LAPTM4A, SFRS12, FAM111A
- SPRY4, TLR8, DGCR14, LAPTM4A, CD1B, FAM111A
- SPRY4, TLR8, DGCR14, SFRS12, CD1B, FAM111A
- SPRY4, TLR8, MRCL3, SMAD2, LAPTM4A, SFRS12
- SPRY4, TLR8, MRCL3, SMAD2, LAPTM4A, CD1B
- SPRY4, TLR8, MRCL3, SMAD2, LAPTM4A, FAM111A
- SPRY4, TLR8, MRCL3, SMAD2, SFRS12, CD1B
- SPRY4, TLR8, MRCL3, SMAD2, SFRS12, FAM111A
- SPRY4, TLR8, MRCL3, SMAD2, CD1B, FAM111A
- SPRY4, TLR8, MRCL3, LAPTM4A, SFRS12, CD1B
- SPRY4, TLR8, MRCL3, LAPTM4A, SFRS12, FAM111A
- SPRY4, TLR8, MRCL3, LAPTM4A, CD1B, FAM111A
- SPRY4, TLR8, MRCL3, SFRS12, CD1B, FAM111A
- SPRY4, TLR8, SMAD2, LAPTM4A, SFRS12, CD1B
- SPRY4, TLR8, SMAD2, LAPTM4A, SFRS12, FAM111A
- SPRY4, TLR8, SMAD2, LAPTM4A, CD1B, FAM111A
- SPRY4, TLR8, SMAD2, SFRS12, CD1B, FAM111A
- SPRY4, TLR8, LAPTM4A, SFRS12, CD1B, FAM111A
- SPRY4, SAMSN1, DGCR14, MRCL3, SMAD2, LAPTM4A
- SPRY4, SAMSN1, DGCR14, MRCL3, SMAD2, SFRS12
- SPRY4, SAMSN1, DGCR14, MRCL3, SMAD2, CD1B
- SPRY4, SAMSN1, DGCR14, MRCL3, SMAD2, FAM111A
- SPRY4, SAMSN1, DGCR14, MRCL3, LAPTM4A, SFRS12
- SPRY4, SAMSN1, DGCR14, MRCL3, LAPTM4A, CD1B
- SPRY4, SAMSN1, DGCR14, MRCL3, LAPTM4A, FAM111A
- SPRY4, SAMSN1, DGCR14, MRCL3, SFRS12, CD1B
- SPRY4, SAMSN1, DGCR14, MRCL3, SFRS12, FAM111A
- SPRY4, SAMSN1, DGCR14, MRCL3, CD1B, FAM111A
- SPRY4, SAMSN1, DGCR14, SMAD2, LAPTM4A, SFRS12
- SPRY4, SAMSN1, DGCR14, SMAD2, LAPTM4A, CD1B
- SPRY4, SAMSN1, DGCR14, SMAD2, LAPTM4A, FAM111A
- SPRY4, SAMSN1, DGCR14, SMAD2, SFRS12, CD1B
- SPRY4, SAMSN1, DGCR14, SMAD2, SFRS12, FAM111A
- SPRY4, SAMSN1, DGCR14, SMAD2, CD1B, FAM111A
- SPRY4, SAMSN1, DGCR14, LAPTM4A, SFRS12, CD1B
- SPRY4, SAMSN1, DGCR14, LAPTM4A, SFRS12, FAM111A
- SPRY4, SAMSN1, DGCR14, LAPTM4A, CD1B, FAM111A
- SPRY4, SAMSN1, DGCR14, SFRS12, CD1B, FAM111A
- SPRY4, SAMSN1, MRCL3, SMAD2, LAPTM4A, SFRS12
- SPRY4, SAMSN1, MRCL3, SMAD2, LAPTM4A, CD1B
- SPRY4, SAMSN1, MRCL3, SMAD2, LAPTM4A, FAM111A
- SPRY4, SAMSN1, MRCL3, SMAD2, SFRS12, CD1B
- SPRY4, SAMSN1, MRCL3, SMAD2, SFRS12, FAM111A
- SPRY4, SAMSN1, MRCL3, SMAD2, CD1B, FAM111A
- SPRY4, SAMSN1, MRCL3, LAPTM4A, SFRS12, CD1B
- SPRY4, SAMSN1, MRCL3, LAPTM4A, SFRS12, FAM111A
- SPRY4, SAMSN1, MRCL3, LAPTM4A, CD1B, FAM111A
- SPRY4, SAMSN1, MRCL3, SFRS12, CD1B, FAM111A
- SPRY4, SAMSN1, SMAD2, LAPTM4A, SFRS12, CD1B
- SPRY4, SAMSN1, SMAD2, LAPTM4A, SFRS12, FAM111A
- SPRY4, SAMSN1, SMAD2, LAPTM4A, CD1B, FAM111A
- SPRY4, SAMSN1, SMAD2, SFRS12, CD1B, FAM111A
- SPRY4, SAMSN1, LAPTM4A, SFRS12, CD1B, FAM111A
- SPRY4, DGCR14, MRCL3, SMAD2, LAPTM4A, SFRS12

- SPRY4, DGCR14, MRCL3, SMAD2, LAPTM4A, CD1B
- SPRY4, DGCR14, MRCL3, SMAD2, LAPTM4A, FAM111A
- SPRY4, DGCR14, MRCL3, SMAD2, SFRS12, CD1B
- SPRY4, DGCR14, MRCL3, SMAD2, SFRS12, FAM111A
- SPRY4, DGCR14, MRCL3, SMAD2, CD1B, FAM111A
- SPRY4, DGCR14, MRCL3, LAPTM4A, SFRS12, CD1B
- SPRY4, DGCR14, MRCL3, LAPTM4A, SFRS12, FAM111A
- SPRY4, DGCR14, MRCL3, LAPTM4A, CD1B, FAM111A
- SPRY4, DGCR14, MRCL3, SFRS12, CD1B, FAM111A
- SPRY4, DGCR14, SMAD2, LAPTM4A, SFRS12, CD1B
- SPRY4, DGCR14, SMAD2, LAPTM4A, SFRS12, FAM111A
- SPRY4, DGCR14, SMAD2, LAPTM4A, CD1B, FAM111A
- SPRY4, DGCR14, SMAD2, SFRS12, CD1B, FAM111A
- SPRY4, DGCR14, LAPTM4A, SFRS12, CD1B, FAM111A
- SPRY4, MRCL3, SMAD2, LAPTM4A, SFRS12, CD1B
- SPRY4, MRCL3, SMAD2, LAPTM4A, SFRS12, FAM111A
- SPRY4, MRCL3, SMAD2, LAPTM4A, CD1B, FAM111A
- SPRY4, MRCL3, SMAD2, SFRS12, CD1B, FAM111A
- SPRY4, MRCL3, LAPTM4A, SFRS12, CD1B, FAM111A
- SPRY4, SMAD2, LAPTM4A, SFRS12, CD1B, FAM111A
- TLR8, SAMSN1, DGCR14, MRCL3, SMAD2, LAPTM4A
- TLR8, SAMSN1, DGCR14, MRCL3, SMAD2, SFRS12
- TLR8, SAMSN1, DGCR14, MRCL3, SMAD2, CD1B
- TLR8, SAMSN1, DGCR14, MRCL3, SMAD2, FAM111A
- TLR8, SAMSN1, DGCR14, MRCL3, LAPTM4A, SFRS12
- TLR8, SAMSN1, DGCR14, MRCL3, LAPTM4A, CD1B
- TLR8, SAMSN1, DGCR14, MRCL3, LAPTM4A, FAM111A
- TLR8, SAMSN1, DGCR14, MRCL3, SFRS12, CD1B
- TLR8, SAMSN1, DGCR14, MRCL3, SFRS12, FAM111A
- TLR8, SAMSN1, DGCR14, MRCL3, CD1B, FAM111A
- TLR8, SAMSN1, DGCR14, SMAD2, LAPTM4A, SFRS12
- TLR8, SAMSN1, DGCR14, SMAD2, LAPTM4A, CD1B
- TLR8, SAMSN1, DGCR14, SMAD2, LAPTM4A, FAM111A
- TLR8, SAMSN1, DGCR14, SMAD2, SFRS12, CD1B
- TLR8, SAMSN1, DGCR14, SMAD2, SFRS12, FAM111A
- TLR8, SAMSN1, DGCR14, SMAD2, CD1B, FAM111A
- TLR8, SAMSN1, DGCR14, LAPTM4A, SFRS12, CD1B
- TLR8, SAMSN1, DGCR14, LAPTM4A, SFRS12, FAM111A
- TLR8, SAMSN1, DGCR14, LAPTM4A, CD1B, FAM111A
- TLR8, SAMSN1, DGCR14, SFRS12, CD1B, FAM111A
- TLR8, SAMSN1, MRCL3, SMAD2, LAPTM4A, SFRS12
- TLR8, SAMSN1, MRCL3, SMAD2, LAPTM4A, CD1B
- TLR8, SAMSN1, MRCL3, SMAD2, LAPTM4A, FAM111A
- TLR8, SAMSN1, MRCL3, SMAD2, SFRS12, CD1B
- TLR8, SAMSN1, MRCL3, SMAD2, SFRS12, FAM111A
- TLR8, SAMSN1, MRCL3, SMAD2, CD1B, FAM111A
- TLR8, SAMSN1, MRCL3, LAPTM4A, SFRS12, CD1B
- TLR8, SAMSN1, MRCL3, LAPTM4A, SFRS12, FAM111A
- TLR8, SAMSN1, MRCL3, LAPTM4A, CD1B, FAM111A
- TLR8, SAMSN1, MRCL3, SFRS12, CD1B, FAM111A
- TLR8, SAMSN1, SMAD2, LAPTM4A, SFRS12, CD1B
- TLR8, SAMSN1, SMAD2, LAPTM4A, SFRS12, FAM111A
- TLR8, SAMSN1, SMAD2, LAPTM4A, CD1B, FAM111A
- TLR8, SAMSN1, SMAD2, SFRS12, CD1B, FAM111A
- TLR8, SAMSN1, LAPTM4A, SFRS12, CD1B, FAM111A
- TLR8, DGCR14, MRCL3, SMAD2, LAPTM4A, SFRS12
- TLR8, DGCR14, MRCL3, SMAD2, LAPTM4A, CD1B
- TLR8, DGCR14, MRCL3, SMAD2, LAPTM4A, FAM111A

- TLR8, DGCR14, MRCL3, SMAD2, SFRS12, CD1B
- TLR8, DGCR14, MRCL3, SMAD2, SFRS12, FAM111A
- TLR8, DGCR14, MRCL3, SMAD2, CD1B, FAM111A
- TLR8, DGCR14, MRCL3, LAPTM4A, SFRS12, CD1B
- TLR8, DGCR14, MRCL3, LAPTM4A, SFRS12, FAM111A
- TLR8, DGCR14, MRCL3, LAPTM4A, CD1B, FAM111A
- TLR8, DGCR14, MRCL3, SFRS12, CD1B, FAM111A
- TLR8, DGCR14, SMAD2, LAPTM4A, SFRS12, CD1B
- TLR8, DGCR14, SMAD2, LAPTM4A, SFRS12, FAM111A
- TLR8, DGCR14, SMAD2, LAPTM4A, CD1B, FAM111A
- TLR8, DGCR14, SMAD2, SFRS12, CD1B, FAM111A
- TLR8, DGCR14, LAPTM4A, SFRS12, CD1B, FAM111A
- TLR8, MRCL3, SMAD2, LAPTM4A, SFRS12, CD1B
- TLR8, MRCL3, SMAD2, LAPTM4A, SFRS12, FAM111A
- TLR8, MRCL3, SMAD2, LAPTM4A, CD1B, FAM111A
- TLR8, MRCL3, SMAD2, SFRS12, CD1B, FAM111A
- TLR8, MRCL3, LAPTM4A, SFRS12, CD1B, FAM111A
- TLR8, SMAD2, LAPTM4A, SFRS12, CD1B, FAM111A
- SAMSN1, DGCR14, MRCL3, SMAD2, LAPTM4A, SFRS12
- SAMSN1, DGCR14, MRCL3, SMAD2, LAPTM4A, CD1B
- SAMSN1, DGCR14, MRCL3, SMAD2, LAPTM4A, FAM111A
- SAMSN1, DGCR14, MRCL3, SMAD2, SFRS12, CD1B
- SAMSN1, DGCR14, MRCL3, SMAD2, SFRS12, FAM111A
- SAMSN1, DGCR14, MRCL3, SMAD2, CD1B, FAM111A
- SAMSN1, DGCR14, MRCL3, LAPTM4A, SFRS12, CD1B
- SAMSN1, DGCR14, MRCL3, LAPTM4A, SFRS12, FAM111A
- SAMSN1, DGCR14, MRCL3, LAPTM4A, CD1B, FAM111A
- SAMSN1, DGCR14, MRCL3, SFRS12, CD1B, FAM111A
- SAMSN1, DGCR14, SMAD2, LAPTM4A, SFRS12, CD1B
- SAMSN1, DGCR14, SMAD2, LAPTM4A, SFRS12, FAM111A
- SAMSN1, DGCR14, SMAD2, LAPTM4A, CD1B, FAM111A
- SAMSN1, DGCR14, SMAD2, SFRS12, CD1B, FAM111A
- SAMSN1, DGCR14, LAPTM4A, SFRS12, CD1B, FAM111A
- SAMSN1, MRCL3, SMAD2, LAPTM4A, SFRS12, CD1B
- SAMSN1, MRCL3, SMAD2, LAPTM4A, SFRS12, FAM111A
- SAMSN1, MRCL3, SMAD2, LAPTM4A, CD1B, FAM111A
- SAMSN1, MRCL3, SMAD2, SFRS12, CD1B, FAM111A
- SAMSN1, MRCL3, LAPTM4A, SFRS12, CD1B, FAM111A
- SAMSN1, SMAD2, LAPTM4A, SFRS12, CD1B, FAM111A
- DGCR14, MRCL3, SMAD2, LAPTM4A, SFRS12, CD1B
- DGCR14, MRCL3, SMAD2, LAPTM4A, SFRS12, FAM111A
- DGCR14, MRCL3, SMAD2, LAPTM4A, CD1B, FAM111A
- DGCR14, MRCL3, SMAD2, SFRS12, CD1B, FAM111A
- DGCR14, MRCL3, LAPTM4A, SFRS12, CD1B, FAM111A
- DGCR14, SMAD2, LAPTM4A, SFRS12, CD1B, FAM111A
- MRCL3, SMAD2, LAPTM4A, SFRS12, CD1B, FAM111A

Thus, combinations of 7 genes suitable for the staging of the tumor include

- MEA1, SPRY4, TLR8, SAMSN1, DGCR14, MRCL3, SMAD2
- MEA1, SPRY4, TLR8, SAMSN1, DGCR14, MRCL3, LAPTM4A
- MEA1, SPRY4, TLR8, SAMSN1, DGCR14, MRCL3, SFRS12
- MEA1, SPRY4, TLR8, SAMSN1, DGCR14, MRCL3, CD1B
- MEA1, SPRY4, TLR8, SAMSN1, DGCR14, MRCL3, FAM111A
- MEA1, SPRY4, TLR8, SAMSN1, DGCR14, SMAD2, LAPTM4A
- MEA1, SPRY4, TLR8, SAMSN1, DGCR14, SMAD2, SFRS12
- MEA1, SPRY4, TLR8, SAMSN1, DGCR14, SMAD2, CD1B
- MEA1, SPRY4, TLR8, SAMSN1, DGCR14, SMAD2, FAM111A

- MEA1, SPRY4, TLR8, SAMSN1, DGCR14, LAPTM4A, SFRS12
- MEA1, SPRY4, TLR8, SAMSN1, DGCR14, LAPTM4A, CD1B
- MEA1, SPRY4, TLR8, SAMSN1, DGCR14, LAPTM4A, FAM111A
- MEA1, SPRY4, TLR8, SAMSN1, DGCR14, SFRS12, CD1B
- MEA1, SPRY4, TLR8, SAMSN1, DGCR14, SFRS12, FAM111A
- MEA1, SPRY4, TLR8, SAMSN1, DGCR14, CD1B, FAM111A
- MEA1, SPRY4, TLR8, SAMSN1, MRCL3, SMAD2, LAPTM4A
- MEA1, SPRY4, TLR8, SAMSN1, MRCL3, SMAD2, SFRS12
- MEA1, SPRY4, TLR8, SAMSN1, MRCL3, SMAD2, CD1B
- MEA1, SPRY4, TLR8, SAMSN1, MRCL3, SMAD2, FAM111A
- MEA1, SPRY4, TLR8, SAMSN1, MRCL3, LAPTM4A, SFRS12
- MEA1, SPRY4, TLR8, SAMSN1, MRCL3, LAPTM4A, CD1B
- MEA1, SPRY4, TLR8, SAMSN1, MRCL3, LAPTM4A, FAM111A
- MEA1, SPRY4, TLR8, SAMSN1, MRCL3, SFRS12, CD1B
- MEA1, SPRY4, TLR8, SAMSN1, MRCL3, SFRS12, FAM111A
- MEA1, SPRY4, TLR8, SAMSN1, MRCL3, CD1B, FAM111A
- MEA1, SPRY4, TLR8, SAMSN1, SMAD2, LAPTM4A, SFRS12
- MEA1, SPRY4, TLR8, SAMSN1, SMAD2, LAPTM4A, CD1B
- MEA1, SPRY4, TLR8, SAMSN1, SMAD2, LAPTM4A, FAM111A
- MEA1, SPRY4, TLR8, SAMSN1, SMAD2, SFRS12, CD1B
- MEA1, SPRY4, TLR8, SAMSN1, SMAD2, SFRS12, FAM111A
- MEA1, SPRY4, TLR8, SAMSN1, SMAD2, CD1B, FAM111A
- MEA1, SPRY4, TLR8, SAMSN1, LAPTM4A, SFRS12, CD1B
- MEA1, SPRY4, TLR8, SAMSN1, LAPTM4A, SFRS12, FAM111A
- MEA1, SPRY4, TLR8, SAMSN1, LAPTM4A, CD1B, FAM111A
- MEA1, SPRY4, TLR8, SAMSN1, SFRS12, CD1B, FAM111A
- MEA1, SPRY4, TLR8, DGCR14, MRCL3, SMAD2, LAPTM4A
- MEA1, SPRY4, TLR8, DGCR14, MRCL3, SMAD2, SFRS12
- MEA1, SPRY4, TLR8, DGCR14, MRCL3, SMAD2, CD1B
- MEA1, SPRY4, TLR8, DGCR14, MRCL3, SMAD2, FAM111A
- MEA1, SPRY4, TLR8, DGCR14, MRCL3, LAPTM4A, SFRS12
- MEA1, SPRY4, TLR8, DGCR14, MRCL3, LAPTM4A, CD1B
- MEA1, SPRY4, TLR8, DGCR14, MRCL3, LAPTM4A, FAM111A
- MEA1, SPRY4, TLR8, DGCR14, MRCL3, SFRS12, CD1B
- MEA1, SPRY4, TLR8, DGCR14, MRCL3, SFRS12, FAM111A
- MEA1, SPRY4, TLR8, DGCR14, MRCL3, CD1B, FAM111A
- MEA1, SPRY4, TLR8, DGCR14, SMAD2, LAPTM4A, SFRS12
- MEA1, SPRY4, TLR8, DGCR14, SMAD2, LAPTM4A, CD1B
- MEA1, SPRY4, TLR8, DGCR14, SMAD2, LAPTM4A, FAM111A
- MEA1, SPRY4, TLR8, DGCR14, SMAD2, SFRS12, CD1B
- MEA1, SPRY4, TLR8, DGCR14, SMAD2, SFRS12, FAM111A
- MEA1, SPRY4, TLR8, DGCR14, SMAD2, CD1B, FAM111A
- MEA1, SPRY4, TLR8, DGCR14, LAPTM4A, SFRS12, CD1B
- MEA1, SPRY4, TLR8, DGCR14, LAPTM4A, SFRS12, FAM111A
- MEA1, SPRY4, TLR8, DGCR14, LAPTM4A, CD1B, FAM111A
- MEA1, SPRY4, TLR8, DGCR14, SFRS12, CD1B, FAM111A
- MEA1, SPRY4, TLR8, MRCL3, SMAD2, LAPTM4A, SFRS12
- MEA1, SPRY4, TLR8, MRCL3, SMAD2, LAPTM4A, CD1B
- MEA1, SPRY4, TLR8, MRCL3, SMAD2, LAPTM4A, FAM111A
- MEA1, SPRY4, TLR8, MRCL3, SMAD2, SFRS12, CD1B
- MEA1, SPRY4, TLR8, MRCL3, SMAD2, SFRS12, FAM111A
- MEA1, SPRY4, TLR8, MRCL3, SMAD2, CD1B, FAM111A
- MEA1, SPRY4, TLR8, MRCL3, LAPTM4A, SFRS12, CD1B
- MEA1, SPRY4, TLR8, MRCL3, LAPTM4A, SFRS12, FAM111A
- MEA1, SPRY4, TLR8, MRCL3, LAPTM4A, CD1B, FAM111A
- MEA1, SPRY4, TLR8, MRCL3, SFRS12, CD1B, FAM111A
- MEA1, SPRY4, TLR8, SMAD2, LAPTM4A, SFRS12, CD1B
- MEA1, SPRY4, TLR8, SMAD2, LAPTM4A, SFRS12, FAM111A

- MEA1, SPRY4, TLR8, SMAD2, LAPTM4A, CD1B, FAM111A
- MEA1, SPRY4, TLR8, SMAD2, SFRS12, CD1B, FAM111A
- MEA1, SPRY4, TLR8, LAPTM4A, SFRS12, CD1B, FAM111A
- MEA1, SPRY4, SAMSN1, DGCR14, MRCL3, SMAD2, LAPTM4A
- MEA1, SPRY4, SAMSN1, DGCR14, MRCL3, SMAD2, SFRS12
- MEA1, SPRY4, SAMSN1, DGCR14, MRCL3, SMAD2, CD1B
- MEA1, SPRY4, SAMSN1, DGCR14, MRCL3, SMAD2, FAM111A
- MEA1, SPRY4, SAMSN1, DGCR14, MRCL3, LAPTM4A, SFRS12
- MEA1, SPRY4, SAMSN1, DGCR14, MRCL3, LAPTM4A, CD1B
- MEA1, SPRY4, SAMSN1, DGCR14, MRCL3, LAPTM4A, FAM111A
- MEA1, SPRY4, SAMSN1, DGCR14, MRCL3, SFRS12, CD1B
- MEA1, SPRY4, SAMSN1, DGCR14, MRCL3, SFRS12, FAM111A
- MEA1, SPRY4, SAMSN1, DGCR14, MRCL3, CD1B, FAM111A
- MEA1, SPRY4, SAMSN1, DGCR14, SMAD2, LAPTM4A, SFRS12
- MEA1, SPRY4, SAMSN1, DGCR14, SMAD2, LAPTM4A, CD1B
- MEA1, SPRY4, SAMSN1, DGCR14, SMAD2, LAPTM4A, FAM111A
- MEA1, SPRY4, SAMSN1, DGCR14, SMAD2, SFRS12, CD1B
- MEA1, SPRY4, SAMSN1, DGCR14, SMAD2, SFRS12, FAM111A
- MEA1, SPRY4, SAMSN1, DGCR14, SMAD2, CD1B, FAM111A
- MEA1, SPRY4, SAMSN1, DGCR14, LAPTM4A, SFRS12, CD1B
- MEA1, SPRY4, SAMSN1, DGCR14, LAPTM4A, SFRS12, FAM111A
- MEA1, SPRY4, SAMSN1, DGCR14, LAPTM4A, CD1B, FAM111A
- MEA1, SPRY4, SAMSN1, DGCR14, SFRS12, CD1B, FAM111A
- MEA1, SPRY4, SAMSN1, MRCL3, SMAD2, LAPTM4A, SFRS12
- MEA1, SPRY4, SAMSN1, MRCL3, SMAD2, LAPTM4A, CD1B
- MEA1, SPRY4, SAMSN1, MRCL3, SMAD2, LAPTM4A, FAM111A
- MEA1, SPRY4, SAMSN1, MRCL3, SMAD2, SFRS12, CD1B
- MEA1, SPRY4, SAMSN1, MRCL3, SMAD2, SFRS12, FAM111A
- MEA1, SPRY4, SAMSN1, MRCL3, SMAD2, CD1B, FAM111A
- MEA1, SPRY4, SAMSN1, MRCL3, LAPTM4A, SFRS12, CD1B
- MEA1, SPRY4, SAMSN1, MRCL3, LAPTM4A, SFRS12, FAM111A
- MEA1, SPRY4, SAMSN1, MRCL3, LAPTM4A, CD1B, FAM111A
- MEA1, SPRY4, SAMSN1, MRCL3, SFRS12, CD1B, FAM111A
- MEA1, SPRY4, SAMSN1, SMAD2, LAPTM4A, SFRS12, CD1B
- MEA1, SPRY4, SAMSN1, SMAD2, LAPTM4A, SFRS12, FAM111A
- MEA1, SPRY4, SAMSN1, SMAD2, LAPTM4A, CD1B, FAM111A
- MEA1, SPRY4, SAMSN1, SMAD2, SFRS12, CD1B, FAM111A
- MEA1, SPRY4, SAMSN1, LAPTM4A, SFRS12, CD1B, FAM111A
- MEA1, SPRY4, DGCR14, MRCL3, SMAD2, LAPTM4A, SFRS12
- MEA1, SPRY4, DGCR14, MRCL3, SMAD2, LAPTM4A, CD1B
- MEA1, SPRY4, DGCR14, MRCL3, SMAD2, LAPTM4A, FAM111A
- MEA1, SPRY4, DGCR14, MRCL3, SMAD2, SFRS12, CD1B
- MEA1, SPRY4, DGCR14, MRCL3, SMAD2, SFRS12, FAM111A
- MEA1, SPRY4, DGCR14, MRCL3, SMAD2, CD1B, FAM111A
- MEA1, SPRY4, DGCR14, MRCL3, LAPTM4A, SFRS12, CD1B
- MEA1, SPRY4, DGCR14, MRCL3, LAPTM4A, SFRS12, FAM111A
- MEA1, SPRY4, DGCR14, MRCL3, LAPTM4A, CD1B, FAM111A
- MEA1, SPRY4, DGCR14, MRCL3, SFRS12, CD1B, FAM111A
- MEA1, SPRY4, DGCR14, SMAD2, LAPTM4A, SFRS12, CD1B
- MEA1, SPRY4, DGCR14, SMAD2, LAPTM4A, SFRS12, FAM111A
- MEA1, SPRY4, DGCR14, SMAD2, LAPTM4A, CD1B, FAM111A
- MEA1, SPRY4, DGCR14, SMAD2, SFRS12, CD1B, FAM111A
- MEA1, SPRY4, DGCR14, LAPTM4A, SFRS12, CD1B, FAM111A
- MEA1, SPRY4, MRCL3, SMAD2, LAPTM4A, SFRS12, CD1B
- MEA1, SPRY4, MRCL3, SMAD2, LAPTM4A, SFRS12, FAM111A
- MEA1, SPRY4, MRCL3, SMAD2, LAPTM4A, CD1B, FAM111A
- MEA1, SPRY4, MRCL3, SMAD2, SFRS12, CD1B, FAM111A
- MEA1, SPRY4, MRCL3, LAPTM4A, SFRS12, CD1B, FAM111A

- MEA1, SPRY4, SMAD2, LAPTM4A, SFRS12, CD1B, FAM111A
- MEA1, TLR8, SAMSN1, DGCR14, MRCL3, SMAD2, LAPTM4A
- MEA1, TLR8, SAMSN1, DGCR14, MRCL3, SMAD2, SFRS12
- MEA1, TLR8, SAMSN1, DGCR14, MRCL3, SMAD2, CD1B
- MEA1, TLR8, SAMSN1, DGCR14, MRCL3, SMAD2, FAM111A
- MEA1, TLR8, SAMSN1, DGCR14, MRCL3, LAPTM4A, SFRS12
- MEA1, TLR8, SAMSN1, DGCR14, MRCL3, LAPTM4A, CD1B
- MEA1, TLR8, SAMSN1, DGCR14, MRCL3, LAPTM4A, FAM111A
- MEA1, TLR8, SAMSN1, DGCR14, MRCL3, SFRS12, CD1B
- MEA1, TLR8, SAMSN1, DGCR14, MRCL3, SFRS12, FAM111A
- MEA1, TLR8, SAMSN1, DGCR14, MRCL3, CD1B, FAM111A
- MEA1, TLR8, SAMSN1, DGCR14, SMAD2, LAPTM4A, SFRS12
- MEA1, TLR8, SAMSN1, DGCR14, SMAD2, LAPTM4A, CD1B
- MEA1, TLR8, SAMSN1, DGCR14, SMAD2, LAPTM4A, FAM111A
- MEA1, TLR8, SAMSN1, DGCR14, SMAD2, SFRS12, CD1B
- MEA1, TLR8, SAMSN1, DGCR14, SMAD2, SFRS12, FAM111A
- MEA1, TLR8, SAMSN1, DGCR14, SMAD2, CD1B, FAM111A
- MEA1, TLR8, SAMSN1, DGCR14, LAPTM4A, SFRS12, CD1B
- MEA1, TLR8, SAMSN1, DGCR14, LAPTM4A, SFRS12, FAM111A
- MEA1, TLR8, SAMSN1, DGCR14, LAPTM4A, CD1B, FAM111A
- MEA1, TLR8, SAMSN1, DGCR14, SFRS12, CD1B, FAM111A
- MEA1, TLR8, SAMSN1, MRCL3, SMAD2, LAPTM4A, SFRS12
- MEA1, TLR8, SAMSN1, MRCL3, SMAD2, LAPTM4A, CD1B
- MEA1, TLR8, SAMSN1, MRCL3, SMAD2, LAPTM4A, FAM111A
- MEA1, TLR8, SAMSN1, MRCL3, SMAD2, SFRS12, CD1B
- MEA1, TLR8, SAMSN1, MRCL3, SMAD2, SFRS12, FAM111A
- MEA1, TLR8, SAMSN1, MRCL3, SMAD2, CD1B, FAM111A
- MEA1, TLR8, SAMSN1, MRCL3, LAPTM4A, SFRS12, CD1B
- MEA1, TLR8, SAMSN1, MRCL3, LAPTM4A, SFRS12, FAM111A
- MEA1, TLR8, SAMSN1, MRCL3, LAPTM4A, CD1B, FAM111A
- MEA1, TLR8, SAMSN1, MRCL3, SFRS12, CD1B, FAM111A
- MEA1, TLR8, SAMSN1, SMAD2, LAPTM4A, SFRS12, CD1B
- MEA1, TLR8, SAMSN1, SMAD2, LAPTM4A, SFRS12, FAM111A
- MEA1, TLR8, SAMSN1, SMAD2, LAPTM4A, CD1B, FAM111A
- MEA1, TLR8, SAMSN1, SMAD2, SFRS12, CD1B, FAM111A
- MEA1, TLR8, SAMSN1, LAPTM4A, SFRS12, CD1B, FAM111A
- MEA1, TLR8, DGCR14, MRCL3, SMAD2, LAPTM4A, SFRS12
- MEA1, TLR8, DGCR14, MRCL3, SMAD2, LAPTM4A, CD1B
- MEA1, TLR8, DGCR14, MRCL3, SMAD2, LAPTM4A, FAM111A
- MEA1, TLR8, DGCR14, MRCL3, SMAD2, SFRS12, CD1B
- MEA1, TLR8, DGCR14, MRCL3, SMAD2, SFRS12, FAM111A
- MEA1, TLR8, DGCR14, MRCL3, SMAD2, CD1B, FAM111A
- MEA1, TLR8, DGCR14, MRCL3, LAPTM4A, SFRS12, CD1B
- MEA1, TLR8, DGCR14, MRCL3, LAPTM4A, SFRS12, FAM111A
- MEA1, TLR8, DGCR14, MRCL3, LAPTM4A, CD1B, FAM111A
- MEA1, TLR8, DGCR14, MRCL3, SFRS12, CD1B, FAM111A
- MEA1, TLR8, DGCR14, SMAD2, LAPTM4A, SFRS12, CD1B
- MEA1, TLR8, DGCR14, SMAD2, LAPTM4A, SFRS12, FAM111A
- MEA1, TLR8, DGCR14, SMAD2, LAPTM4A, CD1B, FAM111A
- MEA1, TLR8, DGCR14, SMAD2, SFRS12, CD1B, FAM111A
- MEA1, TLR8, DGCR14, LAPTM4A, SFRS12, CD1B, FAM111A
- MEA1, TLR8, MRCL3, SMAD2, LAPTM4A, SFRS12, CD1B
- MEA1, TLR8, MRCL3, SMAD2, LAPTM4A, SFRS12, FAM111A
- MEA1, TLR8, MRCL3, SMAD2, LAPTM4A, CD1B, FAM111A
- MEA1, TLR8, MRCL3, SMAD2, SFRS12, CD1B, FAM111A
- MEA1, TLR8, MRCL3, LAPTM4A, SFRS12, CD1B, FAM111A
- MEA1, TLR8, SMAD2, LAPTM4A, SFRS12, CD1B, FAM111A
- MEA1, SAMSN1, DGCR14, MRCL3, SMAD2, LAPTM4A, SFRS12

- MEA1, SAMSN1, DGCR14, MRCL3, SMAD2, LAPTM4A, CD1B
- MEA1, SAMSN1, DGCR14, MRCL3, SMAD2, LAPTM4A, FAM111A
- MEA1, SAMSN1, DGCR14, MRCL3, SMAD2, SFRS12, CD1B
- MEA1, SAMSN1, DGCR14, MRCL3, SMAD2, SFRS12, FAM111A
- MEA1, SAMSN1, DGCR14, MRCL3, SMAD2, CD1B, FAM111A
- MEA1, SAMSN1, DGCR14, MRCL3, LAPTM4A, SFRS12, CD1B
- MEA1, SAMSN1, DGCR14, MRCL3, LAPTM4A, SFRS12, FAM111A
- MEA1, SAMSN1, DGCR14, MRCL3, LAPTM4A, CD1B, FAM111A
- MEA1, SAMSN1, DGCR14, MRCL3, SFRS12, CD1B, FAM111A
- MEA1, SAMSN1, DGCR14, SMAD2, LAPTM4A, SFRS12, CD1B
- MEA1, SAMSN1, DGCR14, SMAD2, LAPTM4A, SFRS12, FAM111A
- MEA1, SAMSN1, DGCR14, SMAD2, LAPTM4A, CD1B, FAM111A
- MEA1, SAMSN1, DGCR14, SMAD2, SFRS12, CD1B, FAM111A
- MEA1, SAMSN1, DGCR14, LAPTM4A, SFRS12, CD1B, FAM111A
- MEA1, SAMSN1, MRCL3, SMAD2, LAPTM4A, SFRS12, CD1B
- MEA1, SAMSN1, MRCL3, SMAD2, LAPTM4A, SFRS12, FAM111A
- MEA1, SAMSN1, MRCL3, SMAD2, LAPTM4A, CD1B, FAM111A
- MEA1, SAMSN1, MRCL3, SMAD2, SFRS12, CD1B, FAM111A
- MEA1, SAMSN1, MRCL3, LAPTM4A, SFRS12, CD1B, FAM111A
- MEA1, SAMSN1, SMAD2, LAPTM4A, SFRS12, CD1B, FAM111A
- MEA1, DGCR14, MRCL3, SMAD2, LAPTM4A, SFRS12, CD1B
- MEA1, DGCR14, MRCL3, SMAD2, LAPTM4A, SFRS12, FAM111A
- MEA1, DGCR14, MRCL3, SMAD2, LAPTM4A, CD1B, FAM111A
- MEA1, DGCR14, MRCL3, SMAD2, SFRS12, CD1B, FAM111A
- MEA1, DGCR14, MRCL3, LAPTM4A, SFRS12, CD1B, FAM111A
- MEA1, DGCR14, SMAD2, LAPTM4A, SFRS12, CD1B, FAM111A
- MEA1, MRCL3, SMAD2, LAPTM4A, SFRS12, CD1B, FAM111A
- SPRY4, TLR8, SAMSN1, DGCR14, MRCL3, SMAD2, LAPTM4A
- SPRY4, TLR8, SAMSN1, DGCR14, MRCL3, SMAD2, SFRS12
- SPRY4, TLR8, SAMSN1, DGCR14, MRCL3, SMAD2, CD1B
- SPRY4, TLR8, SAMSN1, DGCR14, MRCL3, SMAD2, FAM111A
- SPRY4, TLR8, SAMSN1, DGCR14, MRCL3, LAPTM4A, SFRS12
- SPRY4, TLR8, SAMSN1, DGCR14, MRCL3, LAPTM4A, CD1B
- SPRY4, TLR8, SAMSN1, DGCR14, MRCL3, LAPTM4A, FAM111A
- SPRY4, TLR8, SAMSN1, DGCR14, MRCL3, SFRS12, CD1B
- SPRY4, TLR8, SAMSN1, DGCR14, MRCL3, SFRS12, FAM111A
- SPRY4, TLR8, SAMSN1, DGCR14, MRCL3, CD1B, FAM111A
- SPRY4, TLR8, SAMSN1, DGCR14, SMAD2, LAPTM4A, SFRS12
- SPRY4, TLR8, SAMSN1, DGCR14, SMAD2, LAPTM4A, CD1B
- SPRY4, TLR8, SAMSN1, DGCR14, SMAD2, LAPTM4A, FAM111A
- SPRY4, TLR8, SAMSN1, DGCR14, SMAD2, SFRS12, CD1B
- SPRY4, TLR8, SAMSN1, DGCR14, SMAD2, SFRS12, FAM111A
- SPRY4, TLR8, SAMSN1, DGCR14, SMAD2, CD1B, FAM111A
- SPRY4, TLR8, SAMSN1, DGCR14, LAPTM4A, SFRS12, CD1B
- SPRY4, TLR8, SAMSN1, DGCR14, LAPTM4A, SFRS12, FAM111A
- SPRY4, TLR8, SAMSN1, DGCR14, LAPTM4A, CD1B, FAM111A
- SPRY4, TLR8, SAMSN1, DGCR14, SFRS12, CD1B, FAM111A
- SPRY4, TLR8, SAMSN1, MRCL3, SMAD2, LAPTM4A, SFRS12
- SPRY4, TLR8, SAMSN1, MRCL3, SMAD2, LAPTM4A, CD1B
- SPRY4, TLR8, SAMSN1, MRCL3, SMAD2, LAPTM4A, FAM111A
- SPRY4, TLR8, SAMSN1, MRCL3, SMAD2, SFRS12, CD1B
- SPRY4, TLR8, SAMSN1, MRCL3, SMAD2, SFRS12, FAM111A
- SPRY4, TLR8, SAMSN1, MRCL3, SMAD2, CD1B, FAM111A
- SPRY4, TLR8, SAMSN1, MRCL3, LAPTM4A, SFRS12, CD1B
- SPRY4, TLR8, SAMSN1, MRCL3, LAPTM4A, SFRS12, FAM111A
- SPRY4, TLR8, SAMSN1, MRCL3, LAPTM4A, CD1B, FAM111A
- SPRY4, TLR8, SAMSN1, MRCL3, SFRS12, CD1B, FAM111A
- SPRY4, TLR8, SAMSN1, SMAD2, LAPTM4A, SFRS12, CD1B

- SPRY4, TLR8, SAMSN1, SMAD2, LAPTM4A, SFRS12, FAM111A
- SPRY4, TLR8, SAMSN1, SMAD2, LAPTM4A, CD1B, FAM111A
- SPRY4, TLR8, SAMSN1, SMAD2, SFRS12, CD1B, FAM111A
- SPRY4, TLR8, SAMSN1, LAPTM4A, SFRS12, CD1B, FAM111A
- SPRY4, TLR8, DGCR14, MRCL3, SMAD2, LAPTM4A, SFRS12
- SPRY4, TLR8, DGCR14, MRCL3, SMAD2, LAPTM4A, CD1B
- SPRY4, TLR8, DGCR14, MRCL3, SMAD2, LAPTM4A, FAM111A
- SPRY4, TLR8, DGCR14, MRCL3, SMAD2, SFRS12, CD1B
- SPRY4, TLR8, DGCR14, MRCL3, SMAD2, SFRS12, FAM111A
- SPRY4, TLR8, DGCR14, MRCL3, SMAD2, CD1B, FAM111A
- SPRY4, TLR8, DGCR14, MRCL3, LAPTM4A, SFRS12, CD1B
- SPRY4, TLR8, DGCR14, MRCL3, LAPTM4A, SFRS12, FAM111A
- SPRY4, TLR8, DGCR14, MRCL3, LAPTM4A, CD1B, FAM111A
- SPRY4, TLR8, DGCR14, MRCL3, SFRS12, CD1B, FAM111A
- SPRY4, TLR8, DGCR14, SMAD2, LAPTM4A, SFRS12, CD1B
- SPRY4, TLR8, DGCR14, SMAD2, LAPTM4A, SFRS12, FAM111A
- SPRY4, TLR8, DGCR14, SMAD2, LAPTM4A, CD1B, FAM111A
- SPRY4, TLR8, DGCR14, SMAD2, SFRS12, CD1B, FAM111A
- SPRY4, TLR8, DGCR14, LAPTM4A, SFRS12, CD1B, FAM111A
- SPRY4, TLR8, MRCL3, SMAD2, LAPTM4A, SFRS12, CD1B
- SPRY4, TLR8, MRCL3, SMAD2, LAPTM4A, SFRS12, FAM111A
- SPRY4, TLR8, MRCL3, SMAD2, LAPTM4A, CD1B, FAM111A
- SPRY4, TLR8, MRCL3, SMAD2, SFRS12, CD1B, FAM111A
- SPRY4, TLR8, MRCL3, LAPTM4A, SFRS12, CD1B, FAM111A
- SPRY4, TLR8, SMAD2, LAPTM4A, SFRS12, CD1B, FAM111A
- SPRY4, SAMSN1, DGCR14, MRCL3, SMAD2, LAPTM4A, SFRS12
- SPRY4, SAMSN1, DGCR14, MRCL3, SMAD2, LAPTM4A, CD1B
- SPRY4, SAMSN1, DGCR14, MRCL3, SMAD2, LAPTM4A, FAM111A
- SPRY4, SAMSN1, DGCR14, MRCL3, SMAD2, SFRS12, CD1B
- SPRY4, SAMSN1, DGCR14, MRCL3, SMAD2, SFRS12, FAM111A
- SPRY4, SAMSN1, DGCR14, MRCL3, SMAD2, CD1B, FAM111A
- SPRY4, SAMSN1, DGCR14, MRCL3, LAPTM4A, SFRS12, CD1B
- SPRY4, SAMSN1, DGCR14, MRCL3, LAPTM4A, SFRS12, FAM111A
- SPRY4, SAMSN1, DGCR14, MRCL3, LAPTM4A, CD1B, FAM111A
- SPRY4, SAMSN1, DGCR14, MRCL3, SFRS12, CD1B, FAM111A
- SPRY4, SAMSN1, DGCR14, SMAD2, LAPTM4A, SFRS12, CD1B
- SPRY4, SAMSN1, DGCR14, SMAD2, LAPTM4A, SFRS12, FAM111A
- SPRY4, SAMSN1, DGCR14, SMAD2, LAPTM4A, CD1B, FAM111A
- SPRY4, SAMSN1, DGCR14, SMAD2, SFRS12, CD1B, FAM111A
- SPRY4, SAMSN1, DGCR14, LAPTM4A, SFRS12, CD1B, FAM111A
- SPRY4, SAMSN1, MRCL3, SMAD2, LAPTM4A, SFRS12, CD1B
- SPRY4, SAMSN1, MRCL3, SMAD2, LAPTM4A, SFRS12, FAM111A
- SPRY4, SAMSN1, MRCL3, SMAD2, LAPTM4A, CD1B, FAM111A
- SPRY4, SAMSN1, MRCL3, SMAD2, SFRS12, CD1B, FAM111A
- SPRY4, SAMSN1, MRCL3, LAPTM4A, SFRS12, CD1B, FAM111A
- SPRY4, SAMSN1, SMAD2, LAPTM4A, SFRS12, CD1B, FAM111A
- SPRY4, DGCR14, MRCL3, SMAD2, LAPTM4A, SFRS12, CD1B
- SPRY4, DGCR14, MRCL3, SMAD2, LAPTM4A, SFRS12, FAM111A
- SPRY4, DGCR14, MRCL3, SMAD2, LAPTM4A, CD1B, FAM111A
- SPRY4, DGCR14, MRCL3, SMAD2, SFRS12, CD1B, FAM111A
- SPRY4, DGCR14, MRCL3, LAPTM4A, SFRS12, CD1B, FAM111A
- SPRY4, DGCR14, SMAD2, LAPTM4A, SFRS12, CD1B, FAM111A
- SPRY4, MRCL3, SMAD2, LAPTM4A, SFRS12, CD1B, FAM111A
- TLR8, SAMSN1, DGCR14, MRCL3, SMAD2, LAPTM4A, SFRS12
- TLR8, SAMSN1, DGCR14, MRCL3, SMAD2, LAPTM4A, CD1B
- TLR8, SAMSN1, DGCR14, MRCL3, SMAD2, LAPTM4A, FAM111A
- TLR8, SAMSN1, DGCR14, MRCL3, SMAD2, SFRS12, CD1B
- TLR8, SAMSN1, DGCR14, MRCL3, SMAD2, SFRS12, FAM111A

- TLR8, SAMSN1, DGCR14, MRCL3, SMAD2, CD1B, FAM111A
- TLR8, SAMSN1, DGCR14, MRCL3, LAPTM4A, SFRS12, CD1B
- TLR8, SAMSN1, DGCR14, MRCL3, LAPTM4A, SFRS12, FAM111A
- TLR8, SAMSN1, DGCR14, MRCL3, LAPTM4A, CD1B, FAM111A
- TLR8, SAMSN1, DGCR14, MRCL3, SFRS12, CD1B, FAM111A
- TLR8, SAMSN1, DGCR14, SMAD2, LAPTM4A, SFRS12, CD1B
- TLR8, SAMSN1, DGCR14, SMAD2, LAPTM4A, SFRS12, FAM111A
- TLR8, SAMSN1, DGCR14, SMAD2, LAPTM4A, CD1B, FAM111A
- TLR8, SAMSN1, DGCR14, SMAD2, SFRS12, CD1B, FAM111A
- TLR8, SAMSN1, DGCR14, LAPTM4A, SFRS12, CD1B, FAM111A
- TLR8, SAMSN1, MRCL3, SMAD2, LAPTM4A, SFRS12, CD1B
- TLR8, SAMSN1, MRCL3, SMAD2, LAPTM4A, SFRS12, FAM111A
- TLR8, SAMSN1, MRCL3, SMAD2, LAPTM4A, CD1B, FAM111A
- TLR8, SAMSN1, MRCL3, SMAD2, SFRS12, CD1B, FAM111A
- TLR8, SAMSN1, MRCL3, LAPTM4A, SFRS12, CD1B, FAM111A
- TLR8, SAMSN1, SMAD2, LAPTM4A, SFRS12, CD1B, FAM111A
- TLR8, DGCR14, MRCL3, SMAD2, LAPTM4A, SFRS12, CD1B
- TLR8, DGCR14, MRCL3, SMAD2, LAPTM4A, SFRS12, FAM111A
- TLR8, DGCR14, MRCL3, SMAD2, LAPTM4A, CD1B, FAM111A
- TLR8, DGCR14, MRCL3, SMAD2, SFRS12, CD1B, FAM111A
- TLR8, DGCR14, MRCL3, LAPTM4A, SFRS12, CD1B, FAM111A
- TLR8, DGCR14, SMAD2, LAPTM4A, SFRS12, CD1B, FAM111A
- TLR8, MRCL3, SMAD2, LAPTM4A, SFRS12, CD1B, FAM111A
- SAMSN1, DGCR14, MRCL3, SMAD2, LAPTM4A, SFRS12, CD1B
- SAMSN1, DGCR14, MRCL3, SMAD2, LAPTM4A, SFRS12, FAM111A
- SAMSN1, DGCR14, MRCL3, SMAD2, LAPTM4A, CD1B, FAM111A
- SAMSN1, DGCR14, MRCL3, SMAD2, SFRS12, CD1B, FAM111A
- SAMSN1, DGCR14, MRCL3, LAPTM4A, SFRS12, CD1B, FAM111A
- SAMSN1, DGCR14, SMAD2, LAPTM4A, SFRS12, CD1B, FAM111A
- SAMSN1, MRCL3, SMAD2, LAPTM4A, SFRS12, CD1B, FAM111A
- DGCR14, MRCL3, SMAD2, LAPTM4A, SFRS12, CD1B, FAM111A

Thus, combinations of 8 genes suitable for the differentiation of the stage of the tumor include

- MEA1, SPRY4, TLR8, SAMSN1, DGCR14, MRCL3, SMAD2, LAPTM4A
- MEA1, SPRY4, TLR8, SAMSN1, DGCR14, MRCL3, SMAD2, SFRS12
- MEA1, SPRY4, TLR8, SAMSN1, DGCR14, MRCL3, SMAD2, CD1B
- MEA1, SPRY4, TLR8, SAMSN1, DGCR14, MRCL3, SMAD2, FAM111A
- MEA1, SPRY4, TLR8, SAMSN1, DGCR14, MRCL3, LAPTM4A, SFRS12
- MEA1, SPRY4, TLR8, SAMSN1, DGCR14, MRCL3, LAPTM4A, CD1B
- MEA1, SPRY4, TLR8, SAMSN1, DGCR14, MRCL3, LAPTM4A, FAM111A
- MEA1, SPRY4, TLR8, SAMSN1, DGCR14, MRCL3, SFRS12, CD1B
- MEA1, SPRY4, TLR8, SAMSN1, DGCR14, MRCL3, SFRS12, FAM111A
- MEA1, SPRY4, TLR8, SAMSN1, DGCR14, MRCL3, CD1B, FAM111A
- MEA1, SPRY4, TLR8, SAMSN1, DGCR14, SMAD2, LAPTM4A, SFRS12
- MEA1, SPRY4, TLR8, SAMSN1, DGCR14, SMAD2, LAPTM4A, CD1B
- MEA1, SPRY4, TLR8, SAMSN1, DGCR14, SMAD2, LAPTM4A, FAM111A
- MEA1, SPRY4, TLR8, SAMSN1, DGCR14, SMAD2, SFRS12, CD1B
- MEA1, SPRY4, TLR8, SAMSN1, DGCR14, SMAD2, SFRS12, FAM111A
- MEA1, SPRY4, TLR8, SAMSN1, DGCR14, SMAD2, CD1B, FAM111A
- MEA1, SPRY4, TLR8, SAMSN1, DGCR14, LAPTM4A, SFRS12, CD1B
- MEA1, SPRY4, TLR8, SAMSN1, DGCR14, LAPTM4A, SFRS12, FAM111A
- MEA1, SPRY4, TLR8, SAMSN1, DGCR14, LAPTM4A, CD1B, FAM111A
- MEA1, SPRY4, TLR8, SAMSN1, DGCR14, SFRS12, CD1B, FAM111A
- MEA1, SPRY4, TLR8, SAMSN1, MRCL3, SMAD2, LAPTM4A, SFRS12
- MEA1, SPRY4, TLR8, SAMSN1, MRCL3, SMAD2, LAPTM4A, CD1B
- MEA1, SPRY4, TLR8, SAMSN1, MRCL3, SMAD2, LAPTM4A, FAM111A
- MEA1, SPRY4, TLR8, SAMSN1, MRCL3, SMAD2, SFRS12, CD1B

- MEA1, SPRY4, TLR8, SAMSN1, MRCL3, SMAD2, SFRS12, FAM111A
- MEA1, SPRY4, TLR8, SAMSN1, MRCL3, SMAD2, CD1B, FAM111A
- MEA1, SPRY4, TLR8, SAMSN1, MRCL3, LAPTM4A, SFRS12, CD1B
- MEA1, SPRY4, TLR8, SAMSN1, MRCL3, LAPTM4A, SFRS12, FAM111A
- MEA1, SPRY4, TLR8, SAMSN1, MRCL3, LAPTM4A, CD1B, FAM111A
- MEA1, SPRY4, TLR8, SAMSN1, MRCL3, SFRS12, CD1B, FAM111A
- MEA1, SPRY4, TLR8, SAMSN1, SMAD2, LAPTM4A, SFRS12, CD1B
- MEA1, SPRY4, TLR8, SAMSN1, SMAD2, LAPTM4A, SFRS12, FAM111A
- MEA1, SPRY4, TLR8, SAMSN1, SMAD2, LAPTM4A, CD1B, FAM111A
- MEA1, SPRY4, TLR8, SAMSN1, SMAD2, SFRS12, CD1B, FAM111A
- MEA1, SPRY4, TLR8, SAMSN1, LAPTM4A, SFRS12, CD1B, FAM111A
- MEA1, SPRY4, TLR8, DGCR14, MRCL3, SMAD2, LAPTM4A, SFRS12
- MEA1, SPRY4, TLR8, DGCR14, MRCL3, SMAD2, LAPTM4A, CD1B
- MEA1, SPRY4, TLR8, DGCR14, MRCL3, SMAD2, LAPTM4A, FAM111A
- MEA1, SPRY4, TLR8, DGCR14, MRCL3, SMAD2, SFRS12, CD1B
- MEA1, SPRY4, TLR8, DGCR14, MRCL3, SMAD2, SFRS12, FAM111A
- MEA1, SPRY4, TLR8, DGCR14, MRCL3, SMAD2, CD1B, FAM111A
- MEA1, SPRY4, TLR8, DGCR14, MRCL3, LAPTM4A, SFRS12, CD1B
- MEA1, SPRY4, TLR8, DGCR14, MRCL3, LAPTM4A, SFRS12, FAM111A
- MEA1, SPRY4, TLR8, DGCR14, MRCL3, LAPTM4A, CD1B, FAM111A
- MEA1, SPRY4, TLR8, DGCR14, MRCL3, SFRS12, CD1B, FAM111A
- MEA1, SPRY4, TLR8, DGCR14, SMAD2, LAPTM4A, SFRS12, CD1B
- MEA1, SPRY4, TLR8, DGCR14, SMAD2, LAPTM4A, SFRS12, FAM111A
- MEA1, SPRY4, TLR8, DGCR14, SMAD2, LAPTM4A, CD1B, FAM111A
- MEA1, SPRY4, TLR8, DGCR14, SMAD2, SFRS12, CD1B, FAM111A
- MEA1, SPRY4, TLR8, DGCR14, LAPTM4A, SFRS12, CD1B, FAM111A
- MEA1, SPRY4, TLR8, MRCL3, SMAD2, LAPTM4A, SFRS12, CD1B
- MEA1, SPRY4, TLR8, MRCL3, SMAD2, LAPTM4A, SFRS12, FAM111A
- MEA1, SPRY4, TLR8, MRCL3, SMAD2, LAPTM4A, CD1B, FAM111A
- MEA1, SPRY4, TLR8, MRCL3, SMAD2, SFRS12, CD1B, FAM111A
- MEA1, SPRY4, TLR8, MRCL3, LAPTM4A, SFRS12, CD1B, FAM111A
- MEA1, SPRY4, TLR8, SMAD2, LAPTM4A, SFRS12, CD1B, FAM111A
- MEA1, SPRY4, SAMSN1, DGCR14, MRCL3, SMAD2, LAPTM4A, SFRS12
- MEA1, SPRY4, SAMSN1, DGCR14, MRCL3, SMAD2, LAPTM4A, CD1B
- MEA1, SPRY4, SAMSN1, DGCR14, MRCL3, SMAD2, LAPTM4A, FAM111A
- MEA1, SPRY4, SAMSN1, DGCR14, MRCL3, SMAD2, SFRS12, CD1B
- MEA1, SPRY4, SAMSN1, DGCR14, MRCL3, SMAD2, SFRS12, FAM111A
- MEA1, SPRY4, SAMSN1, DGCR14, MRCL3, SMAD2, CD1B, FAM111A
- MEA1, SPRY4, SAMSN1, DGCR14, MRCL3, LAPTM4A, SFRS12, CD1B
- MEA1, SPRY4, SAMSN1, DGCR14, MRCL3, LAPTM4A, SFRS12, FAM111A
- MEA1, SPRY4, SAMSN1, DGCR14, MRCL3, LAPTM4A, CD1B, FAM111A
- MEA1, SPRY4, SAMSN1, DGCR14, MRCL3, SFRS12, CD1B, FAM111A
- MEA1, SPRY4, SAMSN1, DGCR14, SMAD2, LAPTM4A, SFRS12, CD1B
- MEA1, SPRY4, SAMSN1, DGCR14, SMAD2, LAPTM4A, SFRS12, FAM111A
- MEA1, SPRY4, SAMSN1, DGCR14, SMAD2, LAPTM4A, CD1B, FAM111A
- MEA1, SPRY4, SAMSN1, DGCR14, SMAD2, SFRS12, CD1B, FAM111A
- MEA1, SPRY4, SAMSN1, DGCR14, LAPTM4A, SFRS12, CD1B, FAM111A
- MEA1, SPRY4, SAMSN1, MRCL3, SMAD2, LAPTM4A, SFRS12, CD1B
- MEA1, SPRY4, SAMSN1, MRCL3, SMAD2, LAPTM4A, SFRS12, FAM111A
- MEA1, SPRY4, SAMSN1, MRCL3, SMAD2, LAPTM4A, CD1B, FAM111A
- MEA1, SPRY4, SAMSN1, MRCL3, SMAD2, SFRS12, CD1B, FAM111A
- MEA1, SPRY4, SAMSN1, MRCL3, LAPTM4A, SFRS12, CD1B, FAM111A
- MEA1, SPRY4, SAMSN1, SMAD2, LAPTM4A, SFRS12, CD1B, FAM111A
- MEA1, SPRY4, DGCR14, MRCL3, SMAD2, LAPTM4A, SFRS12, CD1B
- MEA1, SPRY4, DGCR14, MRCL3, SMAD2, LAPTM4A, SFRS12, FAM111A
- MEA1, SPRY4, DGCR14, MRCL3, SMAD2, LAPTM4A, CD1B, FAM111A
- MEA1, SPRY4, DGCR14, MRCL3, SMAD2, SFRS12, CD1B, FAM111A
- MEA1, SPRY4, DGCR14, MRCL3, LAPTM4A, SFRS12, CD1B, FAM111A

- MEA1, SPRY4, DGCR14, SMAD2, LAPTM4A, SFRS12, CD1B, FAM111A
- MEA1, SPRY4, MRCL3, SMAD2, LAPTM4A, SFRS12, CD1B, FAM111A
- MEA1, TLR8, SAMSN1, DGCR14, MRCL3, SMAD2, LAPTM4A, SFRS12
- MEA1, TLR8, SAMSN1, DGCR14, MRCL3, SMAD2, LAPTM4A, CD1B
- MEA1, TLR8, SAMSN1, DGCR14, MRCL3, SMAD2, LAPTM4A, FAM111A
- MEA1, TLR8, SAMSN1, DGCR14, MRCL3, SMAD2, SFRS12, CD1B
- MEA1, TLR8, SAMSN1, DGCR14, MRCL3, SMAD2, SFRS12, FAM111A
- MEA1, TLR8, SAMSN1, DGCR14, MRCL3, SMAD2, CD1B, FAM111A
- MEA1, TLR8, SAMSN1, DGCR14, MRCL3, LAPTM4A, SFRS12, CD1B
- MEA1, TLR8, SAMSN1, DGCR14, MRCL3, LAPTM4A, SFRS12, FAM111A
- MEA1, TLR8, SAMSN1, DGCR14, MRCL3, LAPTM4A, CD1B, FAM111A
- MEA1, TLR8, SAMSN1, DGCR14, MRCL3, SFRS12, CD1B, FAM111A
- MEA1, TLR8, SAMSN1, DGCR14, SMAD2, LAPTM4A, SFRS12, CD1B
- MEA1, TLR8, SAMSN1, DGCR14, SMAD2, LAPTM4A, SFRS12, FAM111A
- MEA1, TLR8, SAMSN1, DGCR14, SMAD2, LAPTM4A, CD1B, FAM111A
- MEA1, TLR8, SAMSN1, DGCR14, SMAD2, SFRS12, CD1B, FAM111A
- MEA1, TLR8, SAMSN1, DGCR14, LAPTM4A, SFRS12, CD1B, FAM111A
- MEA1, TLR8, SAMSN1, MRCL3, SMAD2, LAPTM4A, SFRS12, CD1B
- MEA1, TLR8, SAMSN1, MRCL3, SMAD2, LAPTM4A, SFRS12, FAM111A
- MEA1, TLR8, SAMSN1, MRCL3, SMAD2, LAPTM4A, CD1B, FAM111A
- MEA1, TLR8, SAMSN1, MRCL3, SMAD2, SFRS12, CD1B, FAM111A
- MEA1, TLR8, SAMSN1, MRCL3, LAPTM4A, SFRS12, CD1B, FAM111A
- MEA1, TLR8, SAMSN1, SMAD2, LAPTM4A, SFRS12, CD1B, FAM111A
- MEA1, TLR8, DGCR14, MRCL3, SMAD2, LAPTM4A, SFRS12, CD1B
- MEA1, TLR8, DGCR14, MRCL3, SMAD2, LAPTM4A, SFRS12, FAM111A
- MEA1, TLR8, DGCR14, MRCL3, SMAD2, LAPTM4A, CD1B, FAM111A
- MEA1, TLR8, DGCR14, MRCL3, SMAD2, SFRS12, CD1B, FAM111A
- MEA1, TLR8, DGCR14, MRCL3, LAPTM4A, SFRS12, CD1B, FAM111A
- MEA1, TLR8, DGCR14, SMAD2, LAPTM4A, SFRS12, CD1B, FAM111A
- MEA1, TLR8, MRCL3, SMAD2, LAPTM4A, SFRS12, CD1B, FAM111A
- MEA1, SAMSN1, DGCR14, MRCL3, SMAD2, LAPTM4A, SFRS12, CD1B
- MEA1, SAMSN1, DGCR14, MRCL3, SMAD2, LAPTM4A, SFRS12, FAM111A
- MEA1, SAMSN1, DGCR14, MRCL3, SMAD2, LAPTM4A, CD1B, FAM111A
- MEA1, SAMSN1, DGCR14, MRCL3, SMAD2, SFRS12, CD1B, FAM111A
- MEA1, SAMSN1, DGCR14, MRCL3, LAPTM4A, SFRS12, CD1B, FAM111A
- MEA1, SAMSN1, DGCR14, SMAD2, LAPTM4A, SFRS12, CD1B, FAM111A
- MEA1, SAMSN1, MRCL3, SMAD2, LAPTM4A, SFRS12, CD1B, FAM111A
- MEA1, DGCR14, MRCL3, SMAD2, LAPTM4A, SFRS12, CD1B, FAM111A
- SPRY4, TLR8, SAMSN1, DGCR14, MRCL3, SMAD2, LAPTM4A, SFRS12
- SPRY4, TLR8, SAMSN1, DGCR14, MRCL3, SMAD2, LAPTM4A, CD1B
- SPRY4, TLR8, SAMSN1, DGCR14, MRCL3, SMAD2, LAPTM4A, FAM111A
- SPRY4, TLR8, SAMSN1, DGCR14, MRCL3, SMAD2, SFRS12, CD1B
- SPRY4, TLR8, SAMSN1, DGCR14, MRCL3, SMAD2, SFRS12, FAM111A
- SPRY4, TLR8, SAMSN1, DGCR14, MRCL3, SMAD2, CD1B, FAM111A
- SPRY4, TLR8, SAMSN1, DGCR14, MRCL3, LAPTM4A, SFRS12, CD1B
- SPRY4, TLR8, SAMSN1, DGCR14, MRCL3, LAPTM4A, SFRS12, FAM111A
- SPRY4, TLR8, SAMSN1, DGCR14, MRCL3, LAPTM4A, CD1B, FAM111A
- SPRY4, TLR8, SAMSN1, DGCR14, MRCL3, SFRS12, CD1B, FAM111A
- SPRY4, TLR8, SAMSN1, DGCR14, SMAD2, LAPTM4A, SFRS12, CD1B
- SPRY4, TLR8, SAMSN1, DGCR14, SMAD2, LAPTM4A, SFRS12, FAM111A
- SPRY4, TLR8, SAMSN1, DGCR14, SMAD2, LAPTM4A, CD1B, FAM111A
- SPRY4, TLR8, SAMSN1, DGCR14, SMAD2, SFRS12, CD1B, FAM111A
- SPRY4, TLR8, SAMSN1, DGCR14, LAPTM4A, SFRS12, CD1B, FAM111A
- SPRY4, TLR8, SAMSN1, MRCL3, SMAD2, LAPTM4A, SFRS12, CD1B
- SPRY4, TLR8, SAMSN1, MRCL3, SMAD2, LAPTM4A, SFRS12, FAM111A
- SPRY4, TLR8, SAMSN1, MRCL3, SMAD2, LAPTM4A, CD1B, FAM111A
- SPRY4, TLR8, SAMSN1, MRCL3, SMAD2, SFRS12, CD1B, FAM111A
- SPRY4, TLR8, SAMSN1, MRCL3, LAPTM4A, SFRS12, CD1B, FAM111A

- SPRY4, TLR8, SAMSN1, SMAD2, LAPTM4A, SFRS12, CD1B, FAM111A
- SPRY4, TLR8, DGCR14, MRCL3, SMAD2, LAPTM4A, SFRS12, CD1B
- SPRY4, TLR8, DGCR14, MRCL3, SMAD2, LAPTM4A, SFRS12, FAM111A
- SPRY4, TLR8, DGCR14, MRCL3, SMAD2, LAPTM4A, CD1B, FAM111A
- SPRY4, TLR8, DGCR14, MRCL3, SMAD2, SFRS12, CD1B, FAM111A
- SPRY4, TLR8, DGCR14, MRCL3, LAPTM4A, SFRS12, CD1B, FAM111A
- SPRY4, TLR8, DGCR14, SMAD2, LAPTM4A, SFRS12, CD1B, FAM111A
- SPRY4, TLR8, MRCL3, SMAD2, LAPTM4A, SFRS12, CD1B, FAM111A
- SPRY4, SAMSN1, DGCR14, MRCL3, SMAD2, LAPTM4A, SFRS12, CD1B
- SPRY4, SAMSN1, DGCR14, MRCL3, SMAD2, LAPTM4A, SFRS12, FAM111A
- SPRY4, SAMSN1, DGCR14, MRCL3, SMAD2, LAPTM4A, CD1B, FAM111A
- SPRY4, SAMSN1, DGCR14, MRCL3, SMAD2, SFRS12, CD1B, FAM111A
- SPRY4, SAMSN1, DGCR14, MRCL3, LAPTM4A, SFRS12, CD1B, FAM111A
- SPRY4, SAMSN1, DGCR14, SMAD2, LAPTM4A, SFRS12, CD1B, FAM111A
- SPRY4, SAMSN1, MRCL3, SMAD2, LAPTM4A, SFRS12, CD1B, FAM111A
- SPRY4, DGCR14, MRCL3, SMAD2, LAPTM4A, SFRS12, CD1B, FAM111A
- TLR8, SAMSN1, DGCR14, MRCL3, SMAD2, LAPTM4A, SFRS12, CD1B
- TLR8, SAMSN1, DGCR14, MRCL3, SMAD2, LAPTM4A, SFRS12, FAM111A
- TLR8, SAMSN1, DGCR14, MRCL3, SMAD2, LAPTM4A, CD1B, FAM111A
- TLR8, SAMSN1, DGCR14, MRCL3, SMAD2, SFRS12, CD1B, FAM111A
- TLR8, SAMSN1, DGCR14, MRCL3, LAPTM4A, SFRS12, CD1B, FAM111A
- TLR8, SAMSN1, DGCR14, SMAD2, LAPTM4A, SFRS12, CD1B, FAM111A
- TLR8, SAMSN1, MRCL3, SMAD2, LAPTM4A, SFRS12, CD1B, FAM111A
- TLR8, DGCR14, MRCL3, SMAD2, LAPTM4A, SFRS12, CD1B, FAM111A
- SAMSN1, DGCR14, MRCL3, SMAD2, LAPTM4A, SFRS12, CD1B, FAM111A

Thus, combinations of 9 genes suitable for the differentiation of the stage of the tumor include

- MEA1, SPRY4, TLR8, SAMSN1, DGCR14, MRCL3, SMAD2, LAPTM4A, SFRS12
- MEA1, SPRY4, TLR8, SAMSN1, DGCR14, MRCL3, SMAD2, LAPTM4A, CD1B
- MEA1, SPRY4, TLR8, SAMSN1, DGCR14, MRCL3, SMAD2, LAPTM4A, FAM111A
- MEA1, SPRY4, TLR8, SAMSN1, DGCR14, MRCL3, SMAD2, SFRS12, CD1B
- MEA1, SPRY4, TLR8, SAMSN1, DGCR14, MRCL3, SMAD2, SFRS12, FAM111A
- MEA1, SPRY4, TLR8, SAMSN1, DGCR14, MRCL3, SMAD2, CD1B, FAM111A
- MEA1, SPRY4, TLR8, SAMSN1, DGCR14, MRCL3, LAPTM4A, SFRS12, CD1B
- MEA1, SPRY4, TLR8, SAMSN1, DGCR14, MRCL3, LAPTM4A, SFRS12, FAM111A
- MEA1, SPRY4, TLR8, SAMSN1, DGCR14, MRCL3, LAPTM4A, CD1B, FAM111A
- MEA1, SPRY4, TLR8, SAMSN1, DGCR14, MRCL3, SFRS12, CD1B, FAM111A
- MEA1, SPRY4, TLR8, SAMSN1, DGCR14, SMAD2, LAPTM4A, SFRS12, CD1B
- MEA1, SPRY4, TLR8, SAMSN1, DGCR14, SMAD2, LAPTM4A, SFRS12, FAM111A
- MEA1, SPRY4, TLR8, SAMSN1, DGCR14, SMAD2, LAPTM4A, CD1B, FAM111A
- MEA1, SPRY4, TLR8, SAMSN1, DGCR14, SMAD2, SFRS12, CD1B, FAM111A
- MEA1, SPRY4, TLR8, SAMSN1, DGCR14, LAPTM4A, SFRS12, CD1B, FAM111A
- MEA1, SPRY4, TLR8, SAMSN1, MRCL3, SMAD2, LAPTM4A, SFRS12, CD1B
- MEA1, SPRY4, TLR8, SAMSN1, MRCL3, SMAD2, LAPTM4A, SFRS12, FAM111A
- MEA1, SPRY4, TLR8, SAMSN1, MRCL3, SMAD2, LAPTM4A, CD1B, FAM111A
- MEA1, SPRY4, TLR8, SAMSN1, MRCL3, SMAD2, SFRS12, CD1B, FAM111A
- MEA1, SPRY4, TLR8, SAMSN1, MRCL3, LAPTM4A, SFRS12, CD1B, FAM111A
- MEA1, SPRY4, TLR8, SAMSN1, SMAD2, LAPTM4A, SFRS12, CD1B, FAM111A
- MEA1, SPRY4, TLR8, DGCR14, MRCL3, SMAD2, LAPTM4A, SFRS12, CD1B
- MEA1, SPRY4, TLR8, DGCR14, MRCL3, SMAD2, LAPTM4A, SFRS12, FAM111A
- MEA1, SPRY4, TLR8, DGCR14, MRCL3, SMAD2, LAPTM4A, CD1B, FAM111A
- MEA1, SPRY4, TLR8, DGCR14, MRCL3, SMAD2, SFRS12, CD1B, FAM111A
- MEA1, SPRY4, TLR8, DGCR14, MRCL3, LAPTM4A, SFRS12, CD1B, FAM111A
- MEA1, SPRY4, TLR8, DGCR14, SMAD2, LAPTM4A, SFRS12, CD1B, FAM111A
- MEA1, SPRY4, TLR8, MRCL3, SMAD2, LAPTM4A, SFRS12, CD1B, FAM111A
- MEA1, SPRY4, SAMSN1, DGCR14, MRCL3, SMAD2, LAPTM4A, SFRS12, CD1B
- MEA1, SPRY4, SAMSN1, DGCR14, MRCL3, SMAD2, LAPTM4A, SFRS12, FAM111A

- MEA1, SPRY4, SAMSN1, DGCR14, MRCL3, SMAD2, LAPTM4A, CD1B, FAM111A
- MEA1, SPRY4, SAMSN1, DGCR14, MRCL3, SMAD2, SFRS12, CD1B, FAM111A
- MEA1, SPRY4, SAMSN1, DGCR14, MRCL3, LAPTM4A, SFRS12, CD1B, FAM111A
- MEA1, SPRY4, SAMSN1, DGCR14, SMAD2, LAPTM4A, SFRS12, CD1B, FAM111A
- MEA1, SPRY4, SAMSN1, MRCL3, SMAD2, LAPTM4A, SFRS12, CD1B, FAM111A
- MEA1, SPRY4, DGCR14, MRCL3, SMAD2, LAPTM4A, SFRS12, CD1B, FAM111A
- MEA1, TLR8, SAMSN1, DGCR14, MRCL3, SMAD2, LAPTM4A, SFRS12, CD1B
- MEA1, TLR8, SAMSN1, DGCR14, MRCL3, SMAD2, LAPTM4A, SFRS12, FAM111A
- MEA1, TLR8, SAMSN1, DGCR14, MRCL3, SMAD2, LAPTM4A, CD1B, FAM111A
- MEA1, TLR8, SAMSN1, DGCR14, MRCL3, SMAD2, SFRS12, CD1B, FAM111A
- MEA1, TLR8, SAMSN1, DGCR14, MRCL3, LAPTM4A, SFRS12, CD1B, FAM111A
- MEA1, TLR8, SAMSN1, DGCR14, SMAD2, LAPTM4A, SFRS12, CD1B, FAM111A
- MEA1, TLR8, SAMSN1, MRCL3, SMAD2, LAPTM4A, SFRS12, CD1B, FAM111A
- MEA1, TLR8, DGCR14, MRCL3, SMAD2, LAPTM4A, SFRS12, CD1B, FAM111A
- MEA1, SAMSN1, DGCR14, MRCL3, SMAD2, LAPTM4A, SFRS12, CD1B, FAM111A
- SPRY4, TLR8, SAMSN1, DGCR14, MRCL3, SMAD2, LAPTM4A, SFRS12, CD1B
- SPRY4, TLR8, SAMSN1, DGCR14, MRCL3, SMAD2, LAPTM4A, SFRS12, FAM111A
- SPRY4, TLR8, SAMSN1, DGCR14, MRCL3, SMAD2, LAPTM4A, CD1B, FAM111A
- SPRY4, TLR8, SAMSN1, DGCR14, MRCL3, SMAD2, SFRS12, CD1B, FAM111A
- SPRY4, TLR8, SAMSN1, DGCR14, MRCL3, LAPTM4A, SFRS12, CD1B, FAM111A
- SPRY4, TLR8, SAMSN1, DGCR14, SMAD2, LAPTM4A, SFRS12, CD1B, FAM111A
- SPRY4, TLR8, SAMSN1, MRCL3, SMAD2, LAPTM4A, SFRS12, CD1B, FAM111A
- SPRY4, TLR8, DGCR14, MRCL3, SMAD2, LAPTM4A, SFRS12, CD1B, FAM111A
- SPRY4, SAMSN1, DGCR14, MRCL3, SMAD2, LAPTM4A, SFRS12, CD1B, FAM111A
- TLR8, SAMSN1, DGCR14, MRCL3, SMAD2, LAPTM4A, SFRS12, CD1B, FAM111A

Thus, combinations of 10 genes suitable for the differentiation of the stage of the tumor include

- MEA1, SPRY4, TLR8, SAMSN1, DGCR14, MRCL3, SMAD2, LAPTM4A, SFRS12, CD1B
- MEA1, SPRY4, TLR8, SAMSN1, DGCR14, MRCL3, SMAD2, LAPTM4A, SFRS12, FAM111A
- MEA1, SPRY4, TLR8, SAMSN1, DGCR14, MRCL3, SMAD2, LAPTM4A, CD1B, FAM111A
- MEA1, SPRY4, TLR8, SAMSN1, DGCR14, MRCL3, SMAD2, SFRS12, CD1B, FAM111A
- MEA1, SPRY4, TLR8, SAMSN1, DGCR14, MRCL3, LAPTM4A, SFRS12, CD1B, FAM111A
- MEA1, SPRY4, TLR8, SAMSN1, DGCR14, SMAD2, LAPTM4A, SFRS12, CD1B, FAM111A
- MEA1, SPRY4, TLR8, SAMSN1, MRCL3, SMAD2, LAPTM4A, SFRS12, CD1B, FAM111A
- MEA1, SPRY4, TLR8, DGCR14, MRCL3, SMAD2, LAPTM4A, SFRS12, CD1B, FAM111A
- MEA1, SPRY4, SAMSN1, DGCR14, MRCL3, SMAD2, LAPTM4A, SFRS12, CD1B, FAM111A
- MEA1, TLR8, SAMSN1, DGCR14, MRCL3, SMAD2, LAPTM4A, SFRS12, CD1B, FAM111A
- SPRY4, TLR8, SAMSN1, DGCR14, MRCL3, SMAD2, LAPTM4A, SFRS12, CD1B, FAM111A

Additional diagnosis and staging methods of the invention

The authors of the present invention have also clearly shown the possibility of combining the previously defined diagnosis method and staging methods in order to be able to carry out a more complete staging of a tumor as well as to be able to supply a more accurate diagnosis of the type of CRC. Thus, in another aspect the invention relates to a method for determining the stage of a colorectal tumor comprising

(i) determining if the tumor corresponds to stage B/C or to stage D by means of the first staging method of the invention and
(ii) in the event that the tumor is a stage B/C tumor, determining if the tumor corresponds to stage B or to stage C by means of the second staging method of the invention

In another aspect, the invention relates to a method for the diagnosis of colorectal cancer in a patient comprising

(i) determining if he or she has tumor tissue in a colorectal tissue sample by means of the diagnosis of the invention,
(ii) if tumor tissue is identified in step (i), determining if said tumor tissue corresponds to stage B/C by means of the first staging method of the invention and
(iii) if the tumor is identified as stage B/C in step (ii), determining if the tumor corresponds to stage B or to stage C by means of the second staging method of the invention

The methods for the determination of the expression levels of the different genes are essentially carried out as has been described for the first diagnosis method of the invention and for the staging methods of the invention.

Kit of the invention

In another aspect, the invention relates to a kit for the diagnosis of colorectal cancer or for the determination of the stage of a colorectal tumor comprising a first component and, optionally, a second component wherein the first component is a set of reagents consisting of

(i) reagents which allow determining the expression levels of genes TMEM132A, CMTM7, FAM60A, ENC1, ACAT1, MADCAM1 and DDX55,
(ii) reagents which allow determining the expression levels of genes HAS1, LO8961, NHLRC2, ATP6VOE2, RPS23, GTF2H2, FLJ34077, CASP12 and RP5-1077B9.4 or
(iii) reagents which allow determining the expression levels of genes MEA1, SPRY4, TLR8, SAMSN1, DGCR14, MRCL3, SMAD2, LAPTM4A, SFRS12, CD1B and FAM111A

and wherein the second component comprises reagents suitable for determining the expression levels of one or several reference genes.

In the context of the present invention, "kit" is understood as a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

"Reagent which allows determining the expression levels of a gene" means a compound or set of compounds that allows determining the expression levels of a gene both by means of the determination of the levels of mRNA and by means of the determination of the levels of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

In a preferred embodiment, the first component of the kit of the invention consists of a specific probe for each of genes TMEM132A, CMTM7, FAM60A, ENC1, ACAT1, MADCAM1 and DDX55, of a specific probe for genes HAS1, LO8961, NHLRC2, ATP6VOE2, RPS23, GTF2H2, FLJ34077, CASP12 and RP5-1077B9.4 or of a specific probe for genes MEA1, SPRY4, TLR8, SAMSN1, DGCR14, MRCL3, SMAD2, LAPTM4A, SFRS12, CD1B and FAM111A

In another preferred embodiment, the probes or antibodies forming the kit of the invention are coupled to an array.

In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular subsequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogs thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can be of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-strand or double-strand nucleic acids.

The selection of the specific probes for the different target genes is carried out such that they bind specifically to the target nucleic acid with a minimum hybridization to non-related genes. However, there are probes of 20 nucleotides which are not unique for a certain mRNA. Therefore, probes directed to said sequences will show a cross-hybridization with identical sequences that appear in mRNA of non-related genes. In addition, there are probes that do not specifically hybridize with the target genes in the conditions used (because of secondary structures or of interactions with the substrate of the array). This type of probe must not be included in the array. Therefore, the person skilled in the art will observe that the probes that are going to be incorporated in a certain array must be optimized before their incorporation to the array. The optimization of the probes is generally carried out by generating an array containing a plurality of probes directed to the different regions of a certain target polynucleotide. This array is put into contact firstly with a sample containing the target nucleic acid in an isolated form and, secondly, with a complex mixture of nucleic acids. Probes which show a highly specific hybridization with the target nucleic acid but low or no hybridization with the complex sample are thus selected for their incorporation to the arrays of the invention. Additionally, it is possible to include in the array hybridization controls for each of the probes that is going to be studied. In a preferred embodiment, the hybridization controls contain an altered position in the central region of the probe. In the event that high levels of hybridization are observed between the studied probe and its hybridization control, the probe is not included in the array.

In a preferred embodiment, the array contains a plurality of probes complementary to subsequences of the target nucleic acid of a constant length or of variable length in a range of 5 to 50 nucleotides. The array can contain all the specific probes of a certain mRNA of a certain length or can contain probes selected from different regions of an mRNA. Each probe is assayed in parallel with a probe with a changed base, preferably in a central position of the probe. The array is put into contact with a sample containing nucleic acids with sequences complementary to the probes of the array and the signal of hybridization with each of the probes and with the corresponding hybridization controls is determined. Those probes in which a higher difference is observed between the signal of hybridization with the probe and its hybridization control are selected. The optimization process can include a second round of optimization in which the hybridization array is hybridized with a sample that does not contain sequences complementary to the probes of the array. After the second round of selection, those probes having signals of hybridization lower than a threshold level will be selected. Thus, probes which pass both controls, i.e., which show a minimum level of unspecific hybridization and a maximum level of specific hybridization with the target nucleic acid are selected.

The microarrays of the invention contain not only specific probes for the polynucleotides indicating a determined pathophysiological situation, but also containing a series of control probes, which can be of three types: normalization controls, expression level controls and hybridization controls

Normalization controls are oligonucleotides that are perfectly complementary to labeled reference sequences which are added to the preparation of nucleic acids to be analyzed. The signals derived from the normalization controls after the hybridization provide an indication of the variations in the hybridization conditions, intensity of the marker, efficiency of the detection and another series of factors that can result in a variation of the signal of hybridization between different microarrays. The signals detected from the rest of probes of the array are preferably divided by the signal emitted by the control probes, thus normalizing the measurements. Virtually any probe can be used as normalization control. However, it is known that the efficiency of the hybridization varies according to the composition of nucleotides and the length of the probe. Therefore, preferred normalization probes are those which represent the mean length of the probes present in the array, although they can be selected such that they include a range of lengths that reflect the rest of probes present in the array. The normalization probes can be designed such that they reflect the mean composition of nucleotides of the rest of probes present in the array. A limited number of normalization probes is preferably selected such that they hybridize suitably, i.e., they do not have a secondary structure and do not show sequence similarity with any of the probes of the array is used. The normalization probes can be located in any position in the array or in multiple positions in the array to efficiently control variations in hybridization efficiency related to the structure of the array. The normalization controls are preferably located in the corners of the array and/or in the center thereof.

The expression controls levels are probes which hybridize specifically with genes which are expressed constitutively in the sample which is analyzed. The expression level controls are designed to control the physiological state and the metabolic activity of the cell. The examination of the covariance of the expression level control with the expression level of the target nucleic acid indicates if the variations in the expression levels are due to changes in the expression levels or are due to changes in the overall transcriptional rate in the cell or in its general metabolic activity. Thus, in the case of cells which have deficiencies in a certain metabolite essential for cell viability, the observation of a decrease both in the expression levels of the target gene as in the expression levels of the control is expected. On the other hand, if an increase in the expression of the expression of the target gene and of the control gene is observed, it probably due to an increase of the metabolic activity of the cell and not to a differential increase in the expression of the target gene. Any probe corresponding to a gene expressed constitutively, such as genes encoding proteins which exert essential cell functions such as β-2-microglobulin, ubiquitin, ribosomal protein 18S, cyclophilin A, transferrin receptor, actin, GAPDH and the like, can be used. In a preferred embodiment, the expression levels controls are GAPDH, tyrosine 3-monooxygenase/tryptophan 5-monooxygenase activation protein (YWHAZ), ubiquitin, beta-actin and β-2-microglobulin.

Hybridization controls can be included both for the probes directed to target genes and for the probes directed to the expression level or to the normalization controls. Error controls are probes of oligonucleotides identical to the probes directed to target genes but which contain mutations in one or several nucleotides, i.e., which contain nucleotides in certain positions which do not hybridize with the corresponding nucleotide in the target gene. The hybridization controls are selected such that, applying the suitable hybridization conditions, the target gene should hybridize with the specific probe but not with the hybridization control or with a reduced efficiency. The hybridization controls preferably contain one or several modified positions in the center of the probe. The hybridization controls therefore provide an indication of the degree of unspecific hybridization or of cross-hybridization to a nucleic acid in the sample to a probe different from that containing the exactly complementary sequence.

The arrays of the invention can also contain amplification and sample preparation controls which are probes complementary to subsequences of selected control genes because they normally do not appear in the biological sample object of the study, such as probes for bacterial genes. The RNA sample is supplemented with a known amount of a nucleic acid which hybridizes with the selected control probe. The determination of the hybridization to said probe indicates the degree of recovery of the nucleic acids during their preparation as well as an estimation of the alteration caused in the nucleic acids during the processing of the sample.

Once a set of probes showing the suitable specificity and a set of control probes are provided, the latter are arranged in the array in a known position such that, after the steps of hybridization and of detection, it is possible to establish a correlation between a positive signal of hybridization and the particular gene from the coordinates of the array in which the positive signal of hybridization is detected.

The microarrays can be high density arrays with thousands of oligonucleotides by means of photolithographic in situ synthesis methods (Fodor et al., 1991, Science, 767-773). This type of probe is usually redundant, i.e., they include several probes for each mRNA which is to be detected. In a preferred embodiment, the arrays are low density arrays or LDA containing less than 10000 probes per square centimeter. In said low density arrays, the different probes are manually applied with the aid of a pipette in different locations of a solid support (for example, a crystal surface, a membrane). The supports used to fix the probes can be obtained from a large variety of materials, including plastic, ceramics, metals, gels, membranes, crystals and the like. The microarrays can be obtained using any methodology known for the person skilled in the art.

After the hybridization, in the cases in which the non-hybridized nucleic acid is capable of emitting a signal in step of detection, a step of washing is necessary to eliminate said non-hybridized nucleic acid. The step of washing is carried out using methods and solutions known by the person skilled in the art.

In the event that the labeling in the nucleic acid is not directly detectable, it is possible to connect the microarray comprising the target nucleic acids bound to the array with the other components of the system necessary to cause the reaction giving rise to a detectable signal. For example, if the target nucleic acids are labeled with biotin, the array is put into contact with conjugated streptavidin with a fluorescent reagent in suitable conditions so that the binding between biotin and streptavidin occurs. After the incubation of the microarray with the system generating the detectable signal, it is necessary to carry out a step of washing to eliminate all the molecules which have bound non-specifically to the array. The washing conditions will be determined by the person skilled in the art using suitable conditions according to the system generating the detectable signal and which are well known for the person skilled in the art.

The resulting hybridization pattern can be viewed or detected in several different ways, said detection being determined by the type of system used in the microarray. Thus, the detection of the hybridization pattern can be carried out by means of scintillation counting, autoradiography, determination of a fluorescent signal, calorimetric determinations, detection of a light signal and the like.

Prior to the step of detection, it is possible to treat the microarrays with an specific endonuclease for single-strand DNA, such that the DNA that has bound non-specifically to the array is eliminated whereas the double-strand DNA resulting from the hybridization of the probes of the array with the nucleic acids of the sample object of study remains unchanged. Endonucleases suitable for this treatment include the S1 nuclease, mung bean nuclease and the like. In the event that the treatment with endonuclease is carried out in an assay in which the target nucleic acid is not labeled with a directly detectable molecule (for example, in an assay in which the target nucleic acid is biotinylated), the treatment with endonuclease will be carried out before putting the microarray into contact with the other members of the system producing the detectable signal.

After the hybridization and the possible subsequent washing and treatment processes, the hybridization pattern is detected and quantified, for which the signal corresponding to each point of hybridization in the array is compared to a reference value corresponding to the signal emitted by a known number of terminally labeled nucleic acids in order to thus obtain an absolute value of the number of copies of each nucleic acid which is hybridized in a certain point of the microarray.

In the event that the expression levels of several of the proteins identified in the present invention is to be simultaneously determined, compositions containing at least one specific antibody for each of the genes indicated in at least one table selected from the group of Table 1 to 4 are useful. For this purpose, the arrays of antibodies such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763; WO 01/40803 and WO 99/51773A1 are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like. The techniques for preparing said antibodies are very well known for the person skilled in the art and include the methods described by Ausubel et al. ((Current Protocols in Molecular Biology, eds. Ausubel et al, John Wiley & Sons (1992)).

The antibodies of the array can be applied at high speed, for example, using commercially available robotic systems (for example, those produced by Genetic Microsystems or Biorobotics). The substrate of the array can be nitrocellulose, plastic, crystal or can be of a porous material as for example, acrylamide, agarose or another polymer. In another embodiment, it is possible to use cells producing the specific antibodies for detecting the proteins of the invention by means of their culture in array filters. After the induction of the expression of the antibodies, the latter are immobilized in the filter in the position of the array where the producing cell was located.

An array of antibodies can be put into contact with a labeled target and the binding level of the target to the immobilized antibodies can be determined. If the target is not labeled, a sandwich type assay can be used in which a second labeled antibody specific for the polypeptide which binds to the polypeptide which is immobilized in the support is used. The quantification of the amount of polypeptide present in the sample in each point of the array can be stored in a database as an expression profile. The array of antibodies can be produced in duplicate and can be used to compare the binding profiles of two different samples.

In another aspect, the invention relates to the use of a kit of the invention for the diagnosis of colorectal cancer or for the determination of the stage of a colorectal tumor.

The invention is detailed below by means of the following examples which are merely illustrative and by no means limiting for the scope of the invention.

EXAMPLES

Human tissue samples

The Instituto Vasco de Investigaciones Sanitarias (O+Iker) through the Oncology Service of the Hospital de Cruces and in collaboration with Galdakao and San Eloy Hospitals collected, with informed consent, samples from healthy patients with respect to colon cancer, as well as from patients with colorectal cancer in some of its stages, from whom two samples were obtained, one from the tumor and the another non-tumor one obtained from a more distal portion.

A series of protocols for collecting, processing and storing both neoplastic and normal tissue samples were established for the purpose of preventing variability in the studies conducted. At the time of the surgery, the tumor sample was processed in Pathological Anatomy, where an identification of the stage of the tumor was carried out. The extracted sample was introduced in a stabilizing solution known as *RNAlater* (Qiagen) (10 ml of RNAlater per milligram of tissue). The samples were collected in carbon dioxide snow and stored at -80°C. The total tally was 120 paired samples from patients with colon cancer in different stages (Dukes' A, B, C and D) and 13 samples from healthy patients in relation to this pathology.

Extraction of total RNA

For the purpose of assuring the optimal handling conditions of the samples, as well as minimizing the risk of the presence of RNAses in the work environment, material that was previously sterilized and certified as RNAse-free was used. Thus, all the glass material used was subjected to 180°C for at least 8 hours. Additionally, prior to each step carried out, the work area was sterilized with *RNAse Away* (Invitrogen).

The handling of the tissue samples was carried out inside a biological hazard hood (TELSTAR Bio-II-A/P). Maintaining the cryovial in ice, a portion of the sample was extracted with the aid of tweezers and a scalpel previously washed with water treated with 0.1% diethyl pyrocarbonate (DEPC). The selected sample was put in a 2 ml tube and the remaining tissue was again stored at -80°C. Then, 600 ml of RLT buffer with $\beta$-mercaptoethanol ($\beta$-ME) were added and homogenized using the Ultra Turrax IKA T8.01 (Werka) homogenizer, previously autoclaved and submersed in *RNase Away.*

The total RNA was extracted by means of the *RNeasy Mini Spin* kit (Qiagen) according to the instructions of the manufacturer. The amount and the quality of the RNA extracted were determined by means of spectrophotometry at 260 and 280 nm in a quartz cuvette (Helma) and by means of the Agilent 2100 analyzer based on an electrophoresis on channels engraved on a solid support (chip) and subsequent quantification of the amount of RNA by means of the laser excitation of a fluorochrome which binds specifically to this type of molecule.

Extraction of blood RNA

The Paxgene kit (Analytix) was used, starting from 2.5 ml of blood. The purification started with a step of centrifugation to precipitate the nucleic acids in the PAXgene Blood RNA tube (BRT) .

After washing and resuspending the pellet, it was incubated with proteinase K (PK) in optimized buffers to digest the proteins. It was again centrifuged through a PAXgene centrifugation disruption column (PSC) to homogenize the cell lysate and eliminate the cell remains, and the supernatant of the eluted fraction was transferred to a clean microcentrifuge tube. Ethanol was added to adjust the binding conditions and the lysate was loaded in a PAXgene RNA centrifugation column (PRC). During a brief centrifugation, the RNA bound selectively to the PAXgene silica gel membrane while the contaminants traversed it. The remaining contaminants were eliminated in several effective steps of washing. Between the first and the second step, the membrane was treated with DNAse I (RNFD) to eliminate the remains of ligated DNA. After steps of washing the RNA was eluted in elution buffer (BR5) and denatured by heat.

Preparation of the pool of tissue samples

The reference samples were prepared from a pool formed by non-tumor samples of patients with CRC. These samples were selected discarding those which did not reach 2 μg of RNA (sufficient for performing 4 labelings of 500 ng each). The pool thus formed was aliquoted and stored at -80°C.

Amplification and Labeling

Starting from the extracted RNA, complementary DNA (cDNA) was obtained and was labeled with two different fluorochromes using the Low RNA Input Linear Amplification protocol (Agilent Technologies). As a labeling criterion, it was determined that the pool of non-tumor samples were labeled with Cyanine-3-CTP, whereas the tumor samples were labeled with Cyanine-5-CTP.

Synthesis of complementary DNA from total RNA and synthesis of labeled complementary cRNA

To carry out the retrotranscription, 500 ng of RNA were mixed with 1.2 μl of T7 Promoter Primer. The mixture was taken to a final volume of 11.5 μl with nuclease-free water and was denatured for 10 minutes at 65°C, followed by 5 minutes in ice. During said time interval, the complementary DNA synthesis reaction mixture was prepared, comprising 4 μl of 5X First Strand buffer, 2 μl of 0.1M DTT, 1 μl of mixture of dNTPs, 1 μl of MMLV reverse transcriptase and 0.5 μl of RNAse OUT was prepared. 8.5 ml of the reaction mixture were added to the tube containing the RNA sample together with the T7 primer, obtaining a total volume of 20 ml. They were incubated in a bath with stirring (Unitronic OR) at 40°C for 2 hours, followed by a second incubation in thermoblock at 65°C for 15 minutes, inactivating the retrotranscriptase.

The fluorochromes used were cyanine-3-CTP (Cye-3) and cyanine-5-CTP (Cye-5) (Perkin Elmer). The reaction was carried out mixing 2.4 μl of fluorochrome, 20 μl of cDNA and 57.6 μl of a reaction mixture formed by 15.3 μl of nuclease-free water, 20 μl of 4x transcription buffer, 6 μl of 0.1 M DTT, 8 μl of mixture of NTPs. 6.4 μl of 50% PEG, 0.5 μl of RNAse OUT, 0.6 μl of inorganic pyrophosphatase and 0.8 μl of T7RNA polymerase. The samples were incubated for two hours at 40°C in a bath with stirring.

The elimination of the non-incorporated fluorophore was carried out by means of the RNeasy Mini Spin kit (Qiagen) according to the instructions of the manufacturer. The labeled samples were measured in the spectrophotometer at two different wavelengths depending on the fluorochrome with which they were labeled. Those samples labeled with Cye-3 were measured at 550 nm, whereas those labeled with Cye-5 were measured at 650 nm. Likewise, all the samples were measured at 260 nm to verify the amount of RNA present. Once the samples were measured, a series of calculations were carried out to obtain the amount in picomoles of Cye-3 and of Cye-5.

Scheme of hybridization for the tissue samples

A pool was made with the non-tumor samples belonging to patients with CRC and was hybridized against 32 tumor samples and against 33 non-tumor samples.

Hybridization of the samples in Oligo-Microarrays.

The hybridization of the samples was carried out by means of the Agilent 60-mer oligo Microarray processing protocol

(Agilent Technologies).

Firstly, the 10X Control Target reagent was reconstituted with 0.5 µl of nuclease-free water. Then, the 2X cRNA solution was prepared mixing 0.75 µg of the cRNA labeled with Cy3 and of the cRNA labeled with Cy5, 50 µl of 10X control target and nuclease-free water until reaching a total volume of 240 µl. 10 µl of 25X fragmentation buffer were added and incubated for 30 minutes at 60°C. Once the incubation had ended, 250 µl of the hybridization buffer were added. 490 µl of the prepared solution were deposited on a support or gasket previously inserted in the hybridization chamber. The microarray was placed on top and it was verified that the liquid placed between both parts moves when the chamber rotates. Care was taken to prevent the formation of bubbles during the entire process since they interfere in the hybridization, giving as a result the presence of areas in the crystal in which the oligonucleotides do not bind to the cRNA of the samples.

The chambers were placed inside the hybridization oven (Agilent Technologies). They were maintained for 17 hours at a temperature of 60°C and a speed of 4 rpm. Once the hybridization time had elapsed, the chambers were subjected to a washing process to eliminate the remains of fluorochromes from those regions in which they may have been deposited in excess. To that end, solutions of 6xSSPE and 0.005% N-lauroylsarcosine (solution 1), 0.06X SSPE, 0.005% N-lauroylsarcosine (solution 2) and a mixture of ozone and acetonitrile (solution 3) were used.

The images were acquired using the GenePix 4000B scanner (Axon Instruments). This scanner uses two lasers of wavelengths 635nm and 532nm to excite the fluorochromes, as well as two photomultiplier (PM) tubes to detect light emission. Once the crystal was introduced in the scanner, a pre-scan was carried out and the PM tubes were manually adjusted until achieving that the fluorescence intensity of each of the two channels were similar. Due to the fact that the intensity signal of the channels is actually not identical, the software uses a normalization factor. The GenePix Pro 4.1 program calculates said normalization factor based on the premise that the arithmetic mean of the proportions of each spot of a microarray must be 1. When the two channels are perfectly balanced, the normalization factor is 1.

Treatment of the data

Once the image has been scanned, an array grid for the location of the scanned points (grid, supplemented by Agilent Technologies) corresponding to the 22574 genes present in the microarray was superimposed and the image was analyzed. Upon aligning the informative grid to the scanned image of the crystal, the software automatically labels those spots that it does not detect as "Not Found". Manually, once the image has been scanned, all the labels established by the program were eliminated, since it was considered that the spots eliminated for the analysis could contain some type of interesting information. Only those spots that could not be included in the analysis due to presenting the inclusion of a synthetic fiber throughout the hybridization process or incorrect adhesion of the fluorochrome were identified with a label (X). In these cases, the program identifies these points as "Bad Flag". Agilent includes a series of internal controls distributed over the entire crystal (NegativeControl, N/A, Pro25G and eQC). Said controls were eliminated, since their expression values could affect the values of the rest of the genes analyzed.

In this work, the following data pre-processing chain was established: Normalization, quality metrics, imputation of missing values and intra-class differences

(i) Normalization

The need for normalization of the data of microarrays is due to the fact that, in a biological experiment, the number of genes which are differentially expressed has to be low. Once the internal controls were eliminated, the file generated was transferred to the Acuity 4.0 analysis software (Axon Instruments), in which the data was normalized by means of Lowess algorithm (Cleveland, W. S., 1979, 74:829-836), which assumes that the data LogRatio must be symmetrical towards zero in any intensity interval. The LogRatio value is the base 2 logarithm of the proportion of the medians and allows obtaining an activity change index.

Negative values indicate a decrease in the expression between the control and test population, whereas positive values indicate an increase. Each crystal, once the controls were eliminated, comprised a set of 17986 probes or spots. However, as has been previously commented, physical problems can occur throughout the hybridization process, it being necessary to discard some probes. Therefore, it was first necessary to determine the quality of the hybridized microarrays.

(ii) Evaluation of quality of the microarrays hybridized

The fact that an image is bright does not mean that its quality is good. The same probe can vary its intensity based on different detectors, on variations in the processing of the signal and other design differences. Agilent dual-channel microarrays quantify signals in two different wavelengths: 635 nm for fluorochrome Cy5 and 532 nm for Cy3.

To that end, two signals per wavelength must be quantified: the intensity reading of the transcript and the background intensity reading associated to this wavelength. These four readings are carried out by quantifying the regions (pixels) corresponding to each probe within the microarray. Their final value corresponds to a mean or median of unit values, together with their corresponding standard deviation. The limit of detection indicates the minimum size of the signal which a device capable of quantifying correctly. The most reliable measurement for determining a threshold for this limit of detection is the signal-to-noise ratio (SNR).

Collecting the readings carried out by the scanner and following the steps of Chen et al. (CHEN, Y., et al., 2002, Bioinformatics, 18:1207-1215), the use of three quality metrics is proposed. These measurements have values between 0 and 1, 1 being the maximum value and 0 the minimum value of reliability:

1.- Quality of the fluorescence intensity measurement ($W_I$). For a certain gene, an SNR equal to or greater than 6 indicates that the signal is very strong compared to the background, therefore the quality of the signal is perfect. In the same way, if this value is less than 3, it indicates that the accuracy is not reliable.

2.- Quality of the background *flatness* or background ($W_{BR}$) . Due to different anomalies in the background of the array, there may be problems in the detection of signals or incorrect measurements of the intensity level of that background. One way to detect these anomalies is to compare the background intensity of the probe for one of the channels (for example, the red channel) with the global mean intensity of the channel. The background *flatness* of the green channel will be similarly defined and the overall *flatness* coefficient will be defined as the minimum value between both channels.

3.- Quality of the consistency in the signal intensity (wS). Due to physical problems in the technology, it is possible that the obtained intensity level of a signal does not reflect the actual level due to the fact that its standard deviation is unusually high. The described quality metrics for solving this problem is based on the coefficient of variation $CV=\sigma/\mu$ Based on these metrics, the global quality metrics by probe and microarray is calculated as the minimum value of all of them:

$$W_K = min \ (w_I, \ w_{BR}, \ w_S)_K$$

(iii) Imputation of the missing values

Once the probes which did not comply with the established quality criteria were discarded, the number of missing values was determined. The spot intensities for which there are no quantification results are called missing values. Missing values cause problems in the treatment of the microarray data as they interfere with the statistical tests applied. To that end, it was necessary to substitute them with estimated values in a process called imputation. Among the different forms existing for carrying out the imputation, the application of the KNNimpute (K-Nearest Neighbor) algorithm (TROYANSKAYA, O., et al., 2000, Bioinformatics, 17:520-525) was used, which selects all the expression profiles similar to the gene to be imputed.

Statistical Analyses

The normalized data was analyzed by the Grupo de Sistemas Inteligentes (Intelligent Systems Group) of the Universidad del Pais Vasco (UPV/EHU) in order to select the most relevant genes in the study of the samples. A supervised classification model was carried out which was initially formed by 65 instances or cases and 4 classes or values for the supervised variable (non-tumor, stage B, stage C and stage D). Firstly, a series of different univariate metrics allowing the creation of a consensus gene ranking was used. The metrics used were:

- Mutual information. It is an amount measuring the mutual dependence between two genes.
- Euclidean distance. It is the common distance measurement.
- Matusita metric. The original formulation of this metric measures the distance between two probability distributions
- Kullback-Leibler divergence. It is the most known method for measuring the differences between two probability distributions.
- Bhattacharyya metric. It measures the dependence existing between two probability distributions.

The gene rankings separately provided by each metric were then collected and a consensus or common ranking was established. Once established, the analysis was continued with the search for those genes which were relevant and non-redundant using a method known as CFS (correlation-based filter selection). Finally, the analysis was completed with the creation of highly reliable Bayesian networks in which each of the genes was compared with the stage variable.

Real-time Polymerase Chain Reaction (RT-PCR)

For the purpose of validating the results obtained in the study of the tissue samples, a real-time polymerase chain reaction (PCR) or quantitative PCR was carried out, using the 18S gene as a reference or normalizing (housekeeping) gene (BAS, A., et al., 2004, Scand. J. Immunol. 59:566-573.)

This study was validated using 15 samples not included in the previous hybridizations and taking care to select them such that all the studied tumor stages were included. The global tally was 4 non-tumor samples, 3 stage B2 samples, 1 stage B3 sample, 2 stage C samples, 2 stage C2 samples and 3 stage D samples.

The RNA was extracted by means of the procedure described in RNeasy Plus Mini Kit (Qiagen), which includes an elimination column for this type of DNA. Once the RNA was extracted, its quality and amount were checked by means of the Agilent 2100 Bioanalyzer equipment. Starting from 500 ng of total RNA in a volume of 10 μl, the retrotranscription was carried out by means of the Taqman Reverse Transcription Reagents protocol (Applied Biosystems). The reaction mixture was prepared with 2.5 ml of PCR buffer, 5.5 μl of $MgCl_2$, 5 μl of dNTPs, 1.25 μl of random hexamers, 0.25 μl of RNase inhibitor and 0.625 μl of the retrotranscriptase enzyme. 15 μl of said mixture were added to the 10 μl of RNA and the reaction was carried out in a MyiQ Single-Color Real-Time PCR Detection System thermal cycler (Biorad) with successive incubations at 25°C for 10 minutes, 48°C for 60 minutes, 95°C for 5 minutes and at 4°C for 8 minutes.

Once the complementary DNA was obtained, real-time PCR was carried out using Platinum Quantitative PCR Supermix-UDG with ROX procedure (Invitrogen). The reaction mixture was made by adding 2.8 μl of water, 10 μl of the Supermix mixture, 1.2 μl of $MgCl_2$ and 1 μl of the probe with the primers, either for the gene to be studied or for the gene used as a normalizing or housekeeping gene (18S). 5 ml of cDNA (2 ng/μl) were added to 15 μl of the reaction mixture. The process was carried out in 96-well plates. A blank well was added in which cDNA was not included. The program used in the MyiQ thermal cycler (Biorad) consisted of 2 cycles at 95°C for 2 minutes, 1 cycle at 95°C for 15 seconds, 40 cycles at 60°C for 1 minute and 1 cycle at 25°C for 8 minutes.

Once the expression values for each analyzed gene were obtained, the $2^{-\Delta\Delta Ct}$ method (LIVAK, K. J., et al., 2001, Methods, 25:402-408) was applied, using the group of non-tumor samples as a calibrator.

Three different assays were carried out for each gene, with two replicas in assay one and three replicas in the following assays.

EXAMPLE 1

Samples of patients

The 120 samples of patients with colorectal cancer, as well as the 13 samples from healthy patients for said disease were extracted by means of the RNeasy Mini kit protocol (Qiagen). Once the total RNA was isolated, its quality and amount were determined by means of the spectrophotometer and by means of the Agilent 2100 Bioanalyzer (Agilent Technologies). The RIN algorithm was the metric used to establish the selection of samples (Imbeaud et al., 2005, Nucleic Acids Res., 33:1-12). As they were tissue samples, there is a great heterogeneity between different samples and even between different parts of the same sample. For this reason, those extractions which resulted in a bad RNA quality were repeated up to a maximum of 3 times. In this case, the sample acceptance threshold was established in a RIN of 5.6, whereby after extracting the RNA of all the samples, the final tally of accepted samples was included in Table 1.

Table 1: Total number of samples received and subsequently accepted based on the RIN number

|  | Healthy patients for CRC | Patients with diagnosed CRC | |
|---|---|---|---|
|  |  | Non-tumor part | Tumor part |
| Initial samples | 13 | 60 | 60 |
| Valid samples | 4 | 42 | 32 |

Table 1 shows that only 4 healthy patients for CRC obtained an RNA quality within the established criterion. The reason for extracting the RNA of said samples was due to the need for forming a pool of healthy samples against which the rest of the analyzed samples would be compared. Due to the fact that the number of valid healthy samples was very low to form the pool, the decision was made to form a new one from the non-tumor samples belonging to patients with colorectal cancer. To determine which of the non-tumor samples had to be included in the new pool, the availability was fixed as a criterion in order to be able to carry out at least 8 labelings, considering that an amount of extracted total RNA of 500 ng is necessary for each one. According to said criterion, 33 non-tumor samples were selected to form the reference pool which, with the exception of one sample, were the same non-tumor samples which were selected to hybridize against said pool. In relation to the tumor samples, out of the 60 collected initial samples, 32 showed an acceptable RNA quality and amount.

Table 2 shows the complete list of tumor and non-tumor samples used in the study.

Table 2: Total number of samples from valid patients (35 non-tumor samples and 32 tumor samples). The selection was carried out based on the RIN number. The cutoff threshold was established in a RIN of 5.6, corresponding to sample M40. The numbers between brackets indicate the extraction number in each case. For the preparation of the pool, all the non-tumor samples were used, with the exception of sample M106 and sample M116, which was not used due to the fact that it was the one with the lowest amount of RNA. The final tally consisted of 33 non-tumor samples and 32 tumor samples.

| INTERNAL CODE | Tumoral/No Tumoral | AMOUNT ng/ul (BIOANALYZER) | RIN | INTERNAL CODE | Tumoral/No Tumoral | AMOUNT ng/ul (BIOANALYZER) | RIN |
|---|---|---|---|---|---|---|---|
| M1 | No Tumoral | 284 | 8,6 | M56 | Tumoral | 225 | 6,6 |
| M2 | Tumoral | 451 | 9,7 | M59 | Tumoral | 1428 | 7,3 |
| M5 | No Tumoral | 146 | 7,9 | M60(2) | No Tumoral | 339 | 7,2 |
| M6 | Tumoral | 113 | 7,5 | M63 | Tumoral | 536 | 6,9 |
| M7 | Tumoral | 322 | 5,7 | M65 (2) | No Tumoral | 1006 | 7,8 |
| M8 | No Tumoral | 146 | 8,7 | M66 | Tumoral | 270 | 8,4 |
| M9(2) | Tumoral | 1757 | 6,8 | M69 | Tumoral | 510 | 6,2 |
| M10(2) | No Tumoral | 108 | 8 | M71(2) | No Tumoral | 147 | 6,6 |
| M11(2) | No Tumoral | 1111 | 6 | M72(3) | Tumoral | 629 | 6,5 |
| M12 | Tumoral | 512 | 7,6 | M74 | No Tumoral | 364 | 7,3 |
| M13 | Tumoral | 347 | 8,7 | M78 | No Tumoral | 1650 | 7,3 |
| M14(3) | No Tumoral | 129 | 6,6 | M80 | No Tumoral | 160 | 6,8 |
| M16 | Tumoral | 466 | 9,1 | M81(2) | Tumoral | 472 | 5,8 |
| M19 | Tumoral | 528 | 8,9 | M83 | No Tumoral | 1087 | 6 |
| M20 | No Tumoral | 546 | 6,1 | M87 | Tumoral | 415 | 6,5 |
| M21 | Tumoral | 905 | 8,3 | M88 | No Tumoral | 850 | 6,2 |
| M24 | Tumoral | 92 | 6,7 | M89 | Tumoral | 920 | 8,3 |
| M25(2) | No Tumoral | 638 | 8 | M90 | No Tumoral | 1021 | 6,7 |
| M26 | Tumoral | 540 | 7,8 | M94 | No Tumoral | 401 | 6 |
| M29 | No Tumoral | 444 | 6,6 | M95 | Tumoral | 627 | 6,7 |
| M36(2) | Tumoral | 445 | 6,4 | M97(2) | No Tumoral | 267 | 5,8 |
| M37(3) | No Tumoral | 812 | 6,5 | M99 | No Tumoral | 448 | 7,2 |
| M40 | Tumoral | 902 | 5,6 | M100(2) | Tumoral | 1408 | 6,9 |
| M41 | No Tumoral | 415 | 9,2 | M105(3) | Tumoral | 1072 | 7,3 |
| M42 | Tumoral | 1306 | 6,9 | M106 | No Tumoral | 85 | 7,5 |
| M43 | No Tumoral | 643 | 6,5 | M107 | No Tumoral | 124 | 7,7 |
| M44 | Tumoral | 1242 | 7,2 | M109(2) | Tumoral | 779 | 9,2 |
| M49 | No Tumoral | 236 | 6 | M111(3) | Tumoral | 710 | 6,5 |
| M50 | Tumoral | 1248 | 6 | M112 | No Tumoral | 127 | 8 |
| M51 | No Tumoral | 858 | 7,2 | M114 | No Tumoral | 513 | 7,6 |
| M53 | No Tumoral | 264 | 6,4 | M116 | No Tumoral | 72 | 8,6 |
| M54 | Tumoral | 278 | 6,5 | M119 | Tumoral | 849 | 8,5 |
| M55 | No Tumoral | 1002 | 7,3 | M120 | No Tumoral | 1173 | 6 |

Each paired sample corresponded to a certain stage of colorectal cancer, diagnosed by Pathological Anatomy of the Hospital de Cruces. Table 3 shows the clinical data of the patients:

Table 3: Clinical data of the patients diagnosed with CRC. The table shows the tumor samples. The stages have been determined according to two different classifications: the old one (Duke's) and a new one (TNM). The abbreviations of each indicate: localized tumor without ganglions =IA, IIA, IIB, IIC and A, B1, B2; localized tumor with ganglions = IIIA, IIIB, IIIC and C1, C2, C3; metastatic tumor = IV and D. (*)mucinous adenocarcinoma. (**) several polyps in the piece. The sex column indicates M = male and F = female.

| INTERNAL CODE | T/NT | TNM | DUKES | AGE | SEX |
|---|---|---|---|---|---|
| M2 | T | IV | D | 46 | F |
| M6 | T | IIA | B2 | 69 | M |
| M7 | T | IIB | B3 | 68 | M |
| M9(2) | T | IV | D | 63 | M |
| M12 | T | IIIA | C1 | 87 | F |
| N13 | T | IIIC | C2 | 68 | F |
| M16 | T | IV | D | 81 | M |
| M19 | T | IV | D | 77 | F |
| M21 | T | IA | B1 | 73 | M |
| M24 | T | IIIC | C3 | 47 | M |
| M26 | T | IIA | B2 | 71 | F |
| M36(2) | T | IIIB | C2 | 73 | F |
| M40 | T | IIA | B2 | 55 | M |
| M42 | T | IIA | B2 | 66 | F |
| M44 | T | IA | B1 | 77 | M |
| M50 | T | IA | B1 | 46 | M |
| M54 | T | IA | D | 50 | M |
| N56(**) | T | IIIB | D3 | 67 | F |
| N59 | T | IV | D | 83 | M |
| M63 (9***) | T | IV | D | 74 | M |
| M66 | T | IIIB | C2 | 71 | F |
| M69 | T | IIIB | C2 | 65 | M |
| M72(3) | T | IIA | B2 | 57 | M |
| N81(2) | T | IIA | B2 | 71 | M |
| M87 | T | IIA | B2 | 76 | F |
| M89 | T | IIIB | C2 | 57 | F |
| M95 | T | IIA | B2 | 63 | M |
| M100(2) | T | I | A | 76 | M |
| M105(3) | T | IIA | B2 | 75 | F |
| M109(2) | T | IV | D | 69 | F |
| M111(3) | T | IIIA | C1 | 62 | F |
| M119 | T | IIIB | C2 | 67 | M |

Therefore and taking into account Dukes' classification system, the tumor samples consisted of 1 Dukes' stage A sample, 13 Dukes' stage B samples, 10 samples belonging to Dukes' stage C and 8 Dukes' stage D samples.

The constructed pool was labeled with fluorochrome Cye-3, whereas the tumor samples as well as the non-tumor samples were labeled with Cye-5. In both cases, once labeled and measured in the spectrophotometer at the wavelengths corresponding to each fluorochrome (550 and 650 nm respectively), the samples were aliquoted in amounts containing 0.75 mg of labeled cRNA and stored in darkness at -80°C until their hybridization.

In 22K human 1A Oligo Microarrays, non-tumor samples or tumor samples were compared against a pool formed by non-tumor samples. Using 0.75 $\mu$g of labeled complementary RNA complementary, the hybridization was carried out in the manner explained in materials and Methods.

A total of 65 crystals were obtained. Each crystal is identified with a barcode provided by Agilent. Said code was used to identify each of the microarrays. Annex VII shows the microarrays used and the samples hybridized therein.

Results: Tissue Samples

The internal controls present in each of the crystals were eliminated as a step prior to the normalization of the data by means of the Lowess algorithm. Once normalized, the microarrays were sent to the Grupo de Sistemas Inteligentes (Intelligent Systems Group) of the Universidad del Pais Vasco (UPV-EHU), where a supervised model was created. Said model was formed by 4 classes, corresponding to stages B, C and D (according to the Dukes' classification) and to the non-tumor samples. In spite of starting from 65 crystals, it was necessary to eliminate one of them, the hybridized sample in it was the only representative of Dukes' stage A (sample M100) Therefore, the distribution of the 64 samples by cases was divided into:

- 33 non-tumor samples (NT)
- 13 tumor samples in stage B
- 10 tumor samples in stage C
- 8 samples in stage D.

In spite of the fact that 64 crystals were finally accepted, the analysis of the data started taking into account all the crystals.

Determination of the quality

The global quality metric ($W_k$) was calculated. The quality of a gene or probe will be determined by the mean of the qualities of said probe throughout all the microarrays analyzed. In this case, the value of 0.99 was established as the quality acceptance threshold for a probe. In this way, out of the 17986 probes corresponding to genomic sequences, 11120 exceeded the threshold and were accepted to continue with the subsequent analyses.

Imputation of the missing values

In the 65 microarrays analyzed, 17147 missing values were determined, which is 1.47% of the values. The imputation of said values was carried out by means of the KNNimpute algorithm. The value of K was determined in 15 neighbor genes. After the step of quality metrics filtration, the total number of missing intensities was 7534 values, i.e., 1.04% of the values which passed through the quality filtration. The fact that the percentage is so low gives an idea of the reliability of the imputed values.

Intra-class variability

The tissue samples had a high degree of heterogeneity in their composition, since it includes several cell types. Therefore, between samples of the same type, great differences in the expression profiles of certain genes may occur. These differences are known as intra-class variability.
Taking into account that the aim in this case is the search for genes with a differential behavior between the different stages of the cancer, those which had great differences in their expression levels belonging to one and the same class were not considered. Based on the maximum differences detected, a gene acceptance criterion was fixed as that which did not have an intra-class variability greater than or equal to 2-fold. Starting from the 11120 genes which passed the quality criterion, 3016 genes which did not comply with the established criterion were eliminated. Therefore, the set of valid genes for the subsequent analyses consisted of a total of 8104 genes and 64 cases.

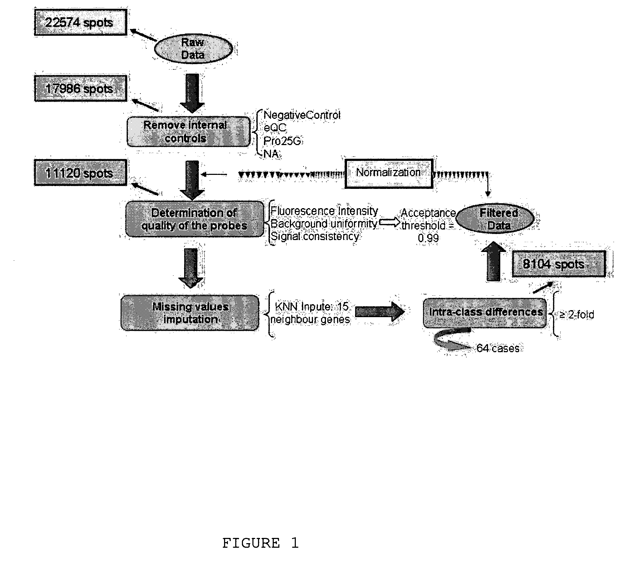

Figure 1 shows a graphic summary of the steps to be carried out for cleaning and pre-treating the data, determining the number of probes or genes useful in subsequent analyses. Obtaining a list of relevant genes
For the purpose of determining a set of representative genes, two filtration approximations were carried out, which allowed detecting a set of relevant genes both individually and collectively. One of the most extended techniques within these approximations is carrying out importance classifications or rankings of genes. Mutual information metrics, Euclidean distance, Batusita metric, Kullback-Leibler divergence and Bhattacharyya metric, explained above, were used to carry out this part.
All these metrics are univariate, i.e., they only take into account the relation existing between the analyzed gene and its corresponding stage. Each of them separately provided a classification of the 8104 genes, such that a consensus with all of them was carried out. Table 4 shows the first 50 positions of the consensus ranking obtained.

Table 4. Consensus gene ranking. The table indicates: the position occupied by each of the genes, the Agilent identification for each probe, the name of the gene to which it belongs, the description and finally the accession number.

| Ranking | Agilent Identifier | GENE | Description | Accession Number |
|---|---|---|---|---|
| 1 | A_23_P213424 | ENC1 | ectodermal-neural cortex (with BTB-like domain) | NM_003633 |
| 2 | A_23_P24515 | ACAT1 | acetyl-Coenzyme A acetyltransferase 1 (acetoacetyl Coenzyme A thiolase) | NM_000019 |
| 3 | A_23_P24716 | TMEM132A | transmembrane protein 132A | NM_017870 |
| 4 | A_23_P40309 | SNRPB2 | small nuclear ribonucleoprotein polypeptide B" | NM_003092 |
| 5 | A_23_P13663 | FAM60A | family with sequence similarity 60, member A | NM_021238 |
| 6 | A_23_P142872 | TCF7L1 | Transcription factor 7-like (T-cell specific HMG | NM_031283 |
| 7 | A_23_P47843 | DDX55 | DEAD (Asp-Glu-Ala-Asp) box polypeptide 55 | NM_020936 |
| 8 | A_23_P114282 | MCTS1 | malignant T cell amplified sequence 1 | NM_014060 |
| 9 | A_23_P103149 | ACO2 | Aconitase 2 | NM_001098 |
| 10 | A_23_P207999 | PMAIP1 | Phorbol-12-mystirate-13-acetate-induced protein 1 | NM_021127 |
| 11 | A_23_P63584 | AHCTF1 | AT hookcontaining transcription factor Similar to adenosylhomocysteinase hidrolase | NM_015446 |
| 12 | A_23_P22086 | LOC649828 | (S-adenosyl-L-homocysteine) (Liver copper binding protein ) (CUBP) | NM_927818 |
| 13 | A_23_P88522 | NMB | Neuromedin B | NM_021077 |
| 14 | A_23_P256413 | CMTM7 | CKLF-like MARVEL transmembrane domain containing 7 | NM_138410 |
| 15 | A_23_P153615 | MADCAM1 | mucosal vascular addressin cell adhesion molecule 1 | NM_130760 |
| 16 | A-23-P112412 | TEX10 | Testis expressed 10 | NM_017746 |
| 17 | A_23_P209070 | LG14 | Leucine rich repeat LGI family | BC087848 |
| 18 | A_23_P123343 | NUDCD1 | NudC domain containing 1 | NM_032869 |
| 19 | A_23_P163179 | CALM1 | Calmodulin 1 (phosphorilase kinase, delta) | NM_006888 |
| 20 | A_23_P253412 | MRLP30 | Mitochondrial ribosomal protein L50 | NM_019051 |
| 21 | A_23_P34018 | RPL39 | ribosomal protein L30 | NM_001000 |
| 22 | A_23_P70827 | KIAA1549 | Unknown function | AL136736 |

(continued)

| Ranking | Agilent Identifier | GENE | Description | Accession Number |
|---|---|---|---|---|
| 23 | A_23_P113634 | CBFB | core-binding factor beta, subunit of dimeric polyomavirus enhancer binding transciption factor | NM_001755 NM_001755 |
| 24 | A_23_P30464 | PRR7 | Proline rich 7 (synaptic) | NM_030567 |
| 25 | A_23_P37375 | RPS6KA5 | Ribosomal protein S6 kinase A5 | NM_004755 |
| 26 | A_23_P48771 | C14orf159 | Chromosome 14 open reading frame | NM_024952 |
| 27 | A_23_P127175 | SAR1A | SAR gen homolog A (S.cerevisiae) | NM_020150 |
| 28 | A-23-P141180 | TOM1L2 | Target of myb-like 2 | AK055959 |
| 29 | A_23_P639399 | MST1 | Macrophage stimulating 1 protein | NM_020998 |
| 30 | A_23_P131846 | SNAL1 | Snail 1 homolog (Drosophila) | NM_005985 |
| 31 | A_23_P121657 | HS3ST1 | Heparan sulfate D-glucosaminyl 3-O-sulfotransferase | NM_003114 |
| 32 | A_23_P33027 | MLXIP | MLX interacting protein | NM_014938 |
| 33 | A_23_P123343 | NUDCD1 | NudC domain containing 1 | NM_032869 |
| 34 | A_23_P108676 | TMEM166 | Transmembrane protein 166 | NM_032181 |
| 35 | A_23_P103201 | PNRC2 | Proline rich nuclear transcription co-activator 2 | NM_017761 |
| 36 | A_23_P51269 | CD641036 | Protein with high similarity to BTF3 | CD641036 |
| 37 | A_23_P123330 | RPL30 | Ribosomal protein L30 | NM_000989 |
| 38 | A_23_P252118 | POLB | DNA polimerase beta | NM_002690 |
| 39 | A_23_P215517 | KLHL7 | Kelch-like 7 | BC00955 |
| 40 | A_23_P145194 | BYSL | Bystin-like protein | NM_004053 |
| 41 | A_23_P134274 | POP7 | Processing of precursor 7, ribonuclease protein subunit | NM_005837 |
| 42 | A_23_P70915 | ORAI2 | ORAL calcium release-activated calcium modulator 2 | NM_032831 |
| 43 | A_23_P156890 | TCF21 | Transcription factor 21 | NM_003206 |
| 44 | A_23_P51906 | PFDN2 | Prefoldin subunit 2 | NM_012394 |
| 45 | A-23-P114232 | PRDX4 | Peroxirredoxin 4 | NM_006406 |
| 46 | A_23_P215525 | OSBPL3 | Oxysterol binding protein like 3 | NM_015550 |
| 47 | A_23_P27867 | PLAUR | Plasminogen activator, urokinase receptor | NM_002659 |
| 48 | A_23_P157405 | CHCHD2 | Coiled-coil-helix-coiled-coil domain containing 2 | NM_016139 |
| 49 | A_23_P118102 | NDUFB10 | NADH dehydrogenase (ubiquinone) 1 beta subcomplex | NM_004548 |

(continued)

| Ranking | Agilent Identifier | GENE | Description | Accession Number |
|---------|-------------------|------|-------------|------------------|
| 50 | A_23_P78423 | **ATP5A1** | ATP synthase H+ transporting mitochondrial F1 | NM_001001937 |

Once this ranking was obtained, the second filtration approximation was carried out, which consisted of the search for a subset of genes. This search has two essentials aspects: redundancy and irrelevance. The selected subset must thus have the lowest possible redundancy and at the same time contain the most relevant genes. To carry out this approximation, a correlation-based method was used: CFS (Correlation-based Filter Selection) presented by Hall and Smith (HALL, M. A., et al., 1997). By means of this method, it was achieved that those irrelevant genes were not included since they were bad stage predictors and at the same time, the genes which were redundant due to their correlation with previously included genes were ignored. The measure of correlation between two genes is known as coefficient of uncertainty. This coefficient can have values comprised between 0 and 1. Thus, a gene will be redundant with another one when the measure of correlation is equal or close to 1, whereas a value of 0 indicates that there is no relation between these two genes. Once the genes have been compared to one another and the redundant and irrelevant genes have been discarded, each gene was compared with the stage variable. In this case, if the value of correlation is close to 1, it would be a relevant gene in colorectal cancer. Finally, based on already filtered data, highly reliable Bayesian networks were prepared. A Bayesian network is defined as a diagram formed by two types of elements: a set of nodes (one for every gene) and a set of directed arcs connecting the nodes. If there is an arc coming out of node X and connecting with Y, it is said that "X is a parent of Y". Intuitively, in a Bayesian network, an arc going from X to Y indicates that there is an direct influence of X on Y. 1000 random re-samplings were carried out from the database and in each of them the CFS technique was applied to select the genes, constructing a Bayesian classifier, kBD (SAHAMI, M., 1996), each time. Then, the statistical dependences and the genes which appeared most times in the entire process were identified with the thousand classifiers. Starting from the 8104 genes, throughout the 1000 gene selections, 3625 genes were collected. If the trend followed by the selection of these genes is observed, it is seen that there are a few genes (77 of the 8104 initial genes) which have been selected by a number equal to or greater than 100 times. Table 5 shows the complete list of the 20 genes selected most times

Table 5. First twenty most selected variables. The table shows the name and description of the gene, its Agilent identifier, the accession number and the amount of times it has been selected in the analyses conducted.

| Ranking | Agilent Identifier | GENE | Description | Accession Number |
|---------|-------------------|------|-------------|------------------|
| 961 | A_23_P24515 | **ACAT1** | acetyl-Coenzyme A acetyltransferase 1 (acetoacetyl Coenzyme A thiolase) | NM_000019 |
| 820 | A_23_P213424 | **ENC1** | ectodermal-neural cortex (with BTB-like domain) | NM_003633 |
| 567 | A_23_P24716 | **TMEM132A** | transmembrane protein 132A | NM_017870 |
| 547 | A_23_P256413 | **CMTM7** | CKLF-like MARVEL transmembrane domain containing 7 | NM_138410 |
| 512 | A_23_P7353 | **LARP2** | La ribonucleopeptide domain family, member 2 | NM_178043 |
| 435 | A_23_P153615 | **MADCAM1** | mucosal vascular addressin cell adhesion molecule 1 | NM_130760 |

(continued)

| Ranking | Agilent Identifier | GENE | Description | Accession Number |
|---|---|---|---|---|
| 330 | A_23_P26717 | RPL23 | Ribosomal protein L23 | NM_000978 |
| 310 | A_23_P13663 | FAM60A | family with sequence similarity 60, member A | NM_021238 |
| 287 | A_23_P47843 | DDX55 | DEAD (Asp-Glu-Ala-Asp) box polypeptide 55 | NM_020936 |
| 275 | A_23_P13102 | CASP12 | Caspase 12 | NM_014383 |
| 273 | A_23_P35521 | P4HA1 | Proline-4-hidroxylase alpha polypeptide 1 | NM_000917 |
| 267 | A_23_P27964 | HOMER-3 | Homer neuronal immediate early gene 3 | NM_004838 |
| 249 | A_23_P40309 | SNRPB2 | small nuclear ribonucleoprotein polypeptide B" | NM_003092 |
| 246 | A_23_P38262 | ELAC2 | ElaC homolog 2 | NM_018127 |
| 238 | A_23_P113634 | CBFB | core-binding factor beta, subunit of dimeric polyomavirus enhancer binding transciption factor | NM_001755 |
| 233 | A_23_P210253 | DGKD | Diacylglycerol kinase delta | NM_003648 |
| 221 | A_23_P33075 | LO8961 | Similar to c-Mer protooncogen tyrosine kinase (human MERTK) | LO8961 |
| 221 | A_23_P131846 | SNAL1 | Snail 1 homolog (Drosophila) | NM_005985 |
| 218 | A_23_P206268 | TRAPPC2L | Trafficking protein particle complex 2-like | NM_016209 |
| 208 | A_23_P35125 | SF3B4 | Splicing factor 3b (SF3B) subunit 4 | NM_005850 |

Relevant genes in the classification of Tumor and Non-tumor Samples

To obtain the relevant genes allowing the implementation of a classificatory model which distinguishes a tumor tissue from a healthy one, the database was configured in: Tumor and No-tumor. Based on the results obtained and the algorithm made, 10 genes were selected based on their importance or weight within the constructed model.

The gene which showed more weight within the model was ENC1 (ectodermal neural cortex-1, NM_003633) and, secondly, the ACAT1 (acetyl-coenzyme A acetyltransferase 1, NM_000019) gene, an enzyme catalyzing cholesterol esterification.

Validation of the results by means of RT-PCR

For the purpose of validating the 10 relevant genes in the healthy/tumor distinction and due to the statistical results obtained in the microarray analysis, it is necessary to use an independent methodological criterion, preferably with different samples (Murphy, D., 2002, Adv. Physiol. Edu. 26:256-270.). One of the most used methods for confirming the results obtained from microarrays is the quantitative real-time polymerase chain reaction (qRT-PCR).

This technique has become the most used method for validating the results obtained from the microarray analysis. The validation was carried out according to the previously described protocol. For the purpose of the validation having a higher reliability, the samples used were different from those included in the study. 18 new samples were received, from which the total RNA total was extracted and furthermore, the RNA of some of the previous unused samples was obtained again in order to eliminate the possible remains of genomic DNA. The final tally of the samples included in the validation is shown in Table 6.

Table 6. Samples included in the RT-PCR validation study. Samples "M" correspond to previous samples which were not included in the hybridizations. The samples annotated with "V" correspond to subsequently received samples. They are assigned with a "plus" to discard that they have been extracted with the genomic DNA elimination kit.

| INTERNAL CODE | T/NT | ng/μl BIOANALYZER | RIN | dUKES STAGE | TNM STAGE |
|---|---|---|---|---|---|
| M30plus | T | 1685 | 8 | B2 | IIA |
| M46plus | T | 1040 | 6 | D | IV |
| M52plus | T | 1600 | 9.4 | C2 | IIIC |
| M84plus | T | 1160 | 7.3 | C2 | IIIC |
| M117plus | T | 1180 | 8 | C | III |
| V1plus | T | 1296 | 8.5 | B2 | T3N0M0 |
| V2plus | T | 2010 | 7.6 | D | T3N1M1 |
| V4plus | T | 2200 | 7 | B3 | T4N0M0 |
| V5plus | T | 2140 | 8 | C | T2N1M0 |
| V6plus | T | 440 | 6.6 | D | T4N2M1 |
| V7plus | T | 2025 | 8 | B2 | T3N0M0 |
| M104plus | NT | 1255 | 6.8 | - | - |
| M116plus | NT | 1740 | 7.7 | - | - |
| V10plus | NT | 1330 | 7.5 | - | - |
| V12plus | NT | 2190 | 7.8 | - | 6 |

The essential parameter in a real-time PCR is the threshold cycle (or Ct). It is defined as the number of PCR cycles from which the amplified product is detected (HU, N., et al., 2006). It is inversely proportional to the number of copies of the gene of interest.

Two different methods are used when quantifying the results. On one hand, there is the use of the calibration line, which is constructed from known DNA concentrations and, once made, the results of the samples are extrapolated. However, in this case, the quantification was carried out by means of the comparative Ct method, also known as the $2^{-\Delta\Delta Ct}$ method, using the 18S rRNA gene as the reference (housekeeping) gene.

The final validation results are shown in Table 7:

Table 7. Comparison of the results obtained in the validation by means of RT-PCR. The expression values obtained from the microarrays are shown at the left of the table, whereas the $2^{-\Delta\Delta Ct}$ values are shown at the right.

| | MICROARRAYS | | | RT-PCR | | |
|---|---|---|---|---|---|---|
| Gene | Dukes B | Dukes C | Dukes D | Dukes B | Dukes C | Dukes D |
| ENC1 | 1.66 | 1,405 | 1,512 | 13.12 | 6.69 | 3.7 |
| ACAT1 | -0.884 | -0.825 | -1.287 | 0.403 | 0.53 | 0.21 |
| TMEM132A | 0.865 | 0.867 | 1.398 | 13.293 | 9.383 | 5.805 |
| CMTM7 | 0.752 | 0.8305 | 1.277 | 3.66 | 7.637 | 2.05 |
| FAM60A | 1.346 | 0.829 | 1.3475 | 2.12 | 2.38 | 1.23 |
| MADCAM1 | -0.534 | -0.831 | -0.5605 | 0.36 | 0.9 | 0.03 |
| DDX55 | 0.876 | 0.779 | 0.675 | 1.97 | 2.59 | 1.76 |

The gene expression values obtained in the microarray analysis are shown as Lowess-normalized base 2 logarithm, such that 0 is established as the threshold value. Positive values indicate an overexpression of that gene, whereas negative values mean that that gene is repressed. The microarrays were grouped according to the stage of the hybridized tumor sample against the pool (13 stage B microarrays, 10 stage C microarrays and 8 stage D microarrays). The median of the LogRatios for each gene analyzed within each stage, as well as the median of the expression values of said gene in the 33 microarrays of non-tumor samples were calculated. The values shown are the result of the difference between the total expression of each gene in stage B, C or D, minus the mean expression value of each gene in the non-tumor samples.

In addition, by means of calculating $2^{-\Delta\Delta Ct}$, the data is shown as the number of times that a gene is expressed in a condition with respect to a control. In this case, the conditions are the different stages and the control are the non-tumor samples used. For the latter, the calculation of $\Delta\Delta Ct$ is 0 and 20 is 1, therefore the value in the non-tumor samples of each of the validated genes is 1. Therefore, values of a gene above 1 indicate that that gene is expressed that number of time more (fold-change) in the condition tested with respect to the control. Values below 1 indicate that that gene is more expressed in the control.

Therefore, the set of the 7 remaining validated genes and the interrelations obtained from the model is capable of classifying tissue samples in tumor and non-tumor samples.

Relevant genes in the classification of samples by stages

- Distinction between stages D and B/C. In this analysis, only the data from the tumor samples was used. After labeling the cases in two stages, D and B/C, the search for relevant genes in this problem was carried out. The resulting set are the 9 genes included in Table 8

Table 8: Relevant genes in the classification of the samples in stages D and B/C. Each gene includes a brief description, its identification and PubMed accession number, as well as the expression values shown in each of the stages B, C and D.

| GENE | Description | Gene ID | Accession Number | Dukes B | Dukes C | Dukes D |
|---|---|---|---|---|---|---|
| HAS1 | Hyaluronan synthase 1 | 3036 | NM_001523 | 0,119 | 0,044 | -0,1675 |
| LO8961 | *Homo sapiens* transmembrane tyrosine kinase mRNA, complete cds. | | LO8961 | | | |
| NHLRC2 | NHL repeat containing 2 | 374354 | AK126751 | -0,007 | 0,0315 | -0,261 |
| ATP6V0E2 | ATPase, H+ transporting V0 subunit e2 | 155066 | NM_145230 | -0,077 | 0,012 | 0,239 |
| RPS23 | Ribosomal protein S23 | 6228 | NM_001025 | 0,022 | 0,08 | -0,1615 |
| GTF2H2 | General Transcription factor IIH polypeptide 2 | 2966 | NM_001515 | 0,167 | 0,1875 | -0,1815 |
| FLJ34077 | Weakly similar to zinc finger protein 195 | 404033 | BC003519 | 0,497 | 0,2495 | -0,0755 |
| CASP12 | Caspase 12 | 120329 | NM_00035 | -0,054 | 0,017 | -0,2565 |
| RP5-1077B9.4 | Invasion inhibitory protein 45 | 60672 | NM_021933 | 0,346 | 0,3545 | 0,6605 |

- Distinction between stages B and C. Finally, only the data from the tumor samples in stages B and C was used. It is known that the distinction between these two stages is complex and many times false positives occur. After labeling the data in two phenotypes, B and C, a total of 11 genes shown in Table 9 were identified.

Table 9. Relevant genes in the classification of the samples in stages B and C. Each gene includes a brief description, its identification and PubMed accession number, as well as the expression values shown in each of the stages B, C and D.

| GENE | Description | Gene ID | Accesion Number | Dukes B | Dukes C | Dukes D |
|---|---|---|---|---|---|---|
| MEA1 | Male-enhanced antigen 1 | 685131 | NM_014623 | 0,065 | -0,165 | 0,167 |
| SPRY4 | Sprouty homolog 4 | 81848 | NM_030964 | 0,409 | 0,156 | 0,219 |
| TLR8 | Toll-like receptor 8 | 51311 | NM-016610 | 0,073 | 0,287 | -0,198 |
| SAMSN1 | SAM domain, SH3 domain and nuclear localization signals 1 | 64092 | NM_022136 | -0,015 | 0,5025 | 0,0705 |
| DGCR14 | DiGeorge syndrome critical region gene 14 | 8220 | NM_022719 | -0,048 | 0,287 | -0,198 |
| MRCL3 | Myosin regulatory light chain MRCL3 | 10627 | NM_006471 | -0,571 | 0,103 | -0,0135 |
| SMAD2 | Smad family member 2 | 4087 | NM_ 001003652 | -0,458 | 0,226 | -0,1935 |
| SFRS12 | Splicing factor, arginine/serine-rich 12 | 140890 | NM_139168 | -0,435 | 0,1795 | -0,009 |
| LAPTM4A | Lysosomal-associated protein transmembrane 4 alpha | 9741 | NM_014713 | 0,028 | 0,347 | -0,024 |
| CD1B | CD1b molecule | 910 | NM_001764 | 0,178 | -0,2965 | -0,0595 |
| FAM111A | Family with sequence similarity 111, member A | 63901 | NM 022074 | -0,016 | 0,3735 | 0,353 |

In the same way as in the case of the 7 previous genes, these 20 genes must be validated by means of RT-PCR using the same samples of the previous validation, for the purpose of verifying if the results of the microarrays are relevant.

**Claims**

1.  A method for the diagnosis of colorectal cancer, for determining the prognosis of a colorectal cancer patient or for determining the effect of a treatment in a colorectal cancer patient comprising determining in a sample of said patient the expression levels of genes TMEM132A, CMTM7, FAM60A, ENC1, ACAT1, MADCAM1 and DDX55, wherein an alteration in the expression levels of said genes with respect to the expression levels in a reference sample indicates that the patient suffers from colorectal cancer, that the colorectal cancer patient has a bad prognosis or that the treatment is not effective.

**2.** A method according to claim 1, wherein the expression alteration is an increase in the expression of genes ENC1, TMEM132A, FAM60A, CMTM7 and DDX55 and a decrease of the expression of genes ACAT1 and MADCAM1.

**3.** A method according to claim 1, wherein the sample of the patient is a colorectal tissue or a biological fluid sample.

**4.** A method according to claim 1, wherein the reference sample comes from non-tumor colorectal tissue.

**5.** A method for differentiating stage B/C colorectal tumors from stage D colorectal tumors comprising determining in a tumor sample the expression levels of genes HAS1, LO8961, NHLRC2, ATP6VOE2, RPS23, GTF2H2, FLJ34077, CASP12 and RP5-1077B9.4, wherein an alteration in the expression levels of said genes with respect to the expression levels in a reference sample indicates that the tumor is a stage B/C tumor according to Dukes' classification.

**6.** Method according to claim 5, wherein the reference sample is a sample coming from a stage D colorectal tumor according to Dukes' classification.

**7.** Method for differentiating stage B colorectal tumors from stage C colorectal tumors comprising determining in a said tumor the expression levels of genes MEA1, SPRY4, TLR8, SAMSN1, DGCR14, MRCL3, SMAD2, LAPTM4A, SFRS12, CD1B and FAM111A, wherein an alteration in the expression levels of said genes with respect to the expression levels in a reference sample indicates that the tumor is a type B tumor according to Dukes' classification.

**8.** A method according to claim 8, wherein the reference sample corresponds to one or several stage C tumors according to Dukes' classification.

**9.** A method for determining the stage of a colorectal tumor comprising

(i) determining if the tumor corresponds to stage B/C or to stage D by means of a method as defined in claim 5 and
(ii) in the event that the tumor is a stage B/C tumor, determining if the tumor corresponds to stage B or to stage C by means of a method as defined in claim 7.

**10.** A method for the diagnosis of colorectal cancer in a patient comprising

(i) determining if he or she has tumor tissue in a colorectal tissue sample by means of a method as defined in claim 1,
(ii) if tumor tissue is detected in step (a), determining if said tumor tissue corresponds to stage B/C by means of a method as defined in claim 5 and
(iii) if the tumor is identified as stage B/C in step (b), determining if the tumor corresponds to stage B or to stage C by means of a method as defined in claim 7.

**11.** A method according to any of the previous claims, wherein the determination of the expression levels of the genes is determined by means of RT-PCR.

**12.** A kit for the diagnosis of colorectal cancer or for the determination of the stage of a colorectal tumor comprising a first component and, optionally, a second component, wherein the first component is a set of reagents consisting of

(i) reagents which allow determining the expression levels of genes TMEM132A, CMTM7, FAM60A, ENC1, ACAT1, MADCAM1 and DDX55,
(ii) reagents which allow determining the expression levels of genes HAS1, LO8961, NHLRC2, ATP6VOE2, RPS23, GTF2H2, FLJ34077, CASP12 and RP5-1077B9.4 or
(iii) reagents which allow determining the expression levels of genes MEA1, SPRY4, TLR8, SAMSN1, DGCR14, MRCL3, SMAD2, LAPTM4A, SFRS12, CD1B and FAM111A

and wherein the second component comprises reagents suitable for determining the expression levels of one or several reference genes.

**13.** A kit as defined in claim 12, wherein the reagents are immobilized in a support.

**14.** Use of a kit as defined in claims 12 or 13 for the diagnosis of colorectal cancer, for determining the prognosis of a colorectal cancer patient, for determining the effect of a treatment in a colorectal cancer patient or for the determination of the stage of a colorectal tumor.

FIGURE 1

FIGURE 2

Europäisches Patentamt

European Patent Office

Office européen des brevets

**PARTIAL EUROPEAN SEARCH REPORT**   Application Number

which under Rule 63 of the European Patent Convention EP 08 38 0279
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/022432 A (CANADA NAT RES COUNCIL [CA]; BELACEL NABIL [CA]; CUPERLOVIC-CULF MIROS) 28 February 2008 (2008-02-28) | 12-14 | INV. C12Q1/68 |
| Y | * the whole document * | 1-4,10, 11 | |
| X | WO 2005/054508 A (IPSOGEN [FR]; INST PAOLI CALMETTES IPC [FR]; INST NAT SANTE RECH MED []) 16 June 2005 (2005-06-16) | 12-14 | |
| Y | * the whole document * <br><br> * tables A-G * <br><br> -/-- | 1-4,10, 11 | |

TECHNICAL FIELDS SEARCHED (IPC)

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:
see sheet C

| Place of search | Date of completion of the search | Examiner |
|---|---|---|
| The Hague | 18 May 2009 | Pinta, Violaine |

EP 2 169 078 A1

Europäisches Patentamt
European Patent Office
Office européen des brevets

**PARTIAL EUROPEAN SEARCH REPORT**

Application Number

EP 08 38 0279

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | BANDRES EVA ET AL: "A gene signature of 8 genes could identify the risk of recurrence and progression in Dukes' B colon cancer patients" ONCOLOGY REPORTS, vol. 17, no. 5, May 2007 (2007-05), pages 1089-1094, XP002514752 ISSN: 1021-335X | 12-14 | |
| Y | * the whole document *<br><br>* page 1089, right-hand column; figure 2; table 2 * | 1-4,10, 11 | |
| X | WANG Y ET AL: "Gene expression profiles and molecular markers to predict recurrence of Dukes' B colon cancer" JOURNAL OF CLINICAL ONCOLOGY, AMERICAN SOCIETY OF CLINICAL ONCOLOGY, US, vol. 22, no. 9, 1 May 2004 (2004-05-01), pages 1564-1571, XP003014769 ISSN: 0732-183X | 12-14 | TECHNICAL FIELDS SEARCHED (IPC) |
| Y | * the whole document *<br><br>* page 1567, right-hand column; table 2 * | 1-4,10, 11 | |
| X | ESCHRICH S ET AL: "Molecular staging for survival prediction of colorectal cancer patients" JOURNAL OF CLINICAL ONCOLOGY, AMERICAN SOCIETY OF CLINICAL ONCOLOGY, US, vol. 23, no. 15, 20 May 2005 (2005-05-20), pages 3526-3535, XP003014768 ISSN: 0732-183X | 12-14 | |
| Y | * the whole document *<br><br>* page 3530; table 5 *<br>* page 3533 * | 1-4,10, 11 | |
| | -/-- | | |

EPO FORM 1503 03.82 (P04C10)

Europäisches Patentamt
European Patent Office
Office européen des brevets

**PARTIAL EUROPEAN SEARCH REPORT**

Application Number

EP 08 38 0279

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | WO 2008/115419 A (GENOMIC HEALTH INC [US]; NSABP FOUNDATION INC [US]; COWENS WAYNE [US];) 25 September 2008 (2008-09-25) | 12-14 | |
| Y | * the whole document * | 1-4,10, 11 | |
| | * page 44 - page 47; example 2; table 3 * | | |
| X | WO 2007/100859 A (PFIZER PROD INC [US]; DEL RIO MARGUERITE [FR]; MOLINA FRANCK [FR]; PAU) 7 September 2007 (2007-09-07) | 12-14 | |
| Y | * the whole document * | 1-4,10, 11 | |
| | * examples 1-6 * | | |
| X | GROENE JOERN ET AL: "Transcriptional census of 36 microdissected colorectal cancers yields a gene signature to distinguish UICCII and III" INTERNATIONAL JOURNAL OF CANCER, JOHN WILEY & SONS, INC, UNITED STATES, SWITZERLAND, GERMANY, vol. 119, no. 8, 1 October 2006 (2006-10-01), pages 1829-1836, XP002466068 ISSN: 0020-7136 | 12-14 | TECHNICAL FIELDS SEARCHED (IPC) |
| Y | * the whole document * | 1-4,10, 11 | |
| A | * page 1835; table II * | 7-11 | |
| | -/-- | | |

EPO FORM 1503 03.82 (P04C10)

Europäisches
Patentamt

European
Patent Office

Office européen
des brevets

## PARTIAL EUROPEAN SEARCH REPORT

Application Number

EP 08 38 0279

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | ZOU TONG-TONG ET AL: "Application of cDNA microarrays to generate a molecular taxonomy capable of distinguishing between colon cancer and normal colon" ONCOGENE, NATURE PUBLISHING GROUP, GB BASINGSTOKE, HANTS, vol. 21, no. 31, 18 July 2002 (2002-07-18), pages 4855-4862, XP002363016 ISSN: 0950-9232 | 12-14 | |
| Y | * the whole document * | 1-4,10, 11 | |
| A | * figure 3b * | 7-11 | TECHNICAL FIELDS SEARCHED (IPC) |
| X | BIRKENKAMP-DEMTRODER KARIN ET AL: "Gene expression in colorectal cancer" CANCER RESEARCH, vol. 62, no. 15, 1 August 2002 (2002-08-01), pages 4352-4363, XP002514753 ISSN: 0008-5472 | 12-14 | |
| Y | * the whole document * | 1-4,10, 11 | |
| A | * figure 4 * | 5-11 | |
| | -/-- | | |

EPO FORM 1503 03.82 (P04C10)

Europäisches
Patentamt

European
Patent Office

Office européen
des brevets

**PARTIAL EUROPEAN SEARCH REPORT**

Application Number

EP 08 38 0279

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | FUJITA MANABU ET AL: "Up-regulation of the ectodermal-neural cortex 1 (ENC1) gene, a downstream target of the beta-catenin/T-cell factor complex, in colorectal carcinomas" CANCER RESEARCH, vol. 61, no. 21, 1 November 2001 (2001-11-01), pages 7722-7726, XP002514754 ISSN: 0008-5472 * the whole document * * figure 2 * | 1,2,10, 11 | |
| Y | REVAZ V ET AL: "The importance of mucosal immunity in defense against epithelial cancers" CURRENT OPINION IN IMMUNOLOGY, ELSEVIER, OXFORD, GB, vol. 17, no. 2, 1 April 2005 (2005-04-01), pages 175-179, XP025299758 ISSN: 0952-7915 [retrieved on 2005-04-01] * the whole document * * page 175, right-hand column * * page 176, left-hand column * | 1-4,10, 11 | TECHNICAL FIELDS SEARCHED (IPC) |
| A | US 2008/014579 A1 (LIU CHUNMEI [US] ET AL) 17 January 2008 (2008-01-17) * the whole document * * paragraph [0006] - paragraph [0010] * * paragraph [0087]; example 1; tables 2,3 * | 7-11 | |

EPO FORM 1503 03.82 (P04C10)

Europäisches
Patentamt

European
Patent Office

Office européen
des brevets

INCOMPLETE SEARCH
SHEET C

**Application Number**

EP 08 38 0279

Claim(s) searched incompletely:
    1-14

Reason for the limitation of the search:

Present claims 1, 5 and 7 relate to determination of expression levels of genes, said genes forming three non-overlapping groups of 7, 9 and 11 genes. When read in the light of the description, especially p. 13 1. 33 to p. 14 1. 3 (regarding claim 1), p. 19 1. 9-14 (regarding claim 5), and p. 37 1. 5-10 (regarding claim 7), it appears that the claims should be interpreted as relating to the determination of expression levels of at least one gene (claim 1) or at least two genes (claims 5 and 7) amongst the groups listed in each of said claims.

It follows that present claims 1, 5, 7 relate to an extremely large number of possible methods and products (respectively: 162, 538 and 2038 combinations), virtually, any method or kit comprising a set of reagents for the detection of at least one or at least two out of the markers listed. The number of possible combinations is such that a lack of clarity and conciseness arises within the meaning of Art. 84 EPC to such an extent that a meaningful search of the whole claimed subject-matter of the claim could not be carried out (Rule 63 EPC and Guidelines B-VIII, 3). The extent of the search was consequently limited.

Consequently, the search has been carried out for those parts of the claims which, in the light of the description and examples, appear to be clear and concise, namely the methods and products based on the detection of the following:
- claims 1-4, 9-14: each of the 7 genes of claim 1 individually and the combination of 7;
- claims 5-6, 9-14: the combination of 9 genes of claim 5;
- claims 7-14: the combination of 11 genes of claim 7.

Europäisches
Patentamt
European
Patent Office
Office européen
des brevets

**Application Number**

EP 08 38 0279

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 08 38 0279

The Search Division considers that the present European patent application does not comply with the requirements of unity of invention and relates to several inventions or groups of inventions, namely:

1. claims: claims 1-4 (completely), 10-14 (partially)

   Methods for the diagnosis, prognosis, staging or determining the effect of a treatment of colorectal cancer comprising determining expression levels of TMEM132A, CMTM7, FAM60A, ENC1, ACAT1, MADCAM1, DDX55, kit comprising a set of reagents for determining the expression of said genes, and use of said kit for the diagnosis, prognosis, staging or for determining the effect of a treatment of colorectal cancer.
   ---

2. claims: claims 5-6 (completely), 9-14 (partially)

   Method for differentiating stage B/C from stage D colorectal tumors comprising determining expression levels of HAS1, LO8961, NHLRC2, ATP6V0E2, RPS23, GTF2H2, FLJ34077, CASP12, RP51077B9.4, said method being further used in methods for staging or diagnosing colorectal cancer, kit comprising a set of reagents for determining the expression of said genes, and use of said kit for the diagnosis, prognosis, staging or for determining the effect of a treatment of colorectal cancer.
   ---

3. claims: claims 7-8 (completely), 9-14 (partially)

   Methods for differentiating stage B from stage C colorectal tumors comprising determining expression levels of MEA1, SPRY4, TLR8, SAMSN1, DGCR14, MRCL3, SMAD2, LAPTM4A, SFRS12, CD1B, FAM111A, said method being further used in methods for staging or diagnosing colorectal cancer, kit comprising a set of reagents for determining the expression of said genes, and use of said kit for the diagnosis, prognosis, staging or for determining the effect of a treatment of colorectal cancer.
   ---

**ANNEX TO THE EUROPEAN SEARCH REPORT
ON EUROPEAN PATENT APPLICATION NO.**                    EP 08 38 0279

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2009

| Patent document cited in search report | | Publication date | Patent family member(s) | | Publication date |
|---|---|---|---|---|---|
| WO 2008022432 | A | 28-02-2008 | NONE | | |
| WO 2005054508 | A | 16-06-2005 | US | 2005287544 A1 | 29-12-2005 |
| WO 2008115419 | A | 25-09-2008 | NONE | | |
| WO 2007100859 | A | 07-09-2007 | CA<br>EP | 2643225 A1<br>1994177 A2 | 07-09-2007<br>26-11-2008 |
| US 2008014579 | A1 | 17-01-2008 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 07032631 A
- WO 06015742 A
- WO 05044990 A
- EP 1439393 A
- WO 03097872 A
- WO 04001072 A
- EP 1355151 A
- US 2008014579 A
- WO 08061527 A
- WO 07101609 A
- WO 2005112973 A
- WO 0140803 A
- WO 9951773 A1

### Non-patent literature cited in the description

- **BOYLE, P. et al.** *BMJ,* 2000, vol. 321, 805-808
- **KEY, T. J. et al.** *Lancet,* 2002, vol. 360, 861-868
- **FERNANDEZ, E. et al.** *J Br Menopause Soc,* 2006, vol. 12 (4), 139-142
- **Dukes, C. E.** *J.Pathol. Bacteriol.,* 1932, vol. 35, 323-332
- **Dowdy ; Wearden.** Statistics for Research. John Wiley & Sons, 1983
- **Sambrook et al.** Molecular cloning: to Laboratory Manual. Cold Spring Harbor Laboratory Press, 2001, vol. 1-3
- **Fodor et al.** *Science,* 1991, 767-773
- **De Wildt et al.** *Nat. Biotechnol.,* 2000, vol. 18, 989-994
- **Lueking et al.** *Anal. Biochem.,* 1999, vol. 270, 103-111
- **Ge et al.** *Nucleic Acids Res.,* 2000, vol. 28 (e3), I-VII
- **MacBeath ; Schreiber.** *Science,* 2000, vol. 289, 1760-1763
- **Ausubel et al.** Current Protocols in Molecular Biology. John Wiley & Sons, 1992
- **CHEN, Y. et al.** *Bioinformatics,* 2002, vol. 18, 1207-1215
- **TROYANSKAYA, O. et al.** *Bioinformatics,* 2000, vol. 17, 520-525
- **BAS, A. et al.** *Scand. J. Immunol.,* 2004, vol. 59, 566-573
- **LIVAK, K. J. et al.** *Methods,* 2001, vol. 25, 402-408
- **Imbeaud et al.** *Nucleic Acids Res.,* 2005, vol. 33, 1-12
- **Murphy, D.** *Adv. Physiol. Edu.,* 2002, vol. 26, 256-270